(19) Europäisches Patentamt
European Patent Office
Office européen des brevets

(11) **EP 2 407 133 A1**

(12) **EUROPEAN PATENT APPLICATION**

(43) Date of publication:
**18.01.2012 Bulletin 2012/03**

(51) Int Cl.:
***A61F 13/15*** (2006.01)

(21) Application number: **10169724.1**

(22) Date of filing: **15.07.2010**

(84) Designated Contracting States:
**AL AT BE BG CH CY CZ DE DK EE ES FI FR GB
GR HR HU IE IS IT LI LT LU LV MC MK MT NL NO
PL PT RO SE SI SK SM TR**
Designated Extension States:
**BA ME RS**

(71) Applicant: **The Procter & Gamble Company**
**Cincinnati, OH 45202 (US)**

(72) Inventors:
 • **Carlucci, Giovanni**
 **66100, CHIETI (IT)**

 • **Peri, Andrea**
 **66020, Sambuceto (Chieti) (IT)**
 • **Tamburro, Maurizio**
 **66020, SAMBUCETO (CHIETI) (IT)**
 • **Di Girolamo, Luigi**
 **66020, Sambuceto (Chieti) (IT)**

(74) Representative: **Briatore, Andrea et al**
**Procter & Gamble Italia S.p.A.**
**Via Aterno, 128-130**
**66020 Sambuceto di S. Giovanni Teatino (Chieti)**
**(IT)**

(54) **ABSORBENT CORE**

(57) An absorbent core structure for disposable absorbent articles, having improved fluid handling properties.

Fig. 1

EP 2 407 133 A1

**Description**

**FIELD OF THE INVENTION**

The present invention relates to an absorbent core structure for absorbent articles, for example sanitary napkins and the like.

**BACKGROUND OF THE INVENTION**

Absorbent articles for absorption of body fluids such as menses or blood or vaginal discharges are well known in the art, and comprise for example feminine hygiene articles such as sanitary napkins, panty liners, tampons, interlabial devices, as well as wound dressings, and the like. When considering for example sanitary napkins, these articles typically comprise a liquid-pervious topsheet as wearer-facing layer, a backsheet as garment-facing layer and an absorbent structure, also referred to as core, between topsheet and backsheet. The body fluids are acquired through the topsheet and subsequently stored in the absorbent core structure. The backsheet typically prevents the absorbed fluids from wetting the wearer's garment.

An absorbent core structure can typically comprise one or more fibrous absorbent materials, which in turn can comprise natural fibres, such as for example cellulose fibres, typically wood pulp fibres, synthetic fibres, or combinations thereof.

Absorbent articles can further comprise, typically in the absorbent core, superabsorbent materials, such as absorbent gelling materials (AGM), usually in finely dispersed form, e.g. typically in particulate form, in order to improve their absorption and retention characteristics. Superabsorbent materials for use in absorbent articles typically comprise water-insoluble, water-swellable, hydrogel-forming crosslinked absorbent polymers which are capable of absorbing large quantities of liquids and of retaining such absorbed liquids under moderate pressure. Absorbent gelling materials can be incorporated in absorbent articles, typically in the core structure, in different ways; for example, absorbent gelling materials in particulate form can be dispersed among the fibres of fibrous layers comprised in the core, or rather localized in a more concentrated arrangement between fibrous layers.

Absorbent cores for absorbent articles having a thin structure can further provide an improved immobilization of absorbent gelling materials, particularly when the article is fully or partially loaded with liquid, and an increased wearing comfort. Such thinner structures provide absorbent articles combining better comfort, discreetness and adaptability, such as for example, thin absorbent structures where the absorbent gelling material is located and somehow kept in selected, e.g. patterned regions of the structure itself.

EP 1447067, assigned to the Procter & Gamble Company, describes an absorbent article, typically a disposable absorbent article, such as a diaper, having an absorbent core which imparts increased wearing comfort to the article and makes it thin and dry. The absorbent core comprises a substrate layer, the substrate layer comprising a first surface and a second surface, the absorbent core further comprising a discontinuous layer of absorbent material, the absorbent material comprising an absorbent polymer material, the absorbent material optionally comprising an absorbent fibrous material which does not represent more than 20 weight percent of the total weight of the absorbent polymer material. The discontinuous layer of absorbent material comprises a first surface and a second surface, the absorbent core further comprising a layer of thermoplastic material, the layer of thermoplastic material comprising a first surface and a second surface and wherein the second surface of the discontinuous layer of absorbent material is in at least partial contact with the first surface of the substrate layer and wherein portions of the second surface of the layer of thermoplastic material are in direct contact with the first surface of the substrate layer and portions of the second surface of the layer of thermoplastic material are in direct contact with the first surface of the discontinuous layer of absorbent material.

Absorbent articles according to EP 1447067 and comprising thin absorbent cores with relatively high amounts of absorbent gelling materials and rather low content of fibrous materials commonly have good absorption and retention characteristics to body fluids. However there still remains room for improvement for fluid handling, and particularly in order to better control rewet, e.g. due to gushing, and fluid acquisition effectiveness, in a core structure which is thin and comfortable, yet highly absorbent.

Low rewet, i.e. the capability of an absorbent structure of effectively and stably entrapping fluid within the structure itself, even after e.g. sudden gushes, with low tendency to give it back upon compression, for example upon squeezing of the absorbent structure which may occur during wear, is typically a characteristic which is in contrast with fast fluid acquisition, particularly in a thin absorbent structure. In other words, in order to have a thin absorbent structure which is also highly absorbent it is typically necessary to compromise between these two apparently contrasting features. In fact a thin absorbent structure, in order to rapidly acquire fluid, can typically have a rather "open" structure, which may in turn not provide for an optimal low rewet.

Thus, an absorbent core structure is desired exhibiting thinness for comfort combined with high absorbent capacity, while at the same time providing low rewet and fast fluid acquisition.

## SUMMARY OF THE INVENTION

The present invention addresses the above need by providing an absorbent core structure for an absorbent article, which comprises a first layer, comprising a first surface and a second surface; the absorbent core further comprises a layer of absorbent polymer material, comprising a first surface and a second surface; the absorbent core also comprises a layer of adhesive, comprising a first surface and a second surface. The layer of absorbent polymer material is comprised between the layer of adhesive material and the first layer. The second surface of the layer of absorbent polymer material is facing the first surface of the first layer, and the first surface of the layer of absorbent polymer material is facing the second surface of the layer of adhesive. The absorbent core structure of the present invention further comprises a second layer having respective first and second surface, positioned such that the second surface of the second layer is facing the first surface of the layer of adhesive. The absorbent core structure has a Virtual Free Fluid at 20 min of below 2.2 g, and a Virtual Acquisition Time at $2^{nd}$ gush of 35 sec or less.

## BRIEF DESCRIPTION OF THE DRAWINGS

Figure 1 is a plan view of a sanitary napkin showing an absorbent core according to an embodiment of the present invention, with portions of some constituent elements cut out in order to show underlying elements.

Figure 2 is a schematic cross section of the sanitary napkin of Figure 1 taken in the transverse axis A-A'.

Figure 3 shows a schematic cross section of an absorbent core according to one embodiment of the present invention.

Figure 4 shows a perspective view of an exemplary absorbent core according to the present invention.

Figure 5 shows an enlarged view of cross-sections of a fluid-swellable composite material with a number of water-swellable material particles with fluid in the pores between the particles and fluid in the particles.

Figure 6 is a schematic of a virtual test environment.

Figure 7 is a block diagram illustrating a computer system for operating a virtual test environment.

Figure 8 shows a two-dimensional virtual representation of the swelling behaviour and the level of fluid saturation of a flat absorbent article, over time.

Figure 9 shows a two-dimensional partial cross section of a spherical shell.

Figure 10 shows the determination of a two dimensional mesh nodal displacement direction.

Figure 11 shows a two-dimensional mesh displacement.

Figure 12 shows the scanning curve in a hysteresis loop: main drying curve (a) and main wetting curve (b).

Figure 13 shows equipment used to determine the capillary pressure, used herein.

Figure 14 shows a graph used to calculate the Stain area.

Figures 15 and 16 show equipment assemblies used in the Porosity under load test described herein.

Figures 17, 18 and 19 show equipment assemblies used in the In Plane Radial Permeability (IPRP) tests for non-swelling Samples and for swelling Samples described herein.

## DETAILED DESCRIPTION OF THE INVENTION

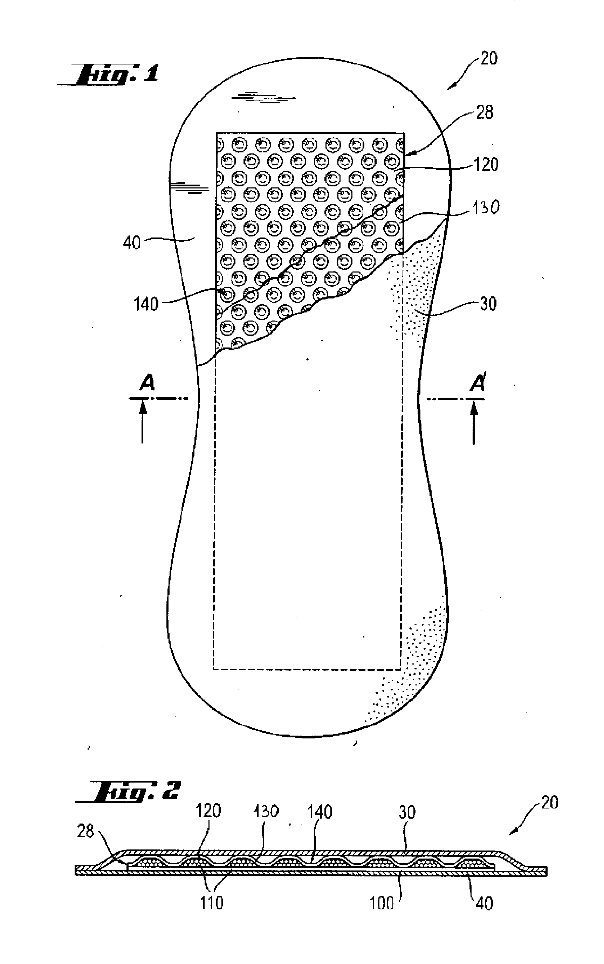

The present invention relates to an absorbent core for absorbent articles such as sanitary napkins, panty liners, tampons, interlabial devices, wound dressings, diapers, adult incontinence articles, and the like, which are intended for the absorption of body fluids, such as menses or blood or vaginal discharges or urine. Exemplary absorbent articles in the context of the present invention are disposable absorbent articles. The term "disposable" is used herein to describe articles, which are not intended to be laundered or otherwise restored or reused as an article (i.e. they are intended to be discarded after a single use and possibly to be recycled, composted or otherwise disposed of in an environmentally compatible manner). The terms "absorbent core"and" absorbent core structure" as used herein, are interchangeable, and refer to the core of the absorbent article. The absorbent article comprising an absorbent core according to the present invention can be for example a sanitary napkin or a panty liner. The absorbent core of the present invention will be herein described in the context of a typical absorbent article, such as, for example, a sanitary napkin 20 as illustrated in Figure 1. Typically, such articles as shown in Figure 1 can comprise the elements of a liquid pervious topsheet 30, a backsheet 40 and an absorbent core 28 intermediate said topsheet 30 and said backsheet 40.

In the following description of the invention, the surface of the article, or of each element thereof, which in use faces in the direction of the wearer is called wearer-facing surface. Conversely, the surface facing in use in the direction of the garment is called garment-facing surface. The absorbent article of the present invention, as well as any element thereof, such as, for example the absorbent core, has therefore a wearer-facing surface and a garment-facing surface.

Topsheet

According to the present invention, the absorbent article can comprise a liquid pervious topsheet. The topsheet suitable for use herein can comprise wovens, non-wovens, and/or three-dimensional webs of a liquid impermeable polymeric film comprising liquid permeable apertures. In Figure 1 the topsheet is indicated with reference numeral 30. The topsheet for use herein can be a single layer or may have a multiplicity of layers. For example, the wearer-facing and contacting surface can be provided by a film material having apertures which are provided to facilitate liquid transport from the wearer facing surface towards the absorbent structure. Such liquid permeable, apertured films are well known in the art. They provide a resilient three-dimensional fibre-like structure. Such films have been disclosed in detail for example in US 3929135, US 4151240, US 4319868, US 4324426, US 4343314, US 4591523, US 4609518, US 4629643, US 4695422 or WO 96/00548.

Absorbent Core

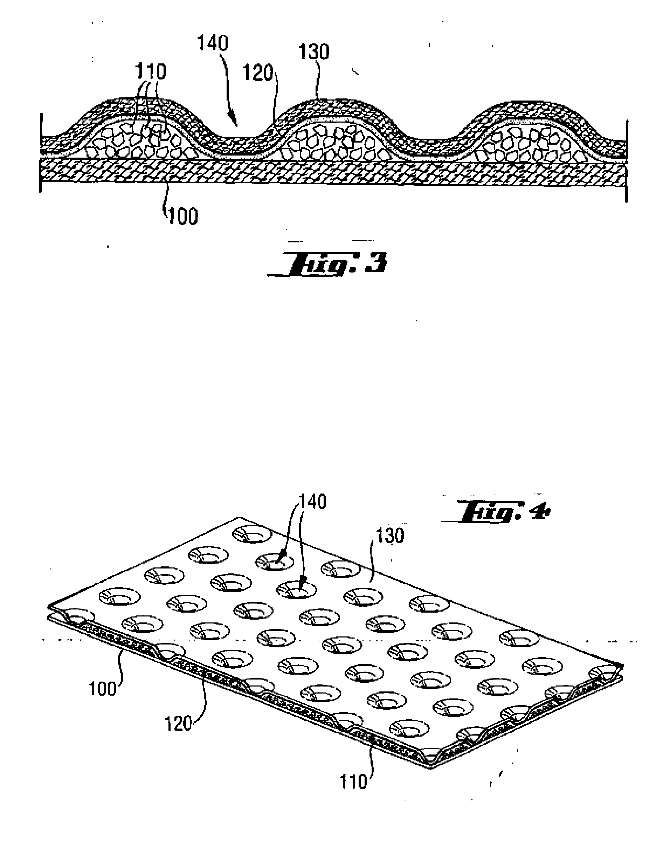

According to the present invention, and as shown for example in the embodiments of Figures 3 and 4, the absorbent core 28 can comprise a first layer, or substrate layer, 100, a layer of absorbent polymer material 110, a layer of adhesive 120, and a second layer, or cover layer, 130. According to the present invention, in the following description the terms "first layer" and "second layer" can be used interchangeably with "substrate layer" and "cover layer" respectively, and are meant to respectively refer to layers 100 and 130 in Figure 3. The terms "substrate" and "cover", referred to the first layer 100 and to the second layer 130, reflect one possible orientation of the absorbent core structure 28 when for example incorporated into an absorbent article, such as for example the sanitary napkin 20 shown in Figure 1, wherein the first layer 100 can actually constitute a substrate layer in that it is a bottom layer, i.e. for example closer to the backsheet 40, and the second layer 130 can actually constitute a cover layer in that it is a top layer, i.e. closer to the topsheet 30. Typically the adhesive can be a hot melt adhesive. According to the present invention, the layer of adhesive 120 can be typically for example a layer of fiberized hot melt adhesive 120. The substrate layer 100 can for example comprise a fibrous material. Suitable materials for the cover layer can be for example nonwoven materials.

The substrate layer 100, the layer of absorbent polymer material 110, the layer of adhesive 120, and the cover layer 130 each comprise a first surface and a second surface. Conventionally, in all the sectional views illustrated in the attached drawings the first surface of each layer is meant to correspond to the top surface, in turn, unless stated otherwise, corresponding to the wearer facing surface of the article 20 incorporating the absorbent core, while the second surface corresponds to the bottom surface, hence in turn the garment facing surface.

In general, in the absorbent core structure 28 of the present invention the arrangement of the various layers is such that the second surface of the layer of absorbent polymer material 110 is facing the first surface of the first or substrate layer 100, the first surface of the layer of absorbent polymer material 110 is facing the second surface of the layer of adhesive 120, and the second surface of the second or cover layer 130 is facing the first surface of the layer of adhesive 120.

According to the present invention, at least portions of the first surface of the substrate layer 100 can be in contact with the layer of absorbent polymer material 110. This layer of absorbent polymer material 110 comprises a first surface and a second surface, and can be typically a uniform or non uniform layer, wherein by "uniform" or "non uniform" it is meant that the absorbent polymer material 110 can be distributed over the substrate layer 100 respectively with uniform or non uniform basis weight over the area interested by the distribution. Conversely, the second surface of the layer of absorbent polymer material 110 can be in at least partial contact with the first surface of the substrate layer 100. According to the present invention, the layer of absorbent polymer material 110 can also be a discontinuous layer that is a layer typically comprising openings, i.e. areas substantially free of absorbent polymer material, which in certain embodiments can be typically completely surrounded by areas comprising absorbent polymer material. Typically these openings have a diameter or largest span of less than 10 mm, or less than 5 mm, or 3 mm, or 2 mm, or 1.5 mm and of more than 0.5 mm, or 1 mm. At least portions of the second surface of the absorbent polymer material layer 110 can be in contact with at least portions of the first surface of the substrate layer material 100. The first surface of the layer of absorbent polymer material 110 defines a certain height of the layer of absorbent polymer material above the first surface of the layer of substrate material 100. When the absorbent polymer material layer 110 is provided as a non uniform layer, typically for example as a discontinuous layer, at least some portions of the first surface of the substrate layer 100 can be not covered by absorbent polymer material 110. The absorbent core 28 further comprises a layer of adhesive 120, for example typically a hot melt adhesive. This typically hot melt adhesive 120 serves to at least partially immobilize the absorbent polymer material 110. According to the present invention, the adhesive 120 can be typically a fiberized hot melt adhesive, i.e., being provided in fibres as a fibrous layer.

The absorbent core 28 comprises a cover layer 130 having respective first and second surface, positioned such that the second surface of the cover layer 130 can be in contact with the first surface of the layer of typically hot melt adhesive 120.

According to the present invention comprising e.g. a non uniform layer of absorbent polymer material 110 the typically hot melt adhesive 120, for example typically provided as a fibrous layer, can be partially in contact with the absorbent polymer material 110 and partially in contact with the substrate layer 100. Figures 3 and 4 show such a structure in an exemplary embodiment of the present invention. In this structure the absorbent polymer material layer 110 is provided as a discontinuous layer, a layer of adhesive 120 is laid down onto the layer of absorbent polymer material 110, typically, for example, a layer of hot melt adhesive in fiberized form, such that the second surface of the adhesive layer 120 can be in direct contact with the first surface of the layer of absorbent polymer material 110, but also in direct contact with the first surface of the substrate layer 100, where the substrate layer is not covered by the absorbent polymer material 110, i.e. typically in correspondence of the openings of the discontinuous layer of the absorbent polymer material 110. By saying "in direct contact", as well as more generally "in contact", as used herein, in contrast to more generally saying "facing", it is meant that there is no further intermediate component layer between e.g. the layer of adhesive 120 and the other respective layer in direct contact thereto, such as for example a further fibrous layer. It is however not excluded that a further adhesive material can be comprised between the layer of adhesive 120 and the cover layer 130, or the layer of absorbent polymer material 110 or, more typically, the substrate layer 100, such as for example a supplementary adhesive material provided onto the first surface of the substrate layer 100 to further stabilize the overlying absorbent polymer material 110. "In direct contact and "in contact" can hence be considered to comprise in this context a direct adhesive contact between the layer of hot melt adhesive 120 and another respective layer as explained above, or more in general direct and, typically, adhesive contact between two layers, e.g. the layer of absorbent polymer material and the substrate layer. This imparts an essentially three-dimensional structure to the fibrous layer of hot melt adhesive 120 which in itself is essentially a two-dimensional structure of relatively small thickness (in z-direction), as compared to the extension in x- and y-direction. In other words, the layer of adhesive 120 undulates between the first surface of the absorbent polymer material 110 and the first surface of the substrate layer 100. The areas where the layer of adhesive 120 is in direct contact with the substrate layer 100, when present according to an embodiment of the present invention, are the areas of junction 140.

Thereby, in such an embodiment the adhesive 120 can provide spaces to hold the absorbent polymer material 110 typically towards the substrate layer 100, and can thereby immobilize this material. In a further aspect, the adhesive 120 can bond to the substrate 100 thus affixing the absorbent polymer material 110 to the substrate 100. Typical hot melt adhesive materials can also penetrate into both the absorbent polymer material 110 and the substrate layer 100, thus providing for further immobilization and affixation.

In the embodiment of Figure 3 portions of the cover layer 130 bond to portions of the substrate layer 100 via the adhesive 120. Thereby, the substrate layer 100 together with the cover layer 130 can provide spaces to immobilize the absorbent polymer material 110.

Of course, while the typically hot melt adhesive materials disclosed herein can provide a much improved wet immobilisation, i.e. immobilisation of absorbent polymer material when the article is wet or at least partially loaded, these hot melt adhesive materials can also provide a very good immobilisation of absorbent polymer material when the article is dry.

In accordance with the present invention, the absorbent polymer material 110 may also be optionally mixed with fibrous material, which can provide a matrix for further immobilization of the absorbent polymer material. However, typically a relatively low amount of fibrous material can be used, for example less than about 40 weight %, less than about 20 weight %, or less than about 10 weight % of the total weight of the absorbent polymer material 110, positioned within the areas of absorbent polymer material.

According to the present invention, in a typically discontinuous layer of absorbent polymer material 110 the areas of absorbent polymer material can be connected to one another, while the areas of junction 140 can be areas, which in an embodiment may correspond to the openings in the discontinuous layer of absorbent polymer material, as shown for example in Figure 4. The areas of absorbent polymer material are then referred to as connected areas. In an alternative embodiment, the areas of junction 140 can be connected to one another. Then, the absorbent polymer material can be deposited in a discrete pattern, or in other words the absorbent polymer material represents islands in a sea of adhesive 120. Hence, in summary, a discontinuous layer of absorbent polymer material 110 may comprise connected areas of absorbent polymer material 110, as e.g. illustrated in Figure 4, or may alternatively comprise discrete areas of absorbent polymer material 110.

The present invention, and for example the embodiments described with reference to Figures 3 and 4 can be typically used to provide the absorbent core of an absorbent article, as illustrated in Figure 1. In that case, no further materials wrapping the core, such as for example a top layer and a bottom layer are being used. With reference to the embodiment of Figure 3 the optional cover layer 130 may provide the function of a top layer and the substrate layer 100 may provide the function of a bottom layer of an absorbent core, wherein top and bottom layers respectively correspond to the body facing and garment facing surfaces of the core 28 in an absorbent article.

With reference to Figures 3 and 4, according to exemplary embodiments of the present invention, the areas of direct contact between the adhesive 120 and the substrate material 100 are referred to as areas of junction 140. The shape, number and disposition of the areas of junction 140 will influence the immobilization of the absorbent polymer material 110. The areas of junction can be for example of squared, rectangular or circular shape. Areas of junction of circular shape can have a diameter of more than 0.5 mm, or more than 1 mm, and of less than 10 mm, or less than 5 mm, or less than 3 mm, or less than 2 mm, or less than 1.5 mm. If the areas of junction 140 are not of circular shape, they can be of a size as to fit inside a circle of any of the diameters given above.

The areas of junction 140, when present, can be disposed in a regular or irregular pattern. For example, the areas of junction 140 may be disposed along lines as shown in Figure 4. These lines may be aligned with the longitudinal axis of the absorbent core, or alternatively they may have a certain angle in respect to the longitudinal edges of the core. A disposition along lines parallel with the longitudinal edges of the absorbent core 28 might create channels in the longitudinal direction which can lead to a lesser wet immobilization, hence for example the areas of junction 140 can be arranged along lines which form an angle of about 20 degrees, or about 30 degrees, or about 40 degrees, or about 45 degrees with the longitudinal edges of the absorbent core 28. Another pattern for the areas of junction 140 can be a pattern comprising polygons, for example pentagons and hexagons or a combination of pentagons and hexagons. Also typical can be irregular patterns of areas of junction 140, which also can give a good wet immobilization. Irregular patterns of areas of junction 140 can also give a better fluid handling behaviour in case of absorption of menses or blood or vaginal discharges, since fluid can start diffusing in whichever direction from any initial acquisition point with substantially the same probability of contacting the absorbent polymer material in the e.g. discontinuous layer. Conversely, regular patterns might create preferential paths the fluid could follow with lesser probability of actually contacting the absorbent polymer material.

According to the present invention the layer of adhesive 120 can comprise any suitable adhesive material. Typically, the layer of adhesive 120 can comprise any suitable hot melt adhesive material.

Without wishing to be bound by theory it has been found that those hot melt adhesive materials can be most useful for immobilizing the absorbent polymer material 110, which combine good cohesion and good adhesion behaviour. Good adhesion can typically ensure that the hot melt adhesive layer 120 maintains good contact with the absorbent polymer material 110 and in particular with the substrate material 100. Good adhesion is a challenge, namely when a nonwoven substrate material is present. Good cohesion ensures that the adhesive does not break, in particular in response to external forces, and namely in response to strain. The adhesive is subject to external forces when the absorbent product has acquired liquid, which is then stored in the absorbent polymer material 110 which in response swells. An exemplary adhesive should allow for such swelling, without breaking and without imparting too many compressive forces, which would restrain the absorbent polymer material 110 from swelling. It may be desirable that the adhesive does not break, which would deteriorate the wet immobilization. Exemplary suitable hot melt adhesive materials can be as described in the already mentioned patent application EP 1447067, particularly at sections [0050] to [0063].

The adhesive material, typically a hotmelt adhesive material, can be typically present in the form of fibres throughout the core, being provided with known means, i.e. the typically hot melt adhesive can be fiberized. Typically, the fibres can have an average thickness from about 1 $\mu$m to about 100 $\mu$m, or from about 25 $\mu$m to about 75 $\mu$m, and an average length from about 5 mm to about 50 cm. In particular the layer of typically hot melt adhesive material can be provided such as to comprise a net-like structure.

The adhesive material constituting the layer of adhesive 120, typically a hot melt adhesive, may have a basis weight of from 11 g/m$^2$ to 3 g/m$^2$, preferably of from 9 g/m$^2$ to 5 g/m$^2$, for example 8 g/m$^2$, or 6 g/m$^2$.

To improve the adhesiveness of the typically hot melt adhesive material 120 to the substrate layer 100 or to any other layer, in particular any other non-woven layer, such layers may be pretreated with an auxiliary adhesive.

In particular, typical parameters of a hot melt adhesive in accordance with the present invention can be as follows.

In an aspect, the loss angle tan Delta of the adhesive at 60˚C should be below the value of 1, or below the value of 0.5. The loss angle tan Delta at 60˚C is correlated with the liquid character of an adhesive at elevated ambient temperatures. The lower tan Delta, the more an adhesive behaves like a solid rather than a liquid, i.e. the lower its tendency to flow or to migrate and the lower the tendency of an adhesive superstructure as described herein to deteriorate or even to collapse over time. This value is hence particularly important if the absorbent article is used in a hot climate.

In a further aspect, typical hot melt adhesives in accordance with the present invention may have a sufficient cohesive strength parameter $\gamma$. The cohesive strength parameter $\gamma$ is measured using the Rheological Creep Test as referred to hereinafter. A sufficiently low cohesive strength parameter $\gamma$ is representative of elastic adhesive which, for example, can be stretched without tearing. If a stress of $\tau$ = 1000 Pa is applied, the cohesive strength parameter $\gamma$ can be less than 100%, less than 90%, or less than 75%. For a stress of $\tau$ = 125000 Pa, the cohesive strength parameter $\gamma$ can be less than 1200%, less than 1000%, or less than 800%.

It is believed that the layer of adhesive 120, typically a hot melt adhesive, provided onto the layer of absorbent polymer material 110, and in direct contact therewith, can provide an effective absorbent structure, stabilizing and containing the absorbent polymer material onto the substrate layer 100, both in dry, and also in wet conditions. This can be particularly relevant when the layer of absorbent polymer material 110 is provided by absorbent polymer particles, wherein the occurrence of loose absorbent polymer particles within the absorbent core structure is minimized.

Materials

Exemplary materials for the substrate layer 100 according to the present invention can comprise nonwoven materials comprising synthetic fibres, or natural fibres, or mixtures thereof, such as for example carded nonwovens, or more typically airlaid or wetlaid fibrous materials. The substrate layer 100 according to the present invention can be selected for example among latex or thermal bonded airlaid fibrous materials, comprising synthetic fibres and 0 to 50% by weight, or 0 to 20% by weight natural fibres, such as for example cellulose fibres.

According to another embodiment of the present invention, the substrate layer 100 can comprise a fibrous material comprising cellulose or cellulose derivative fibres, for example from about 40% to about 100% by weight of cellulose or cellulose derivative fibres, or from about 50% to about 95% by weight of cellulose or cellulose derivative fibres, or also from about 60% to about 90% by weight of cellulose or cellulose derivative fibres. In a core structure according to the present invention a substrate layer 100 constituted by a fibrous material comprising a substantial percentage of cellulose fibres can provide an advantage in terms of liquid distribution towards the liquid fraction which is not immediately absorbed by the upper layer of absorbent polymer material 110, and is directly acquired by the substrate layer 100.

According to the present invention, basis weights for the first or substrate layer 100 can typically range from about 10 $g/m^2$ to about 120 $g/m^2$, or from about 20 $g/m^2$ to about 100 $g/m^2$, or also from about 30 $g/m^2$ to about 70 $g/m^2$.

Exemplary materials for the cover layer 130 can be provided by nonwoven materials comprising synthetic fibres, such as polyethylene (PE), polyethylene terephthalate (PET), polypropylene (PP), and cellulose or cellulose derivative fibres. Exemplary materials can comprise for example from about 0% to about 90% by weight of cellulose or cellulose derivative fibres, or from about 50% to about 85% by weight of cellulose or cellulose derivative fibres, or also from about 60% to about 80% by weight of cellulose or also typically cellulose derivative fibres. As the synthetic polymers used for nonwoven production are usually inherently hydrophobic, they can be typically coated with hydrophilic coatings, for example with durably hydrophilic coatings to provide permanently hydrophilic nonwovens. Other nonwoven materials for the cover layer 130 can comprise composite structures such as a so called SMS material, comprising a spunbonded, a melt-blown and a further spunbonded layer. Basis weights for the second or cover layer 130 can typically range from 10 $g/m^2$ to 80 $g/m^2$, or from 10 $g/m^2$ to 60 $g/m^2$, or also from 20 $g/m^2$ to 40 $g/m^2$.

According to the present invention, the absorbent core structure 28 can have an overall thickness of between 0.5 mm and 2.5 mm, or between 1 mm and 2 mm

Typically the absorbent polymer material 110 for the absorbent cores according to the present invention can comprise absorbent polymer particles, known in the art e.g. as superabsorbent materials, or as absorbent gelling materials (AGM), or also as hydrogel forming materials, as referred to in the Background of the Invention. Typically absorbent polymer particles can have a selected average particle size.

According to the present invention, absorbent polymer materials, typically in particle form, can be selected among polyacrylates and polyacrylate based materials, such as for example partially neutralized, crosslinked polyacrylates.

According to the present invention the absorbent polymer material 110 in the absorbent core 28 is present throughout the area of the absorbent core in an average basis weight of less than about 250 $g/m^2$, or of less than about 200 $g/m^2$, or from about 60 $g/m^2$ to about 180 $g/m^2$, or from about 70 $g/m^2$ to about 150 $g/m^2$. An average basis weight is typically based on the whole area of the zone of application, i.e. interested by the layer of absorbent polymer material, and hence comprising possible openings included in an e.g. discontinuous layer. Typically, the absorbent polymer material 110 can constitute at least about 45%, or at least about 50%, or at least about 55%, by weight of the absorbent core, wherein the absorbent core can typically correspond to the embodiments described with reference to Figures 3 and 4, hence comprising the substrate layer, the layer of absorbent polymer material, the layer of thermoplastic material, the cover layer, and any other material possibly comprised within this structure as described above, namely for example the additional fibrous material mentioned above or the additional adhesive material.

The absorbent polymer particles of the layer of absorbent polymer material 110 can typically have a selected average particle size from about 200 μ to about 600 μ, or from about 300 μ to about 500 μ.

The average particle size of a material in particulate form, namely for example the absorbent polymer material, can be determined as it is known in the art, for example by means of dry sieve analysis. Optical methods, e.g. based on light scattering and image analysis techniques, can also be used.

According to the present invention the absorbent polymer material, typically e.g. in particle form, can be selected among the polyacrylate based polymers described in the PCT Patent Application WO 07/047598, which are polyacrylate based materials very slightly crosslinked, or substantially not crosslinked at all, this further improving the above mentioned synergistic effect. Particularly, said polyacrylate based materials can have an extractable fraction of at least about 30% by weight, between about 30% and about 80% by weight, or between about 32% and about 70% by weight, evaluated according to the Extractables test method described in the above referenced application. Alternatively, said polyacrylate based materials can have a retention capacity of at least about 30 g/g, at least about 35 g/g, or at least about 40 g/g, evaluated according to the Centrifuge Retention Capacity test described in the above referenced application. The absorbent polymer material can also be selected among the polyacrylate based polymers described in the PCT Patent Application WO 07/046052. Said polymers in fact are particularly effective in absorbing complex body fluids such as menses or blood, and upon absorption of such fluids do not generally show a marked swelling, followed by gel blocking, like traditional superabsorbents, but rather act to a certain extent as thickeners of the body fluid, immobilizing it as a sort of gelatinous mass within the absorbent structure, for example in the interstices among the fibres, without causing substantial swelling and in turn a sensible increase of the overall thickness of the absorbent core.

According to the present invention, the absorbent core can provide a more efficient fluid management, in terms of acquisition, immobilization and absorption and a better comfort, during the entire wearing time of the article, as explained above, which can be particularly useful in case of complex body fluids such as menses or blood. Overall, this increased efficiency in the composite structure according to the present invention can translate in a more effective exploitation of the absorbent capacity of the absorbent polymer material, also in presence of problematic body fluids such as menses or blood or vaginal discharges, and possibly also in a more efficient use of the entire structure of the absorbent core.

This is achieved in a structure which is typically thin and flexible, yet capable of employing more completely the absorption and immobilization capacity of the different materials, and having improved fit and resilience during absorption and therefore increased comfort during use.

According to the present invention, the absorbent core structure 28 can be constituted by the layers 100, 110, 120, and 130 described above, or can comprise additional layers. For example, an absorbent article can comprise an absorbent core according to the present invention further comprising a fibrous acquisition layer, for example between the second or cover layer 130 and the topsheet. According to the present invention the acquisition layer can for example comprise fibrous nonwoven materials made by air laying or wet laying of synthetic fibres such as polyethylene (PE), polyethylene terephthalate (PET), or polypropylene (PP), similarly to the cover layer 130 of the absorbent core 28 of the present invention.

Exemplary materials for the fluid acquisition layer could comprise spunbonded or carded nonwoven materials, or airlaid materials such as for example latex bonded or thermal bonded airlaid materials. Basis weights can typically range from about 10 $g/m^2$ to about 60 $g/m^2$, or from about 25 $g/m^2$ to about 40 $g/m^2$.

According to another embodiment of the present invention the absorbent article can comprise a further fibrous layer comprised for example between the first or substrate layer 100 and the backsheet, i.e. typically provided at the garment facing surface of the core. This optional layer can be provided by similar fibrous materials as those already described for the substrate layer 100 of the absorbent core of the present invention. This optional fibrous layer according to this further embodiment of the present invention can act as an added wicking layer receiving and distributing excess fluid. The presence of cellulose fibres can make the layer particularly effective in acquiring and diffusing the fraction of body fluids like menses or blood which is not completely absorbed by the absorbent polymer material of the absorbent core 28.

Further materials, also typically in particle form, can be comprised in the layer of absorbent polymer material, for example known odour control materials, or inert materials such as silica.

Backsheet

The absorbent article of Figure 1 comprising the absorbent core according to the present invention can also comprise a backsheet 40. The backsheet may be used to prevent the fluids absorbed and contained in the absorbent structure from wetting materials that contact the absorbent article such as underpants, pants, pyjamas, undergarments, and shirts or jackets, thereby acting as a barrier to fluid transport. The backsheet according to the present invention can also allow the transfer of at least water vapour, or both water vapour and air through it.

Especially when the absorbent article finds utility as a sanitary napkin or panty liner, the absorbent article can be also provided with a panty fastening means, which provides means to attach the article to an undergarment, for example a panty fastening adhesive on the garment facing surface of the backsheet. Wings or side flaps meant to fold around the crotch edge of an undergarment can be also provided on the side edges of the napkin.

In the present invention, the absorbent core structure can be provided by appropriately selecting its components, and particularly typically the substrate layer, the absorbent polymer material, and the cover layer, in order to improve its fluid handling properties. In a thin absorbent structure as that of the present invention, a high fluid acquisition capacity and a low rewet are two characteristics which are most beneficial to the user, as they ultimately provide for an absorbent product, comprising the absorbent core structure of the invention, which promptly acquires and absorbs fluid, also after sudden gushes, and effectively retains it also under pressure, typically for example when the article is squeezed and to a certain extent deformed by the forces exerted by the body during wear. Rewet of an absorbent structure, as known in the art, corresponds to the tendency of the absorbent structure to give back fluid after its absorption when subjected to compression, and can be measured according to appropriate tests. Hence rewet can be a measure of how effectively absorbed fluid is entrapped within an absorbent structure, and a low rewet generally corresponds to a better capacity of the absorbent structure of holding fluid, and of ultimately providing an absorbent article which can have a less wet, hence a drier surface and thus be more comfortable to the wearer. Typically in an absorbent structure a high fluid acquisition capacity, i.e. namely the capacity of acquiring fluid quickly within the structure, also when provided as a sudden gush, can be associated to a relative openness of the absorbent structure itself, which in turn can be less than optimal for rewet. Hence a high fluid acquisition capacity and a low rewet, though most beneficial for an absorbent structure, can be considered as contrasting features of an absorbent structure, particularly for a thin absorbent structure which can be preferred for comfort and discreetness, for which so far it has been necessary to compromise.

It has been now discovered that, by suitably selecting the component elements of an absorbent structure of the present invention it is possible to achieve both low rewet and high fluid acquisition capacity, moreover in an absorbent structure which is also particularly thin.

While in principle the performances of an absorbent structure in terms of fluid acquisition capacity and rewet could be measured according to appropriate test methods, according to the present invention they can be advantageously evaluated, actually predicted, with a model as that described in the copending European Application n. 09153881.9, filed on 27 February 2009 in the name of the same applicant. The model, which can be typically implemented in a computer system, can simulate the two-dimensional movement of a fluid in an absorbent structure that comprises fluid-swellable composite material, comprising a fluid-swellable solid material, typically superabsorbent material, and that comprises void spaces in the fluid-swellable composite material.

The simulation model, described below in the methods sections, applied to an absorbent core structure of the current invention predicts Virtual Free Fluid (VFF) and Virtual Acquisition Time (VAT) for an absorbent core 28 constituted by a composite structure as described above; the two values are said "virtual' as they are predicted, actually calculated, by the simulation model. The Virtual Free Fluid represents the amount of fluid which is present within the structure of the absorbent core, not being bound to the structure itself, e.g. absorbed by the particulate superabsorbent material or bound to the fibres, but rather "free", which in turn implies it could be squeezed out of the structure under e.g. compression, and generate rewet. Similarly, the Virtual Acquisition Time represents the time necessary for a given amount of fluid to be completely absorbed in an absorbent structure, after it has been provided to the structure in controlled conditions. It is also predicted, actually calculated, by the simulation model according to the present invention.

Virtual Free Fluid and Virtual Acquisition Time can be considered to be directly related to rewet and acquisition capacity of an absorbent core structure according to the present invention, and can be used to represent them, in order to describe the behaviour of the absorbent core structure upon absorption of a fluid at given conditions.

Virtual Free Fluid and Virtual Acquisition Time can be calculated for a given "simulated' absorbent core structure at different times and after provision of multiple gushes of "virtual" fluid. According to the present invention, three gushes of 4 ml of "virtual" fluid, which is representative of artificial menstrual fluid, are provided to the absorbent core structure at time t = 0, and then at time t = 10 min and t = 20 min, and the respective Virtual Acquisition Time is calculated. Virtual Free Fluid can be calculated for the simulated absorbent core structure at any time. According to the present invention, the Virtual Free Fluid calculated values at 20 min, immediately before the provision of the simulated third gush, and at 60 min can be considered as particularly representative of the behaviour of an absorbent core structure in terms of rewet in actual usage conditions, where multiple gushes of fluid are typically received, and in turn of its capacity of effectively manage said fluid gushes within the structure, in an already wet condition. Similarly, the Virtual Acquisition Time after the 2nd and the 3rd gush can be considered representative of the capability of absorbent core structure of effectively receiving subsequent amounts of fluid in an already wet condition, i.e. after a certain amount of fluid has been already acquired.

According to the present invention, an absorbent core structure as that for example illustrated in Figure 3 can have a Virtual Free Fluid at 20 min of below 2.2 g, or below 2 g, or also below 1.5 g, and a Virtual Acquisition Time at the second gush of 35 sec or less, or of 30 sec or less, or also of 25 sec or less.

It has also been found that an absorbent core structure according to the present invention can have a Virtual Free Fluid at 60 min of less than 2.2 g, or of less than 2 g, or also of less than 1.5 g, and a Virtual Acquisition Time at the third gush of 40 sec or less, or of 35 sec or less, or also of 30 sec or less. This is considered representative of an effective behaviour for the absorbent structure in terms of fluid acquisition capacity and rewet in wet conditions, i.e. after receiving multiple gushes of fluid, which is typical of actual usage conditions.

According to the present invention, the component elements of an absorbent core structure as that illustrated in Figure 3 may be suitably selected in order to have certain characteristics, expressed in terms of certain selected parameters which are used to represent them, and which form part of the input of the simulation model. According to the present invention, the parameters are Permeability, Capillary Pressure and Thickness, as will be explained more in detail. Generally speaking, Permeability may be considered in the context of the present invention as representative of the capability of a given material to transport a fluid in the x-y plane, while Capillary Pressure can be considered as representative of a material to wick fluid by capillary action. Indeed, Capillary Pressure is expressed in the context of the present invention in terms of Medium Absorption Pressure (MAP) in AMF, which is a descriptor of the Capillary Pressure of a material, as will be explained.

An absorbent core structure according to the present invention, for example as illustrated in Figure 3, can comprise a second or cover layer 130 with a Permeability of at least 250 Darcy, or at least 300 Darcy, or also at least 400 Darcy. The cover layer 130 may be selected in order to have a MAP of 0.020 m $H_2O$ to 0.050 m $H_2O$. Finally the thickness of said cover layer 130 may be of 0.3 mm to 0.6 mm.

Similarly, the substrate or first layer 100 of an absorbent core structure according to the present invention may be selected such as to have a Permeability of at least 500 Darcy, or at least 600 Darcy, or also at least 1000 Darcy. The substrate layer 100 can have a MAP of 0.01 m $H_2O$ to 0.06 m $H_2O$. Finally the thickness of said substrate layer 100 may be of 0.4 mm to 1.0 mm. The absorbent core structure according to the present invention shall comprise at least the first and the second layer 100, 130, the layer of absorbent polymer material 110 and the layer of adhesive 120, as described herein, and in an embodiment of the present invention can be actually constituted by the above layers. According to the present invention the absorbent core structure can also comprise other layers, as already explained. Any other additional or intermediate layer can be simulated as well with the simulation model, and the entire resulting absorbent core structure can be evaluated in terms of the respective Virtual Free Fluid and Virtual Acquisition Time. As a general criterion, it can be considered that every layer which is comprised between the fluid permeable topsheet and the typically fluid impermeable backsheet of an absorbent article constitute the absorbent core structure comprising the elements as specified above, and all of them can be simulated by means of the simulation model according to the present invention. For example, the first and/or the second layer can be in turn constituted by two or more layers combined together. Alternatively, the absorbent core structure comprised between the topsheet and the backsheet can further comprise additional layers such as an acquisition layer and/or a distribution layer, respectively positioned for example between the cover layer 130 and the topsheet 30, or between the substrate layer 100 and the backsheet 40. In such a case, each individual layer can be simulated by suitably applying the simulation model. Typically, layers made of a "porous medium" are only simulated by the simulation model, wherein as "porous medium" we intend a material with interconnected voids inside which dimension is significantly smaller than the material minimum dimension, typically the thickness for a layer. For a more precise definition, reference is made to the "continuum approach" definition found in "Dynamics of fluids in Porous Media" by Jacob Bear, Dover Science Books, 1988, chapter 1, paragraphs 2 and 3. Layers not constituted by a "porous medium", for example typically non absorbent layers and/or non fibrous materials such as a perforated plastic film, or a plastic net, or similar materials, are not simulated by the simulation model, and are actually not considered in the simulation of an absorbent core structure according to the present invention.

The invention will be illustrated with the following examples, where absorbent core structures are described having a first layer or substrate layer 100 corresponding, when the absorbent core structure is incorporated within an absorbent core product, such as for example typically a sanitary napkin, to a garment-facing surface of the structure itself, while the second or cover layer 130 corresponds to the wearer-facing surface. Hence, in the simulation runs the fluid is meant to enter the absorbent core structure via the second or cover layer 130. It is however meant that an absorbent core structure as defined in the appended claims falls within the scope of the present invention if it meets the values of Virtual Free Fluid and Virtual Acquisition Time when in the simulation run fluid is provided through either the first or the second layer thereof.

Example 1

An absorbent core as that illustrated in Figure 3 comprises a first layer or substrate layer 100 constituted by a 65 g/m$^2$ Latex Bonded AirLaid (LBAL) fibrous layer constituted by a homogeneous blend of 16 g/m$^2$ polyethylene terephthalate (PET), 6.7 dtex, 6 mm long fibres and 19.5 g/m$^2$ pulp fibres laid onto a 10 g/m$^2$ spunbonded polypropylene nonwoven, with 19.5 g/m$^2$ latex, having a thickness of 0.7 mm, a layer of absorbent polymer material 110 constituted by a particulate superabsorbent material available from Nippon Shokubai under the trade name Aqualic L520 distributed onto the substrate layer in a uniform layer having overall an average basis weight of 144 g/m$^2$, a layer of adhesive material 120 constituted by a hot melt adhesive available from HB Fuller under the trade name NV 1151 Zeropack applied in fibres having an average thickness of about 50 $\mu$m at a basis weight of 8 g/m$^2$, the layers 110 and 120 having an overall thickness of 0.5 mm, and a second layer or cover layer 130 constituted by a 28 g/m$^2$ hydrophilic spunbonded nonwoven of bicomponent 80/20 core/sheath polypropylene/polyethylene fibres, treated with 0.5% by weight Silastol PHP26 surfactant made by Schill & Seilacher, Germany, having a thickness of 0.3 mm

The parameters of Permeability, Capillary Pressure, Thickness, as well as all those to be fed into the simulation model are measured according to the attached test methods for the fluid-absorbent and/or fluid-swellable component materials of the absorbent core, namely for the first or substrate layer, the fluid-swellable composite material constituted by the layer of absorbent polymer material and the layer of adhesive, and the second or cover layer and summarized in the tables 1-4 below.

Geometry and mesh:

Table 1

| Property | Value | Unit |
|---|---|---|
| Absorbent core structure length* | 184 | mm |
| Absorbent core structure width (for post processing) | 59 | mm |
| First Layer thickness | 0.5 | mm |
| Fluid-swellable composite material thickness | 0.5 | mm |
| Second Layer thickness | 03 | mm |
| Horizontal number of mesh elements | 634 | 1 |
| First Layer vertical number of mesh elements | 8 | 1 |
| Fluid-swellable vertical number of mesh elements | 6 | 1 |
| Second Layer vertical number of mesh elements | 3 | 1 |
| * same for all the layers in current examples | | |

Intrinsic material properties:

Table 2

| First layer: | Parameter | Value | unit |
|---|---|---|---|
| Porosity | $\varepsilon$ | 0.877 | 1 |
| Permeability | $k$ | 480.18 | darcy |
| | $\delta$ | 4.0 | 1 |
| Capillary pressure Uptake Curve | $s^l_s$ | 1.0 | 1 |
| | $s^l_r$ | 0.0 | 1 |
| | $\alpha$ | 21.85 | 1/m |
| | $n$ | 4.31 | 1 |
| | $m$ | 1.3 | 1 |
| Capillary pressure Retention Curve | $s^l_r$ | 1.0 | 1 |
| | $s^l_r$ | 0.0 | 1 |
| | $\alpha$ | 3.60 | 1/m |
| | $n$ | 2.46 | 1 |
| | $m$ | 10.0 | 1 |

Table 3

| Absorbent polymer + adhesive material | Parameter | Value | unit |
|---|---|---|---|
| Porosity | $\varepsilon_{max}$ | 0.849 | 1 |
| | $\varepsilon_{scale}$ | 0.079 | 1 |
| | $\varepsilon_{exp}$ | 5.16 | 1 |

(continued)

| Absorbent polymer + adhesive material | Parameter | Value | unit |
|---|---|---|---|
| Permeability | $k_{base}$ | 47.45 | darcy |
| | $k_{coeff}$ | 0.505 | 1 |
| | $k_{expcoeff}$ | 1.62 | 1 |
| | $k_{sinecoeff}$ | 0.50 | 1 |
| | $k_{sinephase}$ | -7.92 | 1 |
| | $\delta$ | 2.8 | 1 |
| Capillary pressure Uptake Curve | $s^l_r$ | 1 | 1 |
| | $s^l_r$ | 0 | 1 |
| | $\alpha_{max}$ | 16.17 | 1/m |
| | $n$ | 1.31 | 1 |
| | $m$ | 2.1 | 1 |
| Capillary pressure Retention Curve | $s^l_s$ | 1 | 1 |
| | $s^l_r$ | 0 | 1 |
| | $\alpha_{max}$ | 10.92 | 1/m |
| | $n$ | 1.31 | 1 |
| | $m$ | 1.6 | 1 |
| Capillary pressure Swelling effect | $m^s_{2,threshold}$ | 4.70 | g/g |
| | $\alpha_{scale}$ | 459.29 | g/g |
| | $\alpha_{exp}$ | -0.015 | |
| Fluid swellable material Concentration | $C^s_{AGM0}$ | 165517.2 | g/m3 |
| Speed rate constant and Maximum Fluid swellable material x-load | $m^s_{2max}$ | 22.4 | g/g |
| | $\tau_0$ | 20000 | 1/day |
| | b | 3.61 | 1 |
| | $\beta_{kinexp}$ | 2.0 | 1 |
| | $s^l_{e,threshold}$ | 0.18 | 1 |

Table 4

| Second layer | Parameter | Value | unit |
|---|---|---|---|
| Porosity | $\varepsilon$ | 0.898 | 1 |
| Permeability | $k$ | 160.28 | darcy |
| | $\delta$ | 4.0 | 1 |
| Capillary pressure Uptake Curve | $s^l_s$ | 1 | 1 |
| | $s^l_r$ | 0.0 | 1 |
| | $\alpha$ | 6.77 | 1/m |
| | $n$ | 2.08 | 1 |
| | $m$ | 10.0 | 1 |

(continued)

| Second layer | Parameter | Value | unit |
|---|---|---|---|
| **Capillary pressure Retention Curve** | $s^l_s$ | 1.0 | 1 |
| | $s^l_r$ | 0.0 | 1 |
| | $\alpha$ | 2.22 | 1/m |
| | $n$ | 1.80 | 1 |
| | $m$ | 10.0 | 1 |

The above parameters of the component materials are fed into the simulation model, and the values of a Virtual Acquisition Time of 43 sec at the 2nd gush and of 53 sec at the 3rd gush, and of a Virtual Free Fluid of 2.2 g at 20 min and of 2.2 g at 60 min are calculated for the absorbent core structure, which is the Base Option 1, and are reported in the first row of Table 5 below. In order to improve the performances of the absorbent core a lower rewet combined with a higher fluid acquisition capacity are desirable. Rewet and acquisition capacity are related to, and can be represented in terms of, Virtual Free Fluid and Virtual Acquisition Time, in turn the parameters of the absorbent core structure which can be predicted, actually calculated, with the simulation model as said above.

The simulation model actually allows understanding the influence of each single component materials, namely in terms of the respective parameters that characterize it and are fed in the model itself, onto the final performance of the absorbent core structure made of the component materials. It is possible to vary one or more of the component materials, actually varying the respective representative parameters, and evaluate the effect in terms of resulting Virtual Free Fluid and Virtual Acquisition Time of the simulated composite absorbent structure.

In the example provided, illustrated in Table 1, the influence of variations in the cover layer 130 are evaluated, the other component materials of the absorbent core structure, namely the fluid-swellable composite material and the substrate layer 100 remaining the same. Table 1-4, complementary to Table 5, show in fact the values of all parameters for the all elements of the absorbent core structure, comprising those which remain unchanged in the different options illustrated in Table 5, namely the first layer or substrate 100 and the fluid-swellable composite material constituted by the layer of absorbent polymer material 110 and the layer of adhesive 120.
In order to understand the influence of variations in the cover layer 130 the simulation model is repeatedly run wherein certain parameters of the cover layer 130, namely Permeability, Capillary Pressure, expressed as MAP, and Thickness, are varied as illustrated in Table 5 in virtual Options 2 to 12, all other parameters constituting the simulation model input remaining the same as initially measured for the components of the Base Option 1.

As explained in the model description below MAP is a descriptor of the capillary pressure of a material, to note that, for the current examples, the changes in MAP are reflected in the simulation by only changing the value of $\alpha$ uptake. This allows changing the absolute sucking force of the material without changing the shape of the capillary pressure curve. MAP and $\alpha$ uptake are inversely proportional therefore to increase MAP of a factor x it is necessary to multiply $\alpha$ uptake by 1/x. Similarly MDP is related to $\alpha$ retention. To keep the capillary pressure hysteresis of uptake and retention curve to a meaningful value all the changes in MAP are also done for MDP which then means that the same changes are done to $\alpha$ of the uptake curve are also done for $\alpha$ of the retention curve.

Table 5

| Option | 2nd layer properties | | | VAT (sec) | | VFF(g) | |
|---|---|---|---|---|---|---|---|
| | Permeability (Darcy) | MAP (m $H_2O$) | Thickness (mm) | 2nd gush | 3rd gush | 20 min | 60 min |
| 1 (actual) | 160.28 | 0.04 | 0.3 | 43 | 53 | 2.2 | 2.2 |
| 2 (virtual) | 160.28 | 0.04 | 0.4 | 43 | 55 | 2.2 | 2.2 |
| 4 (virtual) | 160.28 | 0.02 | 0.3 | 48 | 62 | 1.8 | 1.8 |
| 5 (virtual) | 160.28 | 0.06 | 0.3 | 42 | 51 | 2.4 | 2.5 |
| 6 (virtual) | 405.30 | 0.04 | 0.3 | 32 | 38 | 1.8 | 1.8 |
| 7 (virtual) | 607.95 | 0.04 | 0.3 | 25 | 30 | 1.8 | 1.8 |
| 8 (virtual) | 160.28 | 0.06 | 0.6 | 34 | 42 | 2.9 | 3.2 |
| 9 (virtual) | 607.95 | 0.06 | 0.3 | 22 | 25 | 2.1 | 2.2 |

(continued)

| Option | 2nd layer properties | | | VAT (sec) | | VFF(g) | |
|---|---|---|---|---|---|---|---|
| | Permeability (Darcy) | MAP (m $H_2O$) | Thickness (mm) | 2nd gush | 3rd gush | 20 min | 60 min |
| 10 (virtual) | 607.95 | 0.04 | 0.6 | 18 | 20 | 2.3 | 2.4 |
| 11 (virtual) | 607.95 | 0.06 | 0.6 | 15 | 18 | 2.8 | 3 |
| 12 (virtual) | 405.30 | 0.04 | 0.4 | 28 | 33 | 2 | 2 |
| 13 (actual) | 330.79 | 0.03 | 0.5 | 30 | 36 | 2.1 | 2.1 |

Table 5 shows this influence on the ten "virtual' Options 2, 4 to 12, of the absorbent core structure. More in detail, with respect to the cover layer 130, "virtual' Option 2 has an increase in the thickness, "virtual' Options 4 and 5 have a variation in the MAP, "virtual' Options 6 and 7 have a variation in the Permeability; "virtual' Options 8 to 12 have variations in two or even all three (as in "virtual' Option 11) of the parameters characterizing the cover layer 130.

The best results are shown in virtual Options 6 and 7, with a cover layer having increased Permeability and same thickness compared to the Base Option 1, and, to a slightly lesser extent, in virtual Option 12, where the cover layer has increased Permeability and increased thickness compared to the Base Option 1. The three virtual options in fact show better results for both rewet and acquisition capacity, represented by consistently better, i.e. lower, values of the Virtual Acquisition Time both at 20 min and 60 min, and of the Virtual Free Fluid at the 2nd and 3rd gush compared to the Base Option 1. All other combinations in virtual Options 2 to 5 and 8 to 11 never provide improvements in all relevant values, namely Virtual Free Fluid and Virtual Acquisition Time at the two selected conditions respectively. At most some show better acquisition capacity, i.e. lower Virtual Acquisition Time, at the expense of the rewet, or vice versa improve rewet but with a lower acquisition capacity.

Hence the skilled person is provided with a clear understanding and a specific criterion in order to select a material for the cover layer 130 which can in turn lead to a composite absorbent structure having better performances in terms of rewet and acquisition capacity, for example with respect to the Base Option 1 as illustrated in Table 5.

It may be possible that not all virtual materials represented by the selected combinations of parameters which provide the best Virtual Acquisition Time and Virtual Free Fluid for the resulting composite absorbent structure may be readily available. However, it is clearly in the knowledge of the skilled person to identify a suitable material which more closely approximates the characteristics of the selected virtual material. In the example represented in Table 5, a suitable material having characteristics overall similar to those of virtual Option 12 is a 30 $g/m^2$ hydroentangled spunlaced nonwoven comprising PET fibres, available from Ahlstrom Milano s.r.l. under the code MI57422030. The whole set of parameters is measured for this actual material according to the test methods, which comprises a Permeability of 330.79 Darcy, a MAP of 0.03 m $H_2O$, and a thickness of 0.5 mm. A final run of the simulation model with the parameters characterizing the actual material (see table 2a) shows a Virtual Acquisition Time of 30 sec at the 2nd gush and 36 sec at the 3rd gush, and a Virtual Free Fluid of 2.1 g at 20 min and 2.1 g at 60 min, reported in last row of Table 1 as actual Option 13.

The parameters which constitute the input of the simulation model for this actual Option 13, are the same as already shown in tables 1, 3 and 4 for the component of the absorbent core structure which remain the same, besides the thickness of the selected second layer, which is 0.5 mm instead of 0.3 mm as in Table 1. The specific parameters of the selected material of the second layer are shown in Table 2a below.

Table 2a

| MI57422030 | Parameter | Value | unit |
|---|---|---|---|
| Porosity | $\varepsilon$ | 0.941 | 1 |
| Permeability | $k$ | 330.79 | darcy |
| | $\delta$ | 4.0 | 1 |

(continued)

| MI57422030 | Parameter | Value | unit |
|---|---|---|---|
| **Capillary pressure Uptake Curve** | $s^l_s$ | 1.0 | 1 |
| | $s^l_r$ | 0.0 | 1 |
| | $\alpha$ | 31.87 | 1/m |
| | $n$ | 4.56 | 1 |
| | $m$ | 1.0 | 1 |
| **Capillary pressure Retention Curve** | $s^l_s$ | 1.0 | 1 |
| | $s^l_r$ | 0.0 | 1 |
| | $\alpha$ | 13.00 | 1/m |
| | $n$ | 3.63 | 1 |
| | $m$ | 1.0 | 1 |

Of course the same reasoning can be applied to any other material of the composite absorbent structure, namely the substrate layer 100 and the layer of absorbent polymer particles 110, or also to any combinations thereof, hence providing the skilled person with a simple criterion on how to identify specific materials for the composite absorbent structure in order to have improved performances compared to the materials already known in the art.

Example 2

An absorbent core, as schematically illustrated in Figure 3, is selected, being identical to the one of Example 1, therefore all the parameters of tables 1-4 apply as well for this example.
The simulation model is run and the performances of the absorbent core structure in terms of Virtual Acquisition Time and Virtual Free Fluid at the selected conditions are reported in Table 6 as Base Option 1 (same as in Example 1).
In Example 2 the influence of variations in the substrate layer 100 on the overall performance of the absorbent core structure in terms of Virtual Acquisition Time and Virtual Free Fluid is studied. Similarly to Example 1, Table 1-4, complementary to Table 6, show in fact the values of all parameters for the all elements of the absorbent core structure, comprising those which remain unchanged in the different options illustrated in Table 6, namely the first layer or substrate 100 and the fluid-swellable composite material constituted by the layer of absorbent polymer material 110 and the layer of adhesive 120.
In order to understand the influence of variations in the substrate layer 100 the simulation model is repeatedly run wherein the parameters of Permeability, MAP and Thickness of the substrate layer 100 are varied as illustrated in Table 6 in virtual Options 2 to 5, all other parameters constituting the simulation model input remaining the same as initially measured for the components of the Base Option 1. Table 6 shows this influence in virtual Options 2 to 5.

Table 6

| Option | 1st layer properties | | | VAT (sec) | | VFF(g) | |
|---|---|---|---|---|---|---|---|
| | Permeability (Darcy) | MAP (m $H_2O$) | Thickness (mm) | 2nd gush | 3rd gush | 20 min | 60 min |
| 1 (actual) | 480.18 | 0.04 | 0.5 | 43 | 53 | 2.2 | 2.2 |
| 2 (virtual) | 480.18 | 0.04 | 1.0 | 24 | 32 | 2.7 | 2.9 |
| 3 (virtual) | 480.18 | 0.01 | 0.5 | 57 | 72 | 1.5 | 1.4 |
| 4 (virtual) | 303.98 | 0.04 | 0.5 | 68 | 84 | 1 | 1 |
| 5 (virtual) | 1013.25 | 0.01 | 0.7 | 35 | 43 | 1.4 | 1.4 |

From the results of the simulation runs some teachings can be achieved. An increase in the thickness of the substrate layer, as in virtual Option 2, improves the Virtual Acquisition Time compared to the Base Option 1, at the expense of a further increase in Virtual Free Fluid values. A decrease in MAP only, as shown in virtual Option 3, provides better Virtual Free Fluid values, but definitely higher Virtual Acquisition Time values. A lower permeabiliy, as in virtual Option 4, provides a drastic improvement in the Virtual Free Fluid values, but still with rather poor values for the Virtual Acquisition Time.

Finally virtual Options 5 explores a virtual substrate layer with a combination of higher Permeabiliy, lower MAP and different Thickness with more favorable values both in Virtual Free Fluid and in Virtual Acquisition Time.

As already explained with reference to Example 1, not all virtual materials are readily reproducible with actual materials having the same combination of parameters, namely Permeability, MAP and Thickness, which in the virtual Options calculated with the simulation model have provided the most favourable end results for the resulting absorbent core structure in terms of Virtual Acquisition Time and Virtual Free Fluid. However, when considering the results summarized in Table 6, an actual material approximating very closely the features of virtual Option 5 for the substrate layer has been identified. The suitable material having characteristics overall similar to those of virtual Option 5 is a Latex Bonded Air Laid (LBAL) 67 g/m$^2$ fibrous layer constituted by a homogeneous blend of 27.5 g/m$^2$ polyethylene terephthalate (PET) 6.7 dtex, 6 mm long fibres and 10 g/m$^2$ cellulose pulp fibres, layered onto a 10 g/m$^2$ polypropylene nonwoven, with 19.5 g/m$^2$ latex. The whole set of parameters is measured for this actual material according to the test methods, which comprises a Permeability of 960.22 Darcy, a MAP of 0.01 m $H_2O$, and a thickness of 0.7 mm. The parameters which constitute the input of the simulation model for this actual first layer material similar to the one used in Option 5, are shown in Table 4a below.

Table 4a

|  | Parameter | Value | unit |
|---|---|---|---|
| **Porosity** | $\varepsilon$ | 0.907 | 1 |
| **Permeability** | $k$ | 960.2234978 | darcy |
|  | $\delta$ | 4 | 1 |
| **Capillary pressure Uptake Curve** | $s^l_s$ | 1.0 | 1 |
|  | $s^l_r$ | 0.0 | 1 |
|  | $\alpha$ | 88.31 | 1/m |
|  | $n$ | 2.86 | 1 |
|  | $m$ | 1.0 | 1 |
| **Capillary pressure Retention Curve** | $s^l_s$ | 1.0 | 1 |
|  | $s^l_r$ | 0.0 | 1 |
|  | $\alpha$ | 34.37 | 1/m |
|  | $n$ | 3.45 | 1 |
|  | $m$ | 1.0 | 1 |

An additional run of the simulation model is finally conducted on an actual absorbent core structures derived from that of Example 1, Option 13, but comprising as the substrate layer material the actual material similar to the selected to be close to Option 5 in Example 2. The parameters which constitute the input of the simulation model for this best option, are the same as already shown in tables 1, 2a, 3 and 4a for the component of the absorbent core structure which remain the same, besides the thickness of the selected second layer, which is 0.5 mm instead of 0.3 mm and the thickness of the selected first layer, which is 0.7 mm instead of 0.5 mm as in Table 1.

The results are reported in Table 7, and show very favourable values for the Acquisition Time and the Free Fluid of the resulting absorbent core structures comprising the actual materials.

Table 7

| Option | Virtual Acquisition Time (sec) | | Virtual Free Fluid(g) | |
|---|---|---|---|---|
|  | 2nd gush | 3rd gush | 20 min | 60 min |
| Best Option | 28 | 34 | 0.3 | 0.3 |

The combination of the best available materials for the cover layer and for the substrate layer, as identified respectively from Example 1 and Example 2, as shown above, provides the absorbent core structures identified in Table 7 as Best Option which provides a meaningful and consistent improvement with respect to the best actual Option 13 of Example 1 as well as to actual Option 1 (reference), both in terms of Virtual Acquisition Time and Virtual Free Fluid under all selected conditions. In particular, Best Option has a very good acquisition capacity, namely a low Virtual Acquisition Time at the 2nd gush and also at the 3rd gush, i.e. also after subsequent fluid insults, combined with excellent rewet, i.e. very low Virtual Free Fluid values not only at 20 min, but also at 60 min, i.e. substantially at equilibrium conditions.

In order to improve the performances of the absorbent core a lower rewet combined with a higher fluid acquisition capacity are desirable. Rewet and acquisition capacity are proportional to, and can be represented in terms of, Virtual Free Fluid and Virtual Acquisition Time, in turn the parameters of the absorbent core structure which can be predicted, actually calculated, with the simulation model as described above.

Simulation model

The simulation model hereby also referred as "model' is a virtual method for analyzing the two-dimensional movement of a fluid in an absorbent article or in an absorbent core structure that comprises fluid-swellable composite material, which comprise a fluid-swellable solid material, and void spaces in said fluid-swellable composite material, and/or non swellable composite materials which consist of non-swellable solid material, and that comprises void spaces in said non-swellable composite material. Said absorbent core structure and composite material(s) being defined by a virtual two-dimensional mesh.

The method is based on the copending European Application n. 09153881.9, filed on 27 February 2009 in the name of the same applicant.

Said virtual model solves the following equations:

i) an equation for determining the liquid movement in said void spaces, at a given location of said composite material (s) and/ or at a given time;

$$\varepsilon \frac{\partial s^l(\psi^l)}{\partial t} + \varepsilon s^l \overline{\gamma} \frac{\partial \psi^l}{\partial t} - \nabla \cdot [k_r^l \boldsymbol{K}(\nabla \psi^l + e)] = - \left[ \frac{1}{\rho_o^l} \frac{\overline{C}_{AGMo}^s}{f^s} - \varepsilon \frac{\partial s^l}{\partial m_2^s} - s^l \left( \frac{\partial \varepsilon}{\partial m_2^s} + \frac{\varepsilon}{f^s} \frac{\partial f^s}{\partial m_2^s} \right) \right] \frac{\partial m_2^s}{\partial t} - \frac{1}{\rho_o^l} \phi^s(u^s) - \phi^l(u^s)$$

ii) an equation for determining the amount of liquid present in said fluid-swellable solid material, at a given location of said fluid-swellable composite material and/ or at a given time;

$$\frac{\partial \overline{C}_{L \to So}^s}{\partial t} = \tau \, a^{sl}(\hat{s}(s_e^l)) \dot{m}_2^s \overline{C}_{AGMo}^s - f^s \phi^s(u^s)$$

iii) an equation for determining the displacement over time of one or more locations of said mesh of the composite material(s), due to swelling, and the refinement of said mesh over time.

$$\nabla \cdot u^s = d^s$$

A detailed description of the equations and of the symbols above is provided below.

The simulation model is implemented on a computer system having a central processing unit, a graphical user interface including a display communicatively coupled to said central processing unit, and a user interface selection device communicatively coupled to the central processing unit, further details are provided below.

Said method, computer or computer system comprises herein:

a) a computer readable memory device containing data and instructions for analyzing movement of fluid in an absorbent article or absorbent core structure;

b) a means for analyzing movement of fluid in an absorbent article or absorbent core structure;

c) a means for reporting saturation of the absorbent article or absorbent core structure as a function of time and position (or location); and

d) a means for determining at a certain time, displacement of (a) location(s) of said fluid-swellable composite material, expressed as a displacement of a virtual mesh of said composite material, due to swelling thereof (due to liquid absorption), to obtain a location(s) displacement or mesh displacement (at said time); and

e) a means for correlating the amount of liquid in said solid fluid-swellable material and in said pores as a function of time and displacement position (as obtained above) to Virtual Free Fluid (VFF) and Virtual Acquisition Time (VAT).

The properties of said composite material(s), or solid material thereof, inputted into the model are selected from the permeability (k), capillary pressure ($p_c$), porosity ($\varepsilon$), fluid-swellable solid material (e.g. AGM) speed rate constant ($\tau$), maximum fluid-swellable solid material (e.g. AGM) x-load $m_{\max}^{s}$ and concentration (of the fluid-swellable solid material).

Specific methods to determine above properties are specified below in dedicated sections. Composite material geometries and dimensions as well as absorbent core structure and absorbent product geometries and dimensions are also specified.

The model determines the amount of liquid absorbed by said fluid-swellable composite material(s) (thus present in said solid, e.g. particulate, parts of the fluid-swellable composite material(s)) and the amount of liquid present in said void space(s), present between said solid parts (e.g. particles) of the fluid-swellable composite material, and the displacement of said material(s) due to swelling, at a certain time and/ or location in the article. This is typically done at multiple time intervals, to obtain a moving mesh and moving mesh image, which give a virtual picture of said material (s) or article over time. From this information VAT and VFF can be calculated as per their definition which is illustrated into the post-processing section below.

The simulation model provides a solution to evaluate the swelling of fluid-swellable composite materials and/or absorbent core structures and/or absorbent articles comprising said composite material and coupling it with the Richard's equation. Specifically, the flow and deformation processes in swelling porous media are modeled for absorbent hygiene products (e.g., diapers, wipes, papers), in order to determine certain performances or properties thereof, in particular VAT and VFF. The hysteretic unsaturated flow, liquid absorption and deformation of fibrous porous structures are described through a resulting set of equations, including a generalized Richards equation, an equation for the solid-mass conservation with kinetic reaction term, and a relationship for the solid strain. The system of equations must be closed by multiple constitutive relations that include rather complex expressions and make the system highly nonlinear. The swelling porous structures are modeled as a large-scale deformation problem with accumulating discrete spatial movements over finite time intervals.

Importantly, it requires a moving-mesh technique which incrementally updates two-dimensional model domains based directly on the current spatial distribution of the solid displacement within a Picard-type iteration scheme, as described in more detail below, whereby spatial, temporal and residual errors are mutually controlled.

"Fluid-swellable" means herein that the fluid-swellable composite material or fluid-swellable solid material (e.g. particles, fibres) herein changes volume due to contact with a fluid.

The fluid-swellable composite material, for the current invention is typically the combination of the layer of absorbent polymer material 110, and the layer of adhesive 120.

Said fluid-swellable material useable herein typically absorbs fluid, and then swells, due to an osmotic pressure gradient between fluid in said material and fluid outside said material.

Said fluid-swellable composite material useable herein typically absorbs fluid, and then increases in its content and volume and thus swells.

Alternatively, said fluid-swellable composite material useable herein absorbs fluid, and then swells, due to the fact that the fluid changes mechanical properties of the material.

"Non-swellable" means herein that the non-swellable composite material or non-swellable solid material (e.g. particles, fibres) herein don't changes volume due to contact with a fluid.

Non-swellable composite material herein doesn't increase its volume once in contact with liquid but can host liquid within its pore(s).

Some composite material without absorbent polymer material (e.g. AGM), might contain anyway fibers that show low swelling, such as for example cellulose. These composite material without absorbent polymer material even if showing a limited swelling are considered non swellable materials.

For the current invention said non-swellable composite material(s) are typically the first and the second layer 100, 130.

Non-swellable solid materials (part of both fluid swellable composite material and/or non-swellable composite material) can strongly bind some liquid to themselves (e.g. into their cavities or at fiber crossing) even without swelling. This liquid is not released while trying to remove it (e.g. with centrifugation or under vacuum). Non swellable solid materials showing this behavior are defined as absorbing non swellable materials. Non synthetic fibers (e.g. Cellulose, rayon, etc.) usually show this behavior.

Any fluid (also referred herein as "liquid") may be used herein, but fluid (or liquid) used is in the current invention is artificial menstrual fluid (AMF), prepared according to the formulation described herein.

The absorbent article or absorbent core structures herein may comprise i) fluid-swellable composite material (s) and void space in said fluid-swellable composite material(s), e.g. between the fluid-swellable solid material(s), e.g. such as the fluid-swellable superabsorbent material particles or fluid-swellable absorbent gelling materials, or AGM, and optionally fluid-swellable fibers ii) non-swellable composite material(s) and void space in said non-swellable composite material(s), e.g. between the non-swellable solid material, iii) a combination of above i) and ii).

The absorbent article may be typically a sanitary napkin, as is described above.

The absorbent article or absorbent core structure that can be described by the model may comprise said fluid-swellable composite material(s) in a single absorbent region, layer, or in multiple regions or layers, for example distinct layers as described herein below. It may comprise in addition one or more regions or layers that temporarily absorb or distribute fluid, but do not swell.

The fluid-swellable composite material herein comprises at least one fluid-swellable material in solid form, e.g. the particles of absorbent polymer material. The composite material can also comprise at least two such fluid-swellable materials, for example fluid-swellable particles and fluid-swellable fibres. The fluid-swellable composite material additionally comprises non-fluid-swellable (solid) material, including thermoplastic and/ or non-swellable fibrous material, and including the adhesive.

The absorbent core structure or absorbent article can comprise said fluid-swellable composite material, which comprises at least fluid-swellable superabsorbent particles and/or at least fluid-swellable fibres, preferably at least such particles, and optionally also binders, adhesives, non-swellable fibres, fillers.

In said fluid-swellable composite material it is possible to define a fluid-swellable fraction as the sum of all the fluid-swellable components and a non swellable fraction as the sum of all the non swellable components.

The total basis weight of the fluid-swellable composite material is therefore the sum of the basis weight of the fluid-swellable fraction and the basis weight of the non swellable fraction.

The absorbent structure described by the model can also comprise two or more such fluid-swellable composite materials (which may be the same) that are separated at least partially from one another by a non-swellable material that is however water-permeable. For example, this may be an absorbent structure with two or more layers, each comprising a fluid-swellable composite material, (partially) separated from one another by for example a nonwoven layer, or an adhesive layer. In another embodiment, the absorbent structure comprises one or more non-swellable composite materials, for instance an acquisition layer and/or a distribution layer, and one or more fluid-swellable composite material(s). The fluid-swellable composite material(s) can be placed on the top, in the middle or on the bottom of the absorbent structure or anywhere else in the absorbent structure, depending on specific applications.

According to the present invention, and as described herein, the model can simulate an absorbent structure as the absorbent core structure 28 described above, wherein the fluid-swellable composite material typically corresponds to the layer of absorbent polymer material 110 plus the layer of adhesive 120, and possibly fibers and/or inert materials if present in the layer of absorbent polymer material, as described above. The absorbent polymer material alone in turn corresponds to the fluid-swellable (solid) material and the non swellable composite material(s) are the first and/or the second layer. Alternative absorbent core structures comprising more layers, as said above, can be however simulated by suitably adapting and modifying the simulation model.

The virtual simulation model, method and system herein may be in principle a one dimensional or a two or a three dimensional model as the equations are the same for one, two or three dimensional cases. In the specific of the present invention a two dimensional model, method and system are considered.

The third dimension is described parametrically and is necessary to close the conservation equations and the volume calculations.

In particular the length (x-dimension) and the thickness (z-dimension) of the absorbent article are described explicitly in the model while the width (y-dimension) is described parametrically.

The y-dimension is equivalent to the absorbent article or absorbent core structure width and it is conventionally selected to be identical for all the composite materials or layers.

By absorbent article or absorbent core structure width it is herein intended the width (in the direction of the transverse axis) at the middle of the longitudinal axis of the absorbent article or absorbent core structure respectively.

For the description of the simulation model the equations are written for a more generic three dimensional case but the application to the current invention is always to be considered two dimensional.

The method, system and model herein use certain assumptions/approximations including:

1. The fluid-swellable composite material(s) comprises fluid-swellable solid material and may comprise voids between the particles of said material; liquid is either in said voids or inside the fluid-swellable particulate material.

2. Once fluid (liquid) is in the fluid-swellable material and caused it to swell, it remains inside said material.

3. The fluid (liquid) once in the fluid-swellable material and caused it to swell, does not redistribute inside the fluid-swellable material.

4. Fluid can distribute inside the voids; this distribution is governed by Darcy's law and liquid mass conservation.

5. The liquid in the voids is divided in two types: i) bound to the porous structure (fluid swellable or non - swellable) i.e. bound by capillary forces/ surface energies to the fiber surface ii) free into the void space therefore free to move outside the porous structure contributing to the Virtual Free Fluid.

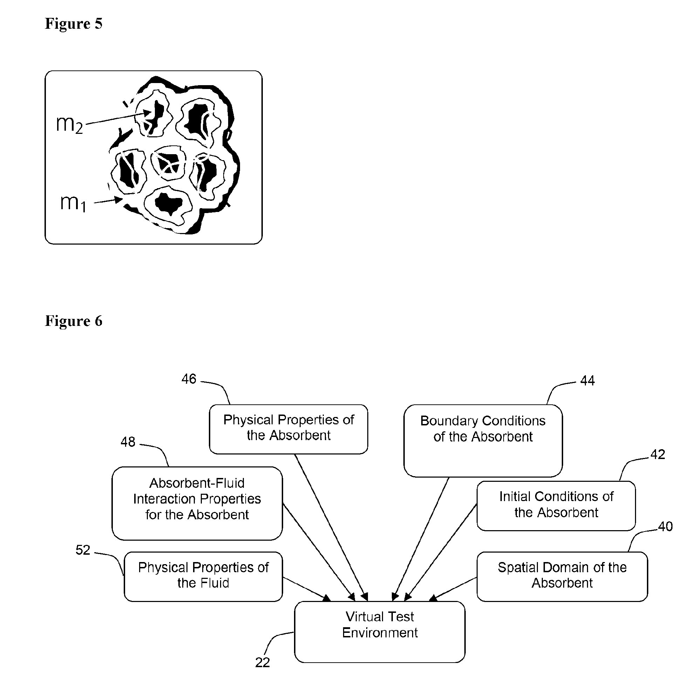

Figure 5 exemplifies how the liquid in the material can be regarded as liquid in voids $m_1$ and liquid in fluid-swellable material, e.g. fluid-swellable material particles, $m_2$, as mentioned above. Solutions to the equations herein above and herein after will depend on initial conditions and boundary conditions.

As stated the simulation model includes solutions of each and all of the following equations:

$$\varepsilon \frac{\partial s^l(\psi^l)}{\partial t} + \varepsilon s^l \gamma \frac{\partial \psi^l}{\partial t} - \nabla \cdot [k_r^l K (\nabla \psi^l + e)] = -\left[\frac{1}{\rho_o^l}\frac{\bar{C}_{AGMo}^s}{f^s} + \varepsilon \frac{\partial s^l}{\partial m_2^s} - s^l\left(\frac{\partial \varepsilon}{\partial m_2^s} + \frac{\varepsilon}{f^s}\frac{\partial f^s}{\partial m_2^s}\right)\right]\frac{\partial m_2^s}{\partial t} - \frac{1}{\rho_o^l}\phi^s(u^s) - \phi^l(u^s)$$

$$\frac{\partial \bar{C}_{L \to So}^s}{\partial t} = \tau \, a^{sl}(\hat{s}(s_e^l)) \dot{m}_2^s \bar{C}_{AGMo}^s - f^s \phi^s(u^s)$$

$$\nabla \cdot u^s = d^s$$

(E1)

Where the Primary variables are $\psi^l$ $\bar{C}_{L \to So}^s$ and $u^s$.

All letters and abbreviations used in the equations herein are described in the nomenclature list below in the description.

In addition to the equations above, the following constitutive relations apply and are included in the method:

$$s_e^l = \frac{s^l - s_r^l}{s_s^l - s_r^l} = \begin{cases} \dfrac{1}{[1 + |\alpha(m_2^s)\psi^l|^{n}]^{m}} & \psi^l < 0 \\ 1 & \upsilon^l \geq 0 \end{cases}$$

(E2-a)

$$\alpha(m_2^s) = \begin{cases} \alpha_{max} & m_2^s \leq m_{2,\,threshold}^s \\ \dfrac{\alpha_{max}}{[1 + \alpha_{scale}(m_2^s - m_{2,\,threshold}^s)]^{\alpha_{exp}}} & m_2^s > m_{2,\,threshold}^s \end{cases}$$

(E2-b)

$$\alpha_{max} = \begin{cases} \alpha_{max,wetting} & \dfrac{\partial \psi^l}{\partial t} > 0 \\ \alpha_{max,drying} & \dfrac{\partial \psi^l}{\partial t} < 0 \end{cases}$$

(E2-c)

$$k_r^l = (s_e^l)^{\delta}$$

(E2-d)

$$K_{base} = \frac{k_{base}\rho_o^l g}{\mu^l}$$

(E2-e)

$$\dot{m}_2^s = \frac{m_{2max}^s - m_2^s}{m_{2max}^s} \qquad \text{(E2-f)}$$

$$K = \frac{k\rho_o^l g}{\mu^l} = K_{base} I [1 - k_{coeff} \exp(k_{expcoeff}\dot{m}_2^s)\sin(2\pi k_{sinecoeff}\dot{m}_2^s - k_{sinephase})] \qquad \text{(E2-g)}$$

$$a^{sl}(\hat{s}(s_e^l)) = \frac{1 - e^{-\beta_{kinexp}\hat{s}}}{1 - e^{-\beta_{kinexp}}} \qquad \text{(E2-h)}$$

$$\hat{s}(s_e^l) = \frac{s_e^l - s_{e,\,threshold}^l}{1 - s_{e,\,threshold}^l} \qquad \text{(E2-i)}$$

$$\varepsilon = \frac{2\varepsilon_{max}}{1 - (m_2^s \varepsilon_{scale} + 1)^{\varepsilon_{exp}}} \qquad \text{(E2-j)}$$

$$\frac{\partial \varepsilon^s}{\partial m_2^s} = \frac{2\varepsilon_{max}\varepsilon_{scale}\varepsilon_{exp}(m_2^s \varepsilon_{scale} - 1)^{\varepsilon_{exp} - 1}}{\left[1 + (m_2^s \varepsilon_{scale} - 1)^{\varepsilon_{exp}}\right]^2} \qquad \text{(E2-k)}$$

$$\varepsilon_o^s = \frac{\overline{C}_{AFo}^s}{\rho_{AFo}^s} + \frac{\overline{C}_{AGMo}^s}{\rho_{AGMo}^s} \qquad \text{(E2-l)}$$

$$\varepsilon_{max} = 1 - \varepsilon_o^s \qquad \text{(E2-m)}$$

$$\frac{\partial s^l}{\partial m_2^s} = -\frac{(s_s^l - s_r^l)\psi^l mn[\alpha(m_2^s)\psi^l]^{n-1}}{\left\{1 + [\alpha(m_2^s/\upsilon^l)]^n\right\}^{m-1}} \frac{\alpha_{max}\alpha_{scale}\alpha_{exp}}{[1 - \alpha_{scale}(m_2^s - m_{2,\,threshold}^s)]^{\alpha_{exp} - 1}} \qquad \text{(E2-n)}$$

$$j^s = \frac{1}{1 - \varepsilon} \left\{\varepsilon_o^s + m_2^s \frac{\overline{C}_{AGMo}^s}{\rho_{H_2O}^s}\right\} \qquad \text{(E2-o)}$$

$$\frac{\partial j^s}{\partial m_2^s} = \frac{1}{1 - \varepsilon}\left(\frac{\overline{C}_{AGMo}^s}{\rho_{H_2O}^s} - j^s \frac{\partial \varepsilon^s}{\partial m_2^s}\right) \qquad \text{(E2-p)}$$

$$d^i = \frac{J_{n+1}^i}{J_n^i} - 1 \qquad \text{(E2-q)}$$

$$\phi^l(u^s) = \frac{\partial u^s}{\partial t} \cdot \nabla(\varepsilon s^l) \qquad \text{(E2-r)}$$

$$\phi^s(u^s) = \frac{\partial u^s}{\partial t} \cdot \nabla(\varepsilon^s \rho^s) \qquad \text{(E2-s)}$$

$$m_2^s = \frac{\overline{C}_{L \to So}^s}{\overline{C}_{AGMo}^s} \qquad \text{(E2-t)}$$

$$\tau = \tau_0 e^{\left(-b\frac{m_2^s}{m_{2max}^s}\right)} \qquad \text{(E2-u)}$$

$$\mu^l = \mu_0\left(1 + \mu_k\left(\left|\nabla\Psi^l\right|\frac{\mu_r}{2} - \mu_\sigma\right)^{\mu_e}\right) \qquad \text{(E2-v)}$$

Hereby, the saturation of the liquid phase $s^l$ is a secondary variable derivable from a VG capillary-pressure relationship at a known pressure head $\psi^l$.

$$s_e^l = \frac{s^l - s_r^l}{s_s^l - s_r^l} = \begin{cases} \dfrac{1}{[1 + |\alpha\psi^l|^n]^m} & \psi^l < 0 \\ 1 & \psi^l \geq 0 \end{cases} \qquad \text{(E3)}$$

Nomenclature for the equations E 1 to E 13:

The following letters and symbols are used herein in the equations E1 to E13 and mean the following:
AGM - refers to the fluid-swellable solid material, e.g. including absorbent gelling material, as described above.
AF- refers to airfelt material or any other non fluid-swellable component of the fluid-swellable solid material, e.g. glues, polymers, etc.

| | | |
|---|---|---|
| $A_p$ | $L^2$ | solid-liquid interface area per REV; |
| $A_{p, total}$ | $L^2$ | maximum solid-liquid interface area per REV; |
| $a^{si}(s^i)$ 1 | $1$ | saturation-dependent fraction of the solid-liquid interface area. |
| $a$ | $1$ | solid displacement direction vector: |
| $a_i$ | $1$ | direction vector at node i: |
| $a_l$ | $1$ | spatial components of $a$ ; |
| $b$ | $1$ | AGM poisoning factor |
| $B$ | $L$ | thickness; |
| $C$ | $ML^{-1}\,T^{-2}$ | stiffness tensor: |
| $C$ | $ML^{-3}$ | intrinsic concentration: |
| $\overline{C}$ | $ML^{-3}$ | bulk concentration: |
| $C_a$ | $L^{-1}$ | pore constant. |

(continued)

| | | | |
|---|---|---|---|
| $D/Dt$ | $T^{-1}$ | material derivative: |
| $d$ | $\mathbf{1}$ | strain vector: |
| $d^s$ | $\mathbf{1}$ | volumetric solid strain; |
| $e$ | $\mathbf{1}$ | $= -g \|g\|$ gravitational unit vector: |
| $f$ | | supply or function: |
| $G$ | $\mathbf{1}$ | geometry constant; |
| $g$ | $LT^{-2}$ | gravity vector, |
| $g$ | $LT^{-2}$ | $=\|g\|$ gravitational acceleration; |
| $H$ | $L^2$ | surface tension head: |
| $h^l$ | $L$ | $= x_1 - \psi^l$, hydraulic head of liquid phase $l$; |
| $I$ | $\mathbf{1}$ | unit vector: |
| $J^s$ | $\mathbf{1}$ | Jacobian of solid domain, volume dilatation function; |
| $j$ | $ML^{-2}T^{-1}$ | diffusive (nonadvective) flux vector; |
| $K$ | $LT^{-1}$ | hydraulic conductivity tensor; |
| $k$ | $L^2$ | permeability tensor. |
| $k_r$ | $\mathbf{1}$ | relative permeability; |
| $k^-$ | $M^{-1}LT^{-1}$ | reaction rate constant: |
| $L$ | $L^{-1}$ | gradient operator; |
| $L$ | $ML^{-3}T^{-1}$ | differential operator: |
| $l_i^s$ | $L$ | side lengths of a small solid cuboid; |
| $M$ | $M$ | molar mass: |
| $m$ | $\mathbf{1}$ | unit tensor; |
| $m$ | $M$ | mass; |
| $m$ | $\mathbf{1}$ | VG curve fitting parameter; |
| $m_2^s$ | $\mathbf{1}$ | AGMx-load: |
| $\hat{m}_2^s$ | $\mathbf{1}$ | $= (m_{2\max}^s - m_2^s)/m_{2\max}^s$. normalized AGM x-load, |
| $m_{\max}^s$ | $\mathbf{1}$ | maximum AGM x-load. |
| $n$ | $\mathbf{1}$ | pore size distribution index, |
| $p$ | $ML^{-1}T^{-2}$ | pressure, |
| $Q$ | $ML'T$ | mass supply, |
| $q$ | $LT^{-1}$ | volumetric Darcy flux, |
| $R$ | $ML^{-3}T^{-1}$ | chemical reaction term; |
| $R$ | $L$ | radius; |
| $r$ | $L$ | pore radius or distance; |
| $s$ | $\mathbf{1}$ | saturation; |
| $n$ | $L$ | solid displacement vector, placement transformation function: |
| $n$ | $L$ | scalar solid displacement norm. |
| $V$ | $L^3$ | REV volume; |
| $V_P$ | $L^3$ | pore volume; |
| $v$ | $LT^{-1}$ | velocity vector; |
| $x$ | $L$ | Eulerian spatial coordinates; |
| $x_i$ | $L$ | component of x ; |
| $\alpha$ | $L^{-1}$ | VG curve fitting parameters |
| $\beta$ | $\mathbf{1}$ | solid-liquid interface area empirical parameter |
| $\Gamma^3$ | $L^2$ | closed boundary of solid control space $\Omega^s$; |
| $\gamma$ | $M^{-1}LT^2$ | liquid compressibility; |
| $\bar{\gamma}$ | $L^{-1}$ | $= \gamma \bar{\rho}_o^l g$, specific liquid compressibility; |

(continued)

| | | |
|---|---|---|
| $\gamma_v$ | **1** | shear strain component; |
| $\delta$ | **1** | exponential fitting parameter; |
| $\delta_v$ | **1** | $= \begin{cases} 1 & i = j \\ 0 & i = j \end{cases}$ Kronecker delta: |
| $\varepsilon$ | **1** | porosity, void space; |
| $\varepsilon^\alpha$ | 1 | volume fraction of $\alpha$-phase: |
| $\mu$ | $ML^{-1}T^{-1}$ | dynamic viscosity: |
| p | $ML^{-3}$ | density or intrinsic concentration: |
| $\sigma$ | $ML^{-1}T^{-2}$ | solid stress tensor: |
| $\sigma^*$ | $ML^{-2}T^{-2}$ | liquid surface tension: |
| $\tau$ | $T^{-1}$ | AGM reaction (speed) rate constant, |
| $\phi^l, \phi^z$ | $T^1, ML^{-1}T^{-1}$ | deformation (sink/source) terms for liquid and solid, respectively, |
| $\psi^l$ | $L$ | pressure head of liquid phase $l$; |
| $\Omega^s$ | $L^3$ | control space of porous solid or domain: |
| $\omega_k$ | **1** | mass fraction of species $k$, |
| $\omega$ | **1** | reaction rate modifier: |
| $\nabla$ | $L^{-1}$ | Nabla (vector) operator (= grad); |
| $\nabla_i$ | | $= \partial/\partial x_i$, partial differentiation with respect to $x_i$; |
| $AGM_{raw}$ | | available AGM in reaction. |
| $AGM_{consumed}$ | | consumed AGM in reaction, |
| AGM | | AGM; |
| $c$ | | capillary; |
| $e$ | | effective or elemental: |
| $H_2O$ | | water. |
| $l$ | | material Lagrangian coordinate, ranging front 1 to 3; |
| $i, j$ | | spatial Eulerian coordinate, ranging from 1 to 3, or nodal in |
| $k$ | | species indicator; |
| L→S | | AGM absorbed liquid, |
| o | | reference, initial or dry; |
| $p$ | | pore; |
| $r$ | | residual, reactive or relative; |
| $\alpha$ | | phase indicator; |
| $D$ | | number of space dimension; |
| $g$ | | gas phase, |
| $l$ | | liquid phase: |
| $s$ | | solid phase; |
| $T$ | | transpose: |
| REV | | representative elementary volume, |
| RHS | | right-hand side; |
| SAP | | superabsorbent polymer: |
| VG | | van Genuchten: |
| [ --- ] | | chemical activity, molar bulk concentration; |
| ( ) · ( ) | | vector dot (scalar) product. |
| ( ) ⊗ ( ) | | tensor (dyadic) product; |

The deformation terms $\Phi^1(u^1)$ and $\Phi^s(u^s)$ herein are negligible if the product from the solid velocity $\delta u^s/\delta t$ and the gradient of the saturation $s^l$ as well as the gradient in the solid fluid-swellable material (i.e. referred to herein as AGM) x-load $m^s_2$ remains small relative to the other terms. This is accepted because the major displacement direction $u^s$ is taken herein to be perpendicular to the gradient of the fluid-swellable material x-load $\nabla m^s_2$ and the gradient of the saturation $\nabla_s{}^l$.

The solid displacement $\boldsymbol{u^s}$ may be computed from the hyperbolic differential equations $\nabla . \boldsymbol{u^s} = d^s$ where the scalar solid strain $d^s$ is a function of the volume dilatation $J^s$ and therefore a function of the AGM x-load $m_2{}^s$. The displacement vector $u^s$ can be decomposed into a scalar displacement norm $u^s$ and a displacement-direction vector $\boldsymbol{a^s}$ of unit size,

$$\boldsymbol{u}^s = u^s \boldsymbol{a}^s = u^s \begin{bmatrix} a_1^s \\ a_2^s \\ a_3^s \end{bmatrix}$$

(E4)

So that

$$\nabla \cdot \boldsymbol{u}^s = \boldsymbol{a}^s \cdot \nabla u^s + u^s \nabla \cdot \boldsymbol{a}^s$$

(E5)

The second term on the RHS will be zero for a homogeneous displacement direction, i.e., if all points of the domain move in the same direction. This term may be neglected if the restriction below is satisfied

$$\boldsymbol{a}^s \cdot \nabla u^s \gg u^s \nabla \cdot \boldsymbol{a}^s$$

(E6)

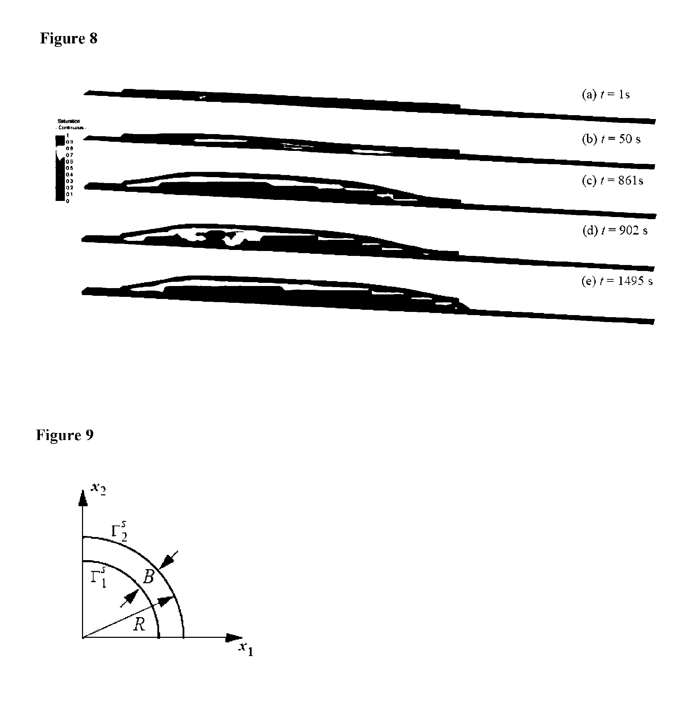

Namely, a geometric constraint on the curvature of the domain can be developed using an idealized domain of thickness $B$ and spherical inner $\left(\Gamma_1^s\right)$ and outer $\left(\Gamma_2^s\right)$ surfaces, both centered at the origin, as shown in Figure 9 ($R$ denote the radius of the outer surface). The inner surface is the fixed domain boundary and the swelling-direction vector field $a^s(x)$ is given by $a_i^s = x_i / r,$ where

$$r = \sqrt{\sum_{i=1}^{D} x_i^2}$$

(E7)

is the distance of x from the origin. The number of spatial dimensions is denoted by $D$. It follows that

$$\nabla \cdot \boldsymbol{a}^s = \frac{D-1}{r}$$

(E8)

Hereby, $u^s = u_{\min}^s = 0$ on $\Gamma_1^s$ and $u^s = u_{\max}^s = \Gamma_2^s$ on, the following order-of-magnitude estimates can be established:

$$O(u^s) = u_{\max}^s$$
$$O(\nabla u^s) = u_{\max}^s / B$$
$$O(a^s) = 1$$

(E9)

In two-dimensional space, $\Delta \cdot a^s = 1/R$ for any point on $\Gamma_2^s$, and, substituting the order-of magnitude estimates into the restriction,

$$u_{\max}^s / B \gg u_{\max}^s / R \qquad (E10)$$

Hereby:

$$R \gg B \qquad (E11),$$

because the error associated with ignoring the divergence of the displacement-direction vector field $a^s$ is negligible as long as the thickness of the domain remains much smaller than the radius of its curvature.

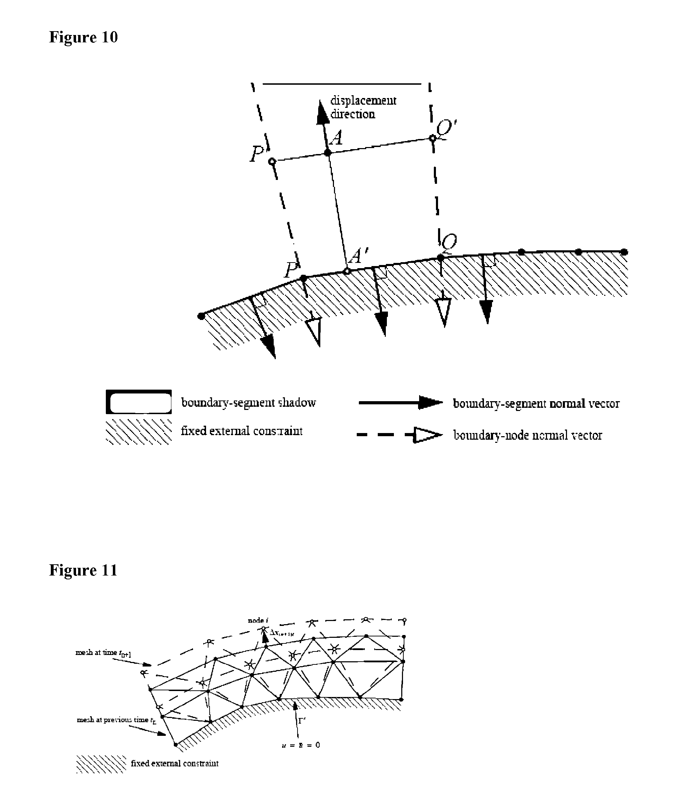

To simplify the numerical implementation, the displacement direction of each point within the domain remains stationary. Figure 10 shows how the mesh displacement direction can be determined.

Any pair of adjacent nodes on the fixed boundary defines a boundary segment. Normal vectors can be defined for boundary nodes as the resultant of the normals of the two segments connected by that node, scaled to unit size (the two end nodes of a boundary sequence have only one adjacent segment each). Denoting the position of mesh node $i$ by $A$, let $P$ and $Q$ be the positions of the two adjacent boundary nodes that delimit the boundary segment where $A$ is located. A line through $A$ that is parallel to line $PQ$ intersects the boundary-normals of $P$ and $Q$ at $P'$ and $Q'$, respectively. Point $A'$ between $P$ and $Q$ on the fixed boundary is then obtained such that

$$\frac{|P'A|}{|P'Q'|} = \frac{|PA'|}{|PQ|} \qquad (E12)$$

Line $A'A$ defines the stationary displacement direction $a_i^s$ for node $i$. A crossing of nodal displacement paths, which would lead to ill-defined mesh geometry, is completely prevented if the fixed boundary has no concave parts. Concave boundary sections are acceptable if the final (maximum) displacement is less than the distance at which the first crossover between neighboring nodes would occur. This condition is expected to be met in many practical applications.

As shown in Figure 10 the sequence of mesh nodes that define the fixed boundary of the model domain can defined. Figure 11 shows how the mesh displacement and mesh refinement as used herein can be done for the purpose of the invention.

As $d^s$ depends on $m_2^s$ the following equation must be solved at each time stage:

$$
\begin{aligned}
(a^s \cdot \nabla u_{(n+1)}^s) &= d_{n-1}^s \\
&= \frac{J_{n-1}^s}{J_n^s} - 1 \\
&= \frac{J^s(m_{2(n+1)}^s)}{J^s(m_{2(n)}^s)} - 1
\end{aligned}
\qquad (E13)
$$

Solution of equation (E13) is obtainable directly by evaluating the Jacobian $J^s$ at the current $(n+1)$ and previous $(n)$ time stages ad in equations (E2) in particular E2-o to E2q

The flow, absorption and deformation processes, described by E1, may be simplified as follows (whereby the *Nomenclature* for equations (E14) to (E32) is described below):

$$\varepsilon \frac{\partial s(\cup)}{\partial t} - \varepsilon s\gamma \frac{\partial \psi}{\partial t} - \nabla \cdot [k_r K(\nabla \psi - e)] = R_\cup(\psi, C, u) \left. \begin{array}{l} \\ \\ \frac{\partial C}{\partial t} = R_c(\psi, C, u) \\ \\ \nabla \cdot (a u) = d(C) \end{array} \right\} \quad \text{(E14)}$$

or in a compact form:

$$L(\phi) = m^T \frac{\partial \phi}{\partial t} + \nabla \cdot (f^c - f^d) - b = 0$$

$$\phi = \left\{ \begin{array}{c} \psi \\ C \\ u \end{array} \right\} \qquad m = \left\{ \begin{array}{c} \varepsilon \left( \frac{\partial s}{\partial \psi} - s\gamma \right) \\ 1 \\ 0 \end{array} \right\} \qquad f^c = \left\{ \begin{array}{c} -k_r K e \\ 0 \\ a u \end{array} \right\} \qquad f^d = \left\{ \begin{array}{c} -k_r K \nabla \psi \\ 0 \\ 0 \end{array} \right\} \qquad b = \left\{ \begin{array}{c} R_\psi \\ R_c \\ d \end{array} \right\}$$

$$\text{(E15)}$$

for solving the pressure head of liquid $\psi$, the sorbed liquid concentration $C \equiv m_2^s \overline{C}_{AGMo}^s$ and the solid displacement $u$.

In (2) $L(\phi)$ is the differential-equation system written in terms of the state variable $\phi(x,t)$. The main nonlinear functional dependence is shown in parentheses. Moreover, dependencies
exist for the saturation $s$ , relative permeability $k_r$, saturated conductivity $K$ and porosity $\varepsilon$ according to

$$s = s(\psi, C)$$

$$k_r = k_r(s) \qquad K = K(C) \qquad \varepsilon = \varepsilon(C) \qquad \text{(E16)}$$

where hysteresis in $s(\psi)$ is implied.

The reaction term $R_\psi$ possesses a sink for mobile liquid due to absorption by the fluid-swellable material. It is a complex relation and implies dependencies on liquid saturation $s$ (accordingly pressure head ($\psi$) with its derivation, porosity $\varepsilon$ with its derivation, solid displacement u and sorbed liquid concentration $C$ of the fluid-swellable solid material (e.g. AGM). The reaction $R_c$ possesses a kinetic production term of sorbed (immobile) liquid and is controlled by a reaction constant rate and said sorbed liquid concentration $C$. Furthermore, $R_c$ incorporates dependencies on liquid saturation $s$ (via the solid-liquid interface area) and the solid displacement $u$. The solid strain $d$ is a function of the said sorbed liquid concentration $C$.
The expressions for $R_\psi$ $R_c$ and $d$ can easily determined by the person skilled in the art via comparing (E14) and (E1).

The first equation of (E14) represents a generalized Richards-type flow equation written in a mixed ($\psi$ - $s$) -form where both variables of pressure head $\psi$ and saturation $s$ are employed, which is superior to a standard Richards-type form, where the saturation variable is substituted by the pressure head from beginning.

The pressure head $\psi$ may be chosen as primary variable in the present ($\psi$ - $s$) -formulation, which is capable of simulating both saturated and unsaturated porous media.

The finite element method known in the art is used to discretize the governing equation system E14. The equations are expressed on the physical domain $\Omega^s \subset R^D$, $t \geq t_o$ of porous solid, with the boundary $\Gamma_s$, lying on $D$-dimensional Euclidean space $R^D$, and for time $t$ starting at, and proceeding from some initial time $t_o$. The domain $\Omega^D$ is time-dependent $\Omega^s = \Omega^s (t)$ due to the swelling dynamics. The temporal dependence is considered within a finite interval ($t_n$, $t_n + \Delta t_n$), where the subscript n denotes the time level and $\Delta t_n$ is a variable time step length. We define $\Omega_n^s = \Omega^s(t_n)$

and $\Omega^s_{n+1} = \Omega^s(t_n + \Delta t_n)$ . The finite element formulation of equations (E14) finally yields the following nonlinear matrix system written in compact form:

$$
\left.
\begin{aligned}
B(C)\dot{S} - O(C)\dot{\Psi} + K(S,C)\Psi &= F(S,C,U) & \text{in} \quad \Omega^s_{n-1} \\
A\dot{C} &= Z(S,C,U) & \text{in} \quad \Omega^s_o \\
P\dot{U} + DU &= Q(C) & \text{in} \quad \Omega^s_{n+1}
\end{aligned}
\right\}
$$

$$(E17)$$

which has to be solved for $\Psi$, $C$, and $U$. Here, $\Psi$, $C$ and $U$ represent the resulting nodal vectors of the liquid pressure head for the deformed (swollen) volume $\Omega^s_{n+1}$, the concentration of absorbed liquid per reference (initial, undeformed) bulk volume $\Omega^s_o$, and the solid displacement for the deformed volume $\Omega^s_{n+1}$, respectively. (Vector $S$ is evaluated with known $\Psi$). The superposed dot indicates differentiation with respect to time $t$. The main nonlinear functional dependence is shown in parentheses. The second equation in (E17) is based on $\Omega^s_o$ as it involves no transport within the domain and its primary variable $C$ is defined with respect to the undeformed geometry. The matrices $B, O, A,$ and $P$ are symmetric. The conductance matrix $K$ is unsymmetrical if a Newton iteration technique is employed for the solution procedure, otherwise it is symmetric. The displacement matrix $D$ is always unsymmetrical. The remaining vectors $F, Z$ and $Q$ represent the RHS terms for liquid sink, kinetic absorption reaction and solid-strain source, respectively.

The third equation of (E17) represents the displacement equation. Due to numerical reasons the hyperbolic equation $\nabla \cdot (au) = d$ must be stabilized. Thus, the displacement equation $\nabla \cdot (au) = d$ is actually solved in a modified (extended) form:

$$
\kappa\frac{\partial u}{\partial t} - a \cdot \nabla u - \nabla \cdot (\sigma^\circ \cdot \nabla u) = d
$$

with

$$
\begin{aligned}
\kappa &= \text{const} \to 0 \\
\sigma^\circ &= \beta_{upwind}(a \otimes a)
\end{aligned}
$$

$$(E18 \text{ a and b})$$

and assuming $u \nabla \cdot a \approx 0$ where $\kappa$ is a small artificial compression factor, and $\beta_{upwind}$ denotes the upwind (dampening) parameter, which can be estimated from a characteristic finite element length $l$ as

$$
\beta_{upwind} \approx l/2
$$

$$(E18 \text{ c})$$

Then, the matrix system (4) is solved in time $t$ by applying a first-order fully implicit predictor-corrector (forward Euler/backward Euler) time stepping scheme with a *residual control,* as known in the art (Diersch and Perrochet (1999), On the primary variable switching techniques for simulating unsatured-saturated flows, Adv. Water Resour. 23 (1999) 271-301) and further described in DHI-WASY GmbH., Fleflow finite element subsurface flow and transport simulation system- User's Manual/ reference Manual/ White papers; Release 5.4; available from DHI-Wasy, Berlin (2008).

It results in the following matrix system

$$R_{n+1}^{\tau} = \frac{B(C_{n-1}^{\tau})}{\Delta t_n} S_{n+1}^{\tau} + \left( \frac{O(S_{n+1}^{\tau}, C_{n+1}^{\tau})}{\Delta t_n} + K(S_{n+1}^{\tau}, C_{n-1}^{\tau}) \right) \Psi_{n+1}^{\tau}$$

$$- \frac{B(C_{n+1}^{\tau})}{\Delta t_n} S_n - \frac{O(S_{n+1}^{\tau}, C_{n+1}^{\tau})}{\Delta t_n} \Psi_n - F(S_{n-1}^{\tau}, C_{n+1}^{\tau}, U_n) = 0 \qquad \text{in} \quad \Omega_{n+1}^s$$

$$\frac{A}{\Delta t_n} C_{n+1} = \frac{A}{\Delta t_n} C_n - Z(S_{n-1}^{\tau}, C_{n-1}^{\tau}, U_n) \qquad \text{in} \quad \Omega_o^s$$

$$\left( \frac{P}{\Delta t_n} - D \right) U_{n-1} = \frac{P}{\Delta t_n} U_n + Q(C_{n+1}^{\tau}) \qquad \text{in} \quad \Omega_{n-1}^s$$

$$(\text{E19 a and b and c})$$

where $\tau$ denotes the iteration counter, $R_{n+1}^{\tau}$ is the residual vector of the discretized Richards equation, $\Psi_{n+1}^{\tau}$ and $C_{n+1}^{\tau}$ represent iteration vectors for pressure and concentration to linearize the matrices and vectors. The iterates are started with $\tau = 0$ at the new time level ($n$ +1) by using predictor values $\Psi_{n+1}^{P}$ and $C_{n+1}^{P}$ according to

$$\left. \begin{array}{l} \Psi_{n+1}^{o} = \Psi_{n+1}^{P} = \Psi_n - \Delta t_n \dot{\Psi}_n \\ C_{n-1}^{o} = C_{n+1}^{P} = C_n + \Delta t_n \dot{C}_n \end{array} \right\}$$

$$(\text{E20})$$

where $\dot{\Psi}_n$ and $\dot{C}_n$ are acceleration vectors which have to be recorded during the adaptive time stepping solution process. The predictor values in relation to the corrector values are used to control the new time step according to

$$\Delta t_{n+1} = \Delta t_n \min \left[ \left( \frac{\delta}{\|d_{\psi}^{n-1}\|_{L_2}} \right)^{1/2}, \left( \frac{\delta}{\|d_c^{n-1}\|_{L_2}} \right)^{1/2}, \left( \frac{\delta}{\|d_u^{n+1}\|_{L_2}} \right)^{1/2} \right]$$

$$(\text{E21})$$

with the error vectors for pressure head, concentration and solid displacement

$$d_{\psi}^{n-1} = \frac{1}{2}(\Psi_{n+1} - \Psi_{n+1}^{P}) \qquad d_c^{n+1} = \frac{1}{2}(C_{n-1} - C_{n-1}^{P}) \qquad d_u^{n+1} = \frac{1}{2}(U_{n+1} - U_{n+1}^{P})$$

$$(\text{E22})$$

where $\|...\|_{L_2}$ are the RMS $L_2$ error norms and $\delta$ is a prescribed temporal error tolerance. This allows an automatic adaptation of the time step size $\Delta t_{n+1}$ in accordance with accuracy requirements.

This is continued by Picard iteration methods,

The matrix system is solved for the primary variable of pressure head and iterated as follows:

$$J(S_{n+1}^{\tau}, C_{n+1}^{\tau}) \Delta \Psi_{n+1}^{\tau} = -R_{n-1}^{\tau}$$

with the solution increment

$$\Delta \Psi_{n-1}^{\tau} = \Psi_{n-1}^{\tau+1} - \Psi_{n+1}^{\tau}$$

and the Jacobian with respect to the pressure

$$J(S_{n-1}^{\tau},\ C_{n-1}^{\tau}) = \frac{\partial R_{n+1}^{\tau}(S_{n+1}^{\tau},\ C_{n+1}^{\tau})}{\partial \Psi_{n+1}^{\tau}}$$

(E23 a, b and c)

The jacobians are given for the picard method as

$$J = \frac{B}{\Delta t_n}\frac{\partial S_{n-1}^{\tau}}{\partial \Psi_{n-1}^{\tau}} + \frac{O}{\Delta t_n} + K$$

(E23 d)

The Jacobian matrices $J$ (10d) and (10e) are symmetrical for the Picard method. The iterations $\tau$ in (10a) may be repeated until a satisfactory convergence is achieved. For example, the iterations are terminated if the residual falls under a user-given error tolerance $\eta$, *viz.*,

$$\left\|R_{n+1}^{\tau}\right\|_{L_2} < \eta$$

(E24)

where the weighted RMS $L_2$ error norm is used.

The remaining matrix equations for the absorbed concentration and solid displacement are solved by a decoupled *sequential iterative approach* (SIA), which is combined in an error-controlled adaptive predictor-corrector time stepping technique. Finally, it solves the coupled matrix system for $\Psi$, $C$ and $U$ at the time level ($n$+1) as follows:

$$\left.\begin{array}{ll} \text{Initialize:}\ \Psi_{n-1}^{o} = \Psi_{n-1}^{p},\ C_{n+1}^{o} = C_{n-1}^{p} \\ \text{Solve:}\quad J\Delta\Psi_{n+1}^{\tau} = -R_{n+1}^{\tau}\ \text{up to}\quad \left\|R_{n-1}^{\tau}\right\|_{L_2} < \eta\quad \text{in}\quad \Omega_{n-1}^{s} \\ \text{Take:}\quad S_{n+1}^{\tau} = S_{n+1}^{\tau}(\Psi_{n-1}^{\tau}),\ C_{n+1}^{\tau} = C_{n-1}^{\tau}(\Psi_{n+1}^{\tau}) \\ \text{Solve:}\quad \frac{A}{\Delta t_n}C_{n-1} = \frac{A}{\Delta t_n}C_n + Z(S_{n-1}^{\tau},\ C_{n-1}^{\tau},\ U_n)\quad \text{in}\quad \Omega_o^s \\ \text{Solve:}\quad \left(\frac{P}{\Delta t_n} + D\right)U_{n-1} = \frac{P}{\Delta t_n}U_n + Q(C_{n+1}^{\tau})\quad \text{in}\quad \Omega_{n+1}^s \end{array}\right\}$$

(E25)

Mesh movement and refinement as used herein are further be done as follows.

The computed solid displacement $u_{(n+1)i}a_i$ at the node i and at the new time level ($n$+1) is used together with the stationary displacement direction $a_i$ to move the finite element mesh in an incremental step according to

$$\Delta x_{(n+1)i} = u_{n+1)i}a_i$$

(E26)

where $\Delta x_{(n+1)i}$ represents the change in position of node between time $t$ and time $t_{n+1}$. Using this procedure the finite element mesh is updated incrementally in time, as for example shown in Figure 11. The applied procedure uses quadrilateral elements.

Since the swelling of fluid-swellable material herein is typically large (more than ten times of the initial geometry) the element shapes can become unfavorably distorted. This is particularly the case for triangular elements, where skewed and obtuse-angled shapes should be avoided due to numerical reasons.

Thus, as preferred feature of the present invention, an adaptive mesh refinement (AMR) procedure is applied which is controlled via *a-posteriori* error estimates of the solution by using the error energy norm

$$\|E\|^2 = \int_{\Omega^s} E^T L(E)d\Omega$$

with

$$E = \phi - \tilde{\phi}$$

(E27 a and b)

where the exact and the approximate finite element solution is denoted by $\phi$ and $\tilde{\phi}$, respectively. An error criterion is used in the form

$$\xi = \frac{\|E\|}{\|\phi\|}$$

(E28)

to refine the meshes, where it can be shown that

$$\|E\|^2 = -\int_{\Omega^e} (\nabla(\phi - \tilde{\phi}) \cdot (f^d - \tilde{f}^d)) d\Omega$$

$$\|\phi\|^2 = -\int_{\Omega^e} (\nabla\phi) \cdot f^d d\Omega$$

(E29)

To evaluate (16), $\nabla\phi$ is determined by a recovery technique and $\nabla\tilde{\phi}$ by a direct differentiation, as known in the art. By applying equation (E28), finite elements are refined, herein also referred to continuous mesh refinement, according to the accuracy requirements. Alternatively, it is possible to do mesh stretching without refinement when the mesh starts in a precompressed shape.

For the current invention this alternative option of the method is selected and simulation uses precompressed quadrangular elements.

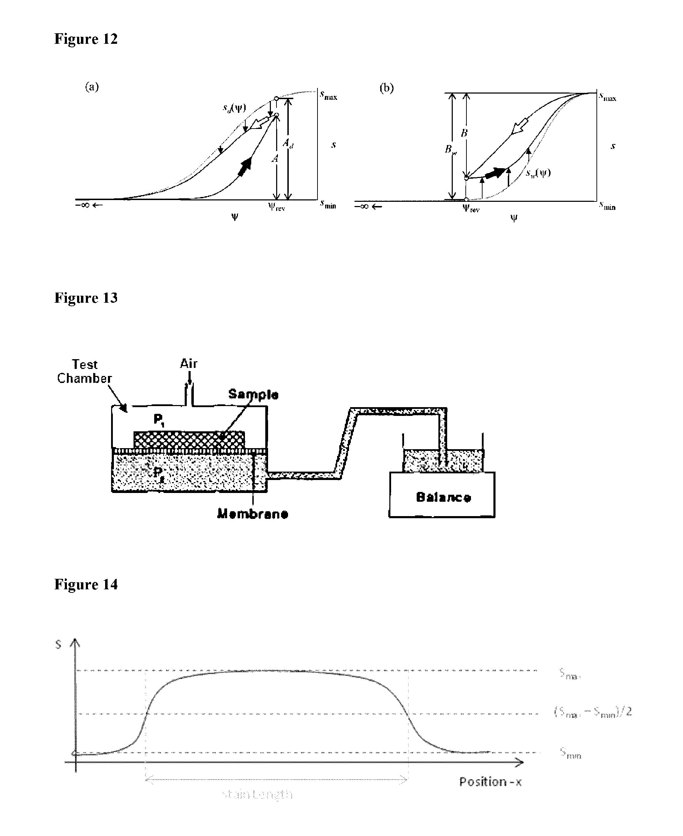

The saturation relationship $s(\Psi)$ implies a strongly hysteretic behavior, represented by a main drying curve and main wetting curve, as shown in Figures 12 a) and b).

Empirical representations of $s(\Psi)$ (e.g., VG) typically predict an effective saturation $s_e(\Psi)$ via an expression involving some parameter vector $\boldsymbol{p}$,

$$\frac{s(\psi) - s_{\min}}{s_{\max} - s_{\min}} = s_e(\psi) = f(\psi, \boldsymbol{p})$$

(E30)

where $s_{\min}$ and $s_{\max}$ denote the minimum (i.e., residual) and maximum saturation values, respectively, for a given material. If a particular node reverses from wetting to drying, the main drying curve is scaled by changing the maximum saturation value for that curve such that the reversal point falls on the resulting curve. Analogously, if the reversal is in the opposite direction, the main wetting curve is scaled by changing the minimum saturation value. An individual scanning curve is maintained for each node.

Assuming a maximum saturation $s_{\max}$ common to both main curves and assuming an asymptotic minimum saturation $s_{\min}$ also common to both main curves, the reversal point $\Psi_{rev}$ is used to define a linear scaling according to Figure 12. The following is the equation for reversal from wetting to drying

$$s(\psi_{rev}) - s_{\min} = c_d [s_d(\psi_{rev}) - s_{\min}]$$

$$c_d = \frac{A}{A_d} = \frac{s(\psi_{rev}) - s_{\min}}{s_d(\psi_{rev}) - s_{\min}}$$

and for the reversal from drying to wetting

$$s_{\max} - s(\psi_{\mathrm{rev}}) = c_w [s_{\max} - s_w(\psi_{\mathrm{rev}})]$$

$$c_w = \frac{B}{B_w} = \frac{s_{\max} - s(\psi_{\mathrm{rev}})}{s_{\max} - s_w(\psi_{\mathrm{rev}})}$$

(E31 and E32,

respectively)

where $c_d$ and $c_w$ represent correction factors. The required scanning curves are then defined by:

$S_d{}^*(\Psi) = c_d s_d(\Psi) + (1-c_d) s_{\min}$ for $\Psi < \Psi_{\mathrm{rev}}$ for drying;
and:
$S_w{}^*(\Psi) = c_w s_w(\Psi) + (1-c_w) s_{\max}$ for $\Psi_{\mathrm{rev}} < \Psi <_0$ for wetting.

Nomenclature for equations (E14) to (E32):

AGM - refers to the fluid-swellable solid material, e.g. including absorbent gelling material, as described herein above.
AF- refers to airfelt material or any other non fluid-swellable component of the fluid-swellable solid material, e.g. glues, polymers, etc.

| | | |
|---|---|---|
| $a_i$ | 1 | direction vector at node $i$: |
| $b$ | $L$ | height; |
| $C$ | $ML^{-3}$ | intrinsic concentration. |
| $\overline{C}$ | $ML^{-3}$ | bulk concentration: |
| $d$ | | error vector; |
| $d$ | 1 | volumetric stolid strain: |
| $E$ | | error vector; |
| $e$ | 1 | gravitational unit vector; |
| $f$ | | generalized flux vector; |
| $h$ | $L$ | $= z + \Psi$ - hydraulic head of liquid: |
| $K$ | $LT^{-1}$ | hydraulic conductivity tensor; |
| $k_r$ | 1 | relative permeability, |
| $J^s$ | 1 | Jacobian of solid domain, volume dilatation function, |
| $L$ | | partial differential equation operator; |
| $I$ | $L$ | characteristic element length, |
| $m_1^s$ | 1 | AGM $x$-load. |
| $m_{\max}^s$ | 1 | maximum AGM $x$-load: |
| $p$ | | parameter vector, |
| $Q$ | $T^{-1}$ | volumetric flow rate; |
| $R$ | $L^3 T^{-1}$ | residual vector: |
| $R$ | $ML^{-3}T^{-1}$ | kinetic reaction term: |
| $s$ | 1 | saturation, |
| $t$ | $T$ | time; |
| $u$ | $L$ | scalar solid displacement norm, |
| $w$ | $L$ | width: |
| $x$ | $L$ | spatial coordinate vector: |
| $z$ | $L$ | vertical coordinate; |
| *reek letters* | | |
| $\beta_{\mathrm{upwind}}$ | $L$ | upwind parameter: |
| $\Gamma$ | $L^2$ | closed boundary |

(continued)

| | | | |
|---|---|---|---|
| $\gamma$ | $L^{-1}$ | specific liquid compressibility, |
| $\Delta$ | | increment or difference: |
| $\delta$ | **1** | temporal error tolerance; |
| $\epsilon$ | **1** | porosity, void space; |
| $\eta$ | $L^3 T^{-1}$ | residual error tolerance; |
| $\kappa$ | $L^{-1} T$ | artificial compression of solid, |
| $\zeta$ | **1** | mesh refinement error criterion: |
| $\sigma^{\circ}$ | $L$ | artificial (dampening) 'diffusive' stress of solid; |
| $\tau$ | $T^{-1}$ | AGM reaction (speed) rate constant; |
| $\phi$ | | state variable vector; |
| $\Psi$ | $L$ | pressure head of liquid; |
| $\Omega$ | $L^3$ | domain, |
| $\nabla$ | $L^{-1}$ | Nabla (vector) operator (= grad); |
| $d$ | | drying: |
| $e$ | | effective; |
| $H_2O$ | | water; |
| $i$ | | nodal index; |
| max | | maximum, |
| min | | minimum, |
| $n$ | | time plane: |
| $o$ | | initial: |
| rev | | reversal: |
| $w$ | | wetting; |

| | |
|---|---|
| *Superscripts* | |
| $c$ | convective; |
| $D$ | number of space dimension: |
| $d$ | diffusive; |
| $L$ | left; |
| $P$ | predictor; |
| $R$ | right; |
| $s$ | solid phase; |
| $T$ | transpose; |
| $\tau$ | iteration counter; |
| *Abbreviations* | |
| AGM | absorbent gelling material: |
| AMR | adaptive mesh refinement; |
| IFM | interface manager: |
| RHS | right-hand side; |
| RMS | root-mean square; |
| SIA. | sequential iterative approach: |
| VG | van Genuchten: |
| 2D | two dimensions or two-dimensional; |
| 3D | three dimensions or three-dimensional; |
| ()·() | vector dot (scalar) product, |
| ()⊗() | tensor (dyadic) product, |

The equations E1 to E32 describing the virtual test environment 22, as shown in Figure 6, which is a schematic representation of said virtual test environment 22, can be solved using direct methods, iterative methods, or any other methods known to those skilled in the art. For example, as done in the examples of current invention, they can be implemented and solved using FeFlow Software by DHI-WASY GmbH (Walterdorfer Str. 105, 12526 Berlin Germany) customized trough a specific proprietary plug-in to implement the constitutive equations that describe the swelling behavior shown in (E2 a to v). Herein this plug-in can be referred as FeFlow plug-in, FeFlow module or AGM module.

Following FeFlow manuals, the skilled person can develop a suitable plug-in to implement the constitutive equations that describe the swelling behavior shown in (E2 a to v).

For the current invention, the simulation model is, in addition to solutions of the equations, comprised of further virtual test environments, as illustrated in Figure 6.

For the current invention, the spatial domain of the absorbent core structure or absorbent article is specified as a series of virtual layers stacked on top of each other and representing the different layers of the absorbent core structure from just below the fluid permeable topsheet to just above the typically fluid impermeable backsheet so to mimic a standard flat acquisition experiment.

For the current inventions the different composite material(s) used, namely the first or substrate layer, the layer of absorbent polymer material plus the layer of adhesive, and the second or cover layer plus any additional porous media layer that might be present are represented by a stack of virtual layers.

The virtual layers (that can be represented as rectangles in the two-dimensional model) have the thickness (height of the rectangle) equal to the thickness of the corresponding composite materials of the absorbent core structure or absorbent article measured with a confining pressure of 0.25 psi, and the length (length of the rectangle) equal to the length of the corresponding composite material of the absorbent core structure or absorbent article which is typically measured along the longitudinal axis of core structure or absorbent article. Thickness and length can be measured with any suitable technique as known in the art.

The third dimension is only parametric therefore its value is not critical for the simulation system and can assume any convenient value. The software arbitrarily assign a convenient value of 1m but any value would delivers the same numerical results once a proper pre and post processing described further below is applied to correct (rescale) all the volumes to the actual width of the absorbent core structure or absorbent article.

The two-dimensional domain of the virtual layers is divided into suitable (two dimensional) "volume" elements, which together form what is commonly referred to as the mesh, further exemplified in Figure 10 and 11. Each vertex of a mesh element is called node. The mesh can be coarse or fine, the choice of which requires consideration of the computing time for the virtual test environment 22 and the precision of results. As with most numerical models, the skilled person must weigh and consider the tradeoffs between the amount of computing time required, fineness of the mesh, and precision of results.

For the present invention it is selected a quadrangular regular mesh for each layer with dimension so to have at least 3 layers of cells in each non -swellable material and 5 for each fluid swellable material and to have an aspect ratio between 1 and 8 and alternatively an aspect ratio between 1.5 and 5. The aspect ratio is defmed as ratio of length and height of the mesh element.

Representative initial conditions of the absorbent core or layer to be simulated are also specified. The initial condition (IC) are defmed as the status of the system at time $t=t_0$.

For the present invention IC considers that initially the materials are partially wet therefore $m_1$ ( $t=t_0$,(x,z)) $\neq 0$ while the fluid-swellable solid material is completely non swollen therefore $m_2$ ( $t=t_0$,(x,z)) = 0. In particular the initial saturation of all the material (swellable and non swellable) is selected so to have an initial head of -0.5 m according to the different material Capillary pressure curves. This choice usually reflects in saturations below 5% and more frequently below 1%. This choice is meant to take into account the initial humidity of the material in standard storing conditions and guarantee a smoother numerics.

Boundary conditions (BC) define the type of liquid insult protocol applied to the system. The BC used in the present invention defines that fluid can neither enter nor leave the composite material at boundary areas of the fluid-swellable composite material except for the loading area.

On the loading area, which for the current invention is 3.14 cm$^2$ (equivalent to a circle of 2 cm diameter) and positioned in the middle of the product, on the surface of the absorbent core structure which is meant, in use within an absorbent product, to correspond to the wearer facing surface, a falling head condition is applied so to deliver 3 gushes of 4 g each starting at time t = to =0 with 20 minutes waiting time from the start of one gush to the start of the following. For falling head is intended a time variable head applied to the loading area nodes, that changes depending on the amount of liquid that needs to enter and the liquid already entered into the system. In practice it considers a column of liquid whose height is calculated by dividing the volume that still needs to enter the system by the application area according to the following equation.

$$\psi_{BC}(t) = \left. \left(V_t(t) - V_i(t)\right) \middle/ A \right. \qquad \text{(E33)}$$

Where $\Psi_{BC}(t)$ *is the BC head,* $V_t(t)$ is the volume that needs to be added at a given time according to the virtual test protocol, $V_i(t)$ the volume already infiltrated at a given time and A the BC area. Falling head BC is switched off once $\Psi_{BC}$ becomes zero. Falling head is possibly switched on again to apply the subsequent gush(es). Once falling head is off, the standard boundary condition is applied (no liquid entering or leaving the composite material).

As the 3$^{rd}$ dimension of the model is parametrical the boundary condition is actually applied to the two-dimensional domain in a line whose length is calculated by dividing the application area by the absorbent article or absorbent core structure width.

The arbitrary width (1m) selected for the simulation model has no effect on the BC length and head value because the actual volume of liquid applied in the simulation is rescaled as indicated below from the protocol one:

$$V_S = V_R \frac{W_S}{W_R} \qquad (E34)$$

where:

   $V_s$ Volume to be applied into the simulation model, $V_R$ volume of the gush, $W_S$ parametric width of the layer in the simulation system, $W_R$ width of the layer.

For the current invention we apply gushes of 4g of AMF that correspond to $V_R$ = 3.846 ml, while the parametric width Ws = 1 m:

Representative physical properties of the absorbent core or layer are permeability, capillary pressure, fluid-swellable composite swelling speed, fluid-swellable composite maximum capacity, porosity, fluid-swellable composite material concentration. Representative absorbent-fluid interaction properties 48 for the absorbent core or layer 46 are also specified and include parameters of capillary pressure as function of saturation, relative permeability as function of saturation; they include all the dependencies of permeability, capillary pressure, swelling speed, and porosity on Fluid swellable material (AGM) x-load.

Physical properties of the fluid 52 are also specified and include the fluid density, fluid viscosity as function of the applied stress. In the context of the present invention, these correspond to fluid density and fluid viscosity as function of the applied stress at 23˚C $\pm$ 2˚C of a reference Artificial Menstrual Fluid (AMF), prepared according to the enclosed formulation.

Specifically the density of such reference AMF is 1.04 g/cm$^3$, viscosity as a function of the applied stress is specified below.

Physical properties, dimensions and geometries of the fluid-swellable (composite) material (also referred to as absorbent core or absorbent structure, typically an absorbent core structure having a structure as illustrated in Figure 3) in the absorbent article are also specified for the virtual model of an absorbent article.

The physical properties of the composite material(s) of the absorbent core structure or absorbent article are obtained from direct measurements of the properties and curve-fitting with specific constitutive equations (in E2) as specified in the test methods section.

Solver conditions and convergence criteria as inserted in FeFlow for the current invention are here specified: i) start time (0 sec) ii) end time (3600 sec) iii) initial time-step (le-13 sec) iv) maximum time-step increase (1.2) v) time error tolerance (le-4), vi) residual error tolerance (le-2 ml/s). v) Solver for symmetric equation systems: PCG, vi) Solver for non- symmetric equation systems: BICGSTABP, vii) Unsaturated flow iteration scheme: PICARD.

If the model is implemented into a different software, the equivalent convergence parameters can be found by the skilled person via sensitivity study of the convergence parameters with the intent of increasing the prediction accuracy.

In the current invention the gravitational force is considered perpendicular to the layers and directed from the wearer-facing side, to the garment-facing side.

As relevant part of the results, the Virtual test environment 22 generates a virtual spatial map of saturation as a function of location as a function of time, a virtual spatial map of Fluid swellable material (AGM) x-load as a function of location as a function of time and the liquid head at loading area boundary as a function of time.

As known to the skilled person, properly integrating these values over the proper spatial domains allow calculating for example:

   1. Total liquid volume present into the composite material(s) pores per each layer,
   2. Total liquid volume absorbed into fluid-swellable solid material

In the specific implementation of current invention this integration is performed automatically by the above mentioned FeFlow plug-in, but can be solved with any suitable method known to the skilled person.

When FeFlow is executed, it creates output files as specified by the output controls containing the above results. Similar output files can be obtained also from other suitable solution methods and tools.

In addition the tailored FeFlow modules create additional output files reporting saturation, liquid content by layer and liquid content into fluid-swellable material.

For the current invention it is selected to report results every second and saturation is reported with a spatial definition of 5 mm.

This information is finally analyzed as specified into the post-processing section to calculate as final output of the virtual test environment 22, the Virtual Free Fluid at the selected time and the Virtual Acquisition Time at the selected gush.

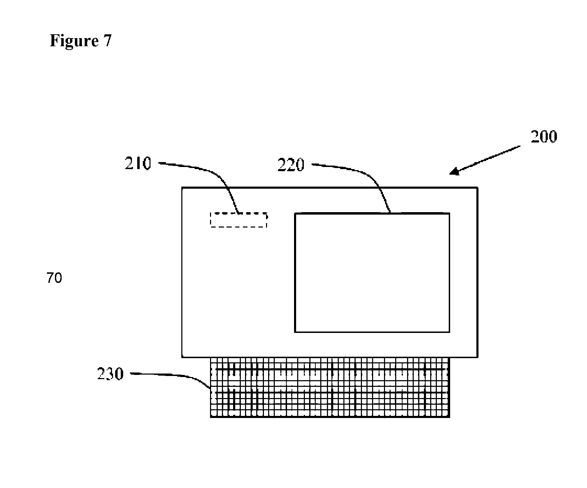

Figure 7 is a block diagram illustrating one example of a computer system 200 for operating the virtual test environment 22 and the virtual model of an absorbent article. The computer system 200 comprises a central processing unit 210, a graphical user interface 220 including a display communicatively coupled to the central processing unit 210, and a user interface selection device 230 communicatively coupled to the central processing unit 210. The user interface selection device 230 is used to input data and information into the central processing unit 210. The central processing unit 210 can include or has access to memory or data storage units, e.g., hard drive(s), compact disk(s), tape drive(s), and similar memory or data storage units for storing various data and inputs which can be accessed and used in operating the virtual test environment 22 and the virtual model of an absorbent article. For the current invention, the Central processing unit 210 is part of a Dell workstation using INTEL® PC architecture with an Intel Xeon dual core CPU at 2.8 gHz and running a MICROSOFT WINDOWS® XP professional 64bit operating system. In the machine is installed the 64-bit version of FeFlow 5.4(p10).

Post-processing

According to the setting specified (time resolution 1s, spatial resolution 5mm for the current invention) the simulation system creates output files reporting also the following relevant information:

1. Liquid head in the loading area boundary as function of time
2. Saturation in each layer at each length (x-dimension) averaged across the thickness of the layer (z-dimension) as function of time
3. Volume of liquid in pores void for each layer as function of time
4. Volume of liquid in Fluid swellable material (AGM) as function of time

Post-processing is meant to calculate relevant simulation final output (VFF and VAT) from the above data.

VAT is defined as the time required for absorbing a gush. It is calculated from the Liquid head in the loading area boundary as a function of time. It is the difference from the time the gush ends ($t_f$, identified as the time at which liquid head reaches the value of 0) and the time the gush starts ($t_i$ identified by the protocol)

$$VAT = t_f - t_i \qquad (E35)$$

VFF is a time dependent property therefore once reporting VFF it is always necessary to report the correspondent time (i.e. VFF at 10 minute). VFF at a given time is defined as the difference from the total liquid that is entered into the structure at that time (virtual total volume , $V_T$), the liquid which is absorbed by the Fluid swellable material (AGM) at that time ($V_A$) and the liquid that absorbed by the non swelling fraction of the composite material ($V_F$)

$$VFF(t) = V_T(t) - (V_A(t) + V_F(t)) \qquad (E36\text{-}a)$$

$V_T$ is defined as the sum of $V_A$ and the virtual pore volume $V_P$, therefore

$$VFF(t) = V_P(t) - V_F(t) \qquad (E36\text{-}b)$$

$V_P$ is directly reported for each layer by the simulation system.

$V_F$ as a function of time is calculated multiplying the virtual stain area ($A_{st}$) of each layer (fluid swellable composite material or non swellable composite material) as a function of time by the non-swellable basis weight ($BW_{NS}$) by the non-swellable material retention ($ret_{NS}$) then summing over all the layers.

$$V_F(t) = \sum_{i=1}^{n_{layers}} A_{st_i}(i) \cdot BW_{NS_i} \cdot ret_{NS_i} \qquad (E36\text{-}c)$$

As defined above by $BW_{NS}$ it is intended the total basis weight of the material minus the fluid-swellable basis weight ($BW_{SM}$) (e.g. the basis weight of the fluid-swellable solid material, typically AGM).
The $BW_{NS}$ can be then further split in two contributes: the absorbing non swellable basis weight ($BW_{NSAF}$) and the non absorbing non swellable basis weight ($BW_{NSNA}$).
In the context of the current invention $BW_{NSAF}$ is tipically basis weight of non synthetic fiber such as cellulose or rayon, while $BW_{NSNA}$ is typically the basis weight of synthetic fibers such as PP, PET, or of the hot melt adhesives.
The non swellable material retention ($ret_{NS}$) is the amount of liquid that is blocked into the pores of a structure. It is liquid that is not into the fluid swellable solid material but anyway can't leave the structure as strongly bound to the porous structure.
$ret_{NS}$ is expressed in g/g and is calculated as follows:

$$ret_{NS} = \begin{cases} 0 & BW_{NS} = 0 \\ \dfrac{BW_{NSAF} \cdot 1.5}{BW_{NS}} & BW_{NS} > 0 \end{cases} \qquad (E36\text{-}d)$$

the factor 1.5 is assumed to be the absorption in g/g of the absorbing non swellable portion of a material. In the context of the current invention it is considered constant across the different composite materials.
Combining (E36-c) and (E36-d) it is possible to simplify the definition of $V_F(t)$ as follows:

$$.V_F(t) = \sum_{i=1}^{n_{layers}} A_{st_i}(i) \cdot BW_{NSAF_i} \cdot 1.5 \qquad (E36\text{-}c)$$

For example, according to the current invention $BW_{NSAF}$ can be equal to 0 for the absorbent polymer layer while it can be equal to the non synthetic fiber (e.g. cellulose, rayon etc.) basis weight for the other layers, as illustrated in the examples.
The Stain area as a function of time is calculated by the saturation profile for each layer.
As the system is 2D, the Stain area is defined as the stain length times the product Width ($W_R$) The Stain length is calculated as follow according with Figure 14:
Saturation profile data are searched for the maximum ($S_{max}$) and the minimum ($S_{min}$) saturation values, a medium saturation ($S_{mid}$) is then calculated as ($S_{max}$ + $S_{min}$)/2, As the system provides Saturation profile for discrete intervals a linear interpolation of the data is adopted. At this point scanning saturation profile form one side to the other of the product it is searched the position at which saturation raise to a value equal to $S_{mid}$ this point is considered the Stain starting point. In case saturation at the starting point (x=0) is already above $S_{mid}$ the stain reached the edge therefore the starting point is assumed to be zero. Continue scanning the saturation profile a position at which Saturation drops below $S_{mid}$ is searched, this point is considered the stain end point. In case at the final point (x=length) the saturation is above $S_{mid}$ the stain reached the edge therefore the end point is assumed as the product length. Stain length is the difference of End point and initial point coordinates.
In the context of the present invention the simulation model herein is used to predict the performance of an absorbent core structure containing fluid-swellable composite material as that illustrated as such in the sectional view of Figure 3, and also illustrated in Figures 1 and 2 as comprised within a sanitary napkin, being typically represented in a 2D flat geometry representation, such as exemplified in Figure 8. The absorbent core structure is in the form of a layered structure typically comprising different materials, namely the substrate layer 100, the layer of absorbent polymer material 110, the layer of adhesive, typically hot melt adhesive 120, and the cover layer 130. As said above, the simulation model is run in order to provide as output, according to the present invention, the Free Fluid at 20 min and 60 min and the Acquisition Time at the 2nd gush and at the 3rd gush of the fluid-swellable composite material, which can be hence called Virtual Free Fluid at 20 min and Virtual Free Fluid at 60 min and Virtual Acquisition Time at the 2nd gush and Virtual Acquisition Time at the 3rd gush, simulating the application of three gushes of 4 g each of Artificial Menstrual Fluid. The 1st gush is applied at time t=0, the 2nd gush is applied at time t=10 min, and the 3rd gush is applied at time t=20 min. The Virtual Free fluid is calculated at time t=1 min, at time t=20 min, immediately before the application of the 3rd gush, and at time t=60 min, which can be considered to represent an equilibrium condition

In order to implement the model, and actually run the simulation of the absorbent core structure comprising fluid-swellable composite material and obtain the output, namely the Virtual Free Fluid and the Virtual Acquisition Time of the absorbent core structure comprising fluid-swellable composite material as stated above, it is necessary to obtain certain properties of the composite materials constituting the absorbent core structure comprising and namely:

1. Porosity ($\varepsilon$).
2. Fluid-swellable solid material (e.g. AGM) speed rate constant ($\tau$):

3. Maximum fluid-swellable solid material (e.g. AGM) x-load $m^s_{\max}$

4. Swellable solid material concentration
5. Permeability (k);
6. Capillary pressure ($p_c$):

As explained, most of the above properties are saturation dependent and most of them might also change with the swelling, i.e. the x-load of the fluid-swellable composite material, therefore the test methods to determine the above properties might change if the material is fluid-swellable or non swellable as specified for each property below

Fluid-swellable material typically corresponds to the absorbent polymer material as such, however for fluid-swellable materials the above properties are typically referred to the fluid-swellable composite material, which typically corresponds to the layer of absorbent polymer material 110, the layer of adhesive 120, and possibly fibres and/or inert materials if present in the layer of absorbent polymer material.

These dependencies on x-load and saturation also mean that the above properties are not identified by a single number coming from a single experimental determination but by an experimental curve. To make sure this behavior is well captured; constitutive equations leading to multiple parameters are used as shown in E2.

To determine the values of the parameters of the constitutive equations any known fitting technique of the experimental value with the above constitutive equation might be used. For example iterative techniques based on standard least square principle. This can easily be performed for example by using the "Solver" tool in Microsoft® Excel or the "modeling nonlinear tool in JMP®.

It is known to the person skilled in the art, that all of the properties above also depend on the confining pressure applied onto the sample during the measurement; the standard confining pressure is intended to be 0.25 psi if not stated otherwise as this resembles the experimental condition that the system needs to simulate.

Menses simulating fluids such as AMF are known to be Non Newtonian, therefore the viscosity needs to be provided as function of stress. Within a given porous media the stress is related to the hydraulic head gradient therefore to describe the shear thinning behavior the semi empirical constitutive equation specified in E2-v is selected. For the reference AMF used in the current invention its parameters are:

$$\mu_0 = 7.2 \ \text{cpoise}$$

$$\mu_r = 3.00\text{E-}05$$

$$\mu_\sigma = 0.002$$

$$\mu_e = -1.456$$

$$\mu_k = 0.014$$

Thickness

The thickness of a layer of the absorbent core structure according to the present invention, as well as of a combinations of layers, for example of an entire absorbent core structure, can be measured with any available method known to the skilled person under the selected confining pressure of 0.25 ± 0.01 psi. For example, the INDA standard test method WSP 120.1 (05) can be used, wherein for the "Thickness testing gage" described under section 5.1, the "applied force", section 5.1.e, is set at 0.25 ± 0.01 psi, and the "Readability", section 5.1.f, has to be 0.01 mm

Porosity (ε)

Porosity of a specific composite material, typically a material constituting the absorbent core structure according to the present invention, is the void volume fraction of the total volume of a material. In the context of the present invention porosity can be referred to non fluid-swellable materials and to fluid-swellable materials, which are both present in the absorbent core structure.

For non swellable materials the porosity can be easily calculated knowing the composition, the thickness under the desired confining pressure (i.e. 0.25 psi) and the bulk density of each single component according the following equation.

$$\varepsilon = 1 - \sum_i \frac{BW_i}{\rho_i \cdot B} \qquad (E37)$$

Where $i$ is the index counting over all the component, $BW_i$ is the Basis Weight of a specific component, $\rho_i$ is the bulk density of a specific component and B the thickness of the material under the desired confining pressure. The thickness can be measured with any available technique known by the skilled person under the selected confining pressure, for example following the method as said above. For non fluid-swellable material the porosity of the material is described into the model by only one parameter ($\varepsilon$).

For fluid-swellable materials the porosity depends on the Fluid swellable material (AGM) x-load, therefore measurements at different swelling extent is necessary. Data of porosity as a function of the fluid-swellable composite material x-load ( $m_2^s$ ) are measured with the Porosity vs. load method specified below and then fitted with the constitutive equation:

$$\varepsilon = \frac{2 \varepsilon_{max}}{1 + (m_2^s \varepsilon_{scale} + 1)^{\varepsilon_{exp}}} \qquad (E38)$$

In the equation above $\varepsilon_{max}$, $\varepsilon_{scale}$ and $\varepsilon_{exp}$ are fitting parameters, to be determined with fitting methods, known in the art and represent the input the model requires.

Fluid swellable material (AGM) speed rate constant ($\tau$) and Maximum Fluid swellable material (AGM) x-load ( $m_{max}^s$ )

Fluid swellable material speed rate constant and Maximum Fluid swellable material x-load are properties dependent on the type of fluid-swellable solid material used, they describe the kinetics and the retention of the swellable solid material according to the following equations:

$$\frac{\partial \overline{C}_{L \to So}^s}{\partial t} = \tau \ a^{sl}(\hat{s}(s_e^l)) \dot{m}_2^s \overline{C}_{AGMo}^s - f^s \phi^s(u^s) \qquad (E39\text{-}a)$$

$$\hat{m}_2^s = \frac{m_{2max}^s - m_2^s}{m_{2max}^s} \qquad \text{(E39-b)}$$

$$a^{sl}(\hat{s}(s_e^l)) = \frac{1 - e^{-\beta_{kinexp}\hat{s}}}{1 - e^{-\beta_{kinexp}}} \qquad \text{(E39-c)}$$

$$\hat{s}(s_e^l) = \frac{s_e^l - s_{e,\text{threshold}}^l}{1 - s_{e,\text{threshold}}^l} \qquad \text{(E39-d)}$$

$$\tau = \tau_0 e^{\left(-b\frac{m_2^s(t)}{m_{2max}^s}\right)} \qquad \text{(E39-e)}$$

The parameter $a^{sl}(\hat{s}(s_e^l))$, takes into account the absorption from a partially saturated media, its parameters ($\beta_{kinexp}$ and $s_{e,\text{threshold}}^l$ are assumed to be 2.0 and 0.18 respectively for the current invention.

For a fully immersed sample the relation of x-load (uptake) over time becomes the following:

$$\frac{dm_2^s(t)}{dt} = \left(\tau_0 e^{\left(-b\frac{m_2^s(t)}{m_{2max}^s}\right)}\right)\frac{m_{2max}^s - m_2^s(t)}{m_{2max}^s} \qquad \text{(E40)}$$

This simplified equation allows the calculation of the remaining parameters.

The M-CRC method is used to measure the fluid-swellable material uptake at different times, which is then to be fitted with equation (E40). Equation (E40) is a differential equation and its integration is not trivial therefore experimental data can be fitted applying the standard least square principle iteratively with the numerical solution of the differential equation as known by the skilled person. As example, a convenient tool to perform this task is the Estimations tool of gPROMS®.

Fitting the equation above allows getting the unknown parameters needed for the simulation: i) the Fluid swellable material speed rate constant at zero load ($\tau_0$), ii) the poisoning factor (b) and iii) Maximum Fluid swellable material x-load ( $m_{2max}^s$ ).

Swellable solid material concentration (C$^s$<sub>AGM0</sub>)

This parameter corresponds to the concentration of the Fluid swellable solid material (e.g. AGM) in the fluid-swellable composite material and is not measured but it is easily calculated dividing the Fluid swellable material (AGM) amount by the total volume of the fluid-swellable composite material.

Permeability (k)

Permeability of a specific composite material, typically a material constituting the absorbent core structure according to the present invention is an important property to determine the ability of such material to allow fluid movement.

For a fluid-swellable (composite) material it is in general a function (*f*) of fluid-swellable composite material x-load ( $m_2^s$ ) and saturations $s_l^e$ ,

$$Permeabili\,ty = f(s_l^e, m_2^s) = \mathsf{k}(m_2^s) \cdot k_r^l(s_l^e, m_2^s) \qquad \text{(E41-a)}$$

For the non swellable material the permeability is function (*f*) of the saturation $s_l^e$

$$Permeability = f(s_l^e) = \mathsf{k} \cdot k_r^l(s_l^e) \qquad \text{(E41-b)}$$

where

$k$ is the saturated permeability

$k_r^l$ is the relative permeability

In general permeability is a tensor, i.e. the components in the different directions, should be considered, as described in the model equations. For the current invention the permeability is considered isotropic therefore the same value is assigned in all the directions.

It is described in more detail in the literature on the dependency of the permeability on the swelling extent of the porous media, e.g. Model of Liquid Permeability in Swollen Composites of Superabsorbent Polymer and Fiber, Fredric L. Buchholz Dow Chemical Company, Journal of Applied Polymer Science, Vol. 102, 4075-4084 (2006).

This complex dependency from saturation and eventually x-load, is handled in the model by assuming that the dependence of saturation $s_l^e$ follows the equation:

$$k_r^l = (s_e^l)^\delta \qquad \text{(E42)}$$

and that for fluid-swellable composite materials permeability depends on fluid-swellable composite material load $m_2^s$ according the following equation:

$$K = \frac{k\rho_o^l g}{\mu^l} = K_{\text{base}} I[1 - k_{\text{coeff}} \exp(k_{\text{expcoeff}} \hat{m}_2^s) \sin(2\pi k_{\text{sinecoeff}} \hat{m}_2^s + k_{\text{sinephase}})] \qquad \text{(E43-a)}$$

$$K_{\text{base}} = \frac{k_{\text{base}} \rho_o^l g}{\mu^l} \qquad \text{(E43-b)}$$

Hereby, ***K*** being the conductivity; *k* being the permeability, $\rho$ being the density, g being the gravitational acceleration, and $\mu$ being the viscosity, ***I*** being the unity vector, as described herein and/ or as defined in the first nomenclature list above.

For non-swellable composite materials the value of k can be obtained directly using the IPRP for Non-Swelling Samples test.

K, the actual model input for non swellable materials can be calculated as indicated by (E43-b) from the value of k.

For fluid-swellable composite material the dependency of k as function of x-load $m_2^s$ can be obtained using the IPRP for Swelling Samples test and the interpolation of the Centrifuge Retention Capacity (CRC). In fact, using E40 on CRC data, it is possible to know the AGM load at each time. As IPRP for Swelling Samples test provides the permeability after different imbibition times, while the fitting of CRC provides the x-load at different times, it is possible to create the relationship between permeability and the material x-load.

The fitting of the data with the equations (E43-a) allows calculating the model input parameters ($k_{base}$, $k_{coeff}$, $k_{expcoeff}$, $k_{sinecoeff}$, $k_{sinephase}$)-$K_{base}$, the actual model input for fluid-swellable materials can be calculated as indicated by E43 from the value of $k_{base}$.

In the formula (E42) above, the relative permeability is described through a power model, where the coefficient δ can be estimated based on the literature values. For the current invention δ is assumed to be 4 for the non swellable materials and 2.8 for the liquid-swellable materials.

Capillary Pressure (Pc)

Capillary pressure of a specific composite material, typically a material constituting the absorbent core structure according to the present invention is herein given as a complex function of saturation and possibly fluid Swellable material x-load both for liquid uptake (also called absorption or wetting) and liquid retention (also called desorption or drying). Uptake and retention regimes can be conveniently expressed as follow:

$$\text{Uptake: } \frac{\partial \psi^l}{\partial t} > 0 \qquad\qquad \text{Retention: } \frac{\partial \psi^l}{\partial t} < 0$$

To handle this complex dependency in a model, it is herein assumed the following equations

$$s_e^l = \frac{s^l - s_r^l}{s_s^l - s_r^l} = \begin{cases} \dfrac{1}{[1 + |\alpha(m_2^s)\psi^l|^n]^m} & \psi^l < 0 \\ 1 & \psi^l \geq 0 \end{cases} \qquad (E44)$$

With a different set of parameters for Uptake and Retention regimes.

For a fluid-swellable (composite) material moreover the parameter α is function of the composite material x-load ( $m_2^s$ ) as follow.

$$\alpha(m_2^s) = \begin{cases} \alpha_{max} & m_2^s \leq m_{2,\,threshold}^s \\ \dfrac{\alpha_{max}}{[1 + \alpha_{scale}(m_2^s - m_{2,\,threshold}^s)]^{\alpha_{exp}}} & m_2^s > m_{2,\,threshold}^s \end{cases} \qquad (E45\text{-}a)$$

$$\alpha_{max} = \begin{cases} \alpha_{max,wetting} & \dfrac{\partial \psi^l}{\partial t} > 0 \\ \alpha_{max,drying} & \dfrac{\partial \psi^l}{\partial t} < 0 \end{cases} \qquad (E45\text{-}b)$$

The parameters in equations (E44) and (E45), namely $s_r^1$ $s_s^1$, n, m $\alpha_{max}$, $\alpha_{scale}$, $\alpha_{exp}$, $m^s_{2,threshold}$ can be determined from fitting experimental capillary pressure curves at different Fluid swellable material (AGM) x-load, both for uptake and retention curves.

Capillary pressure at a given load is measured according the test method specified in the below section repeating the test at different loads, the full data set is collected and analyzed at once.

For simplicity, as their effect on the final result is somehow limited in the ranges of interest, for the current invention the values of $s_r^1$, $s_s^1$ both for uptake and retention curves are assumed to be 0 and 1 respectively.

As the capillary pressure Vs saturation curve of a composite material is defined in the model by so many parameters each of which describing fine details of the whole curve, it is unpractical to compare the different materials using the full lot of parameters. Moreover many of the resulting performances are explained by the overall sucking force of the material and not by the fine details therefore a practical comprehensive parameter is defined to describe the capillarity of a material. This parameter is the Medium Absorption Pressure (MAP). It is clear that MAP is a specific property of the interaction of the material and the liquid, it is therefore necessary to specify the liquid in which MAP is measured. For the current invention the MAP is intended as measured in AMF if not stated otherwise. MAP of a material in a specific liquid, is defined as the capillary pressure of the uptake curve at 50% saturation (measured in the specific liquid). Dimensionally MAP is a pressure and can be conveniently expressed in m $H_2O$ (independently of the liquid considered). For non-swellable (composite) materials it is easily calculated with linear interpolation of the Capillary pressure data. For fluid swellable (composite) materials a different MAP exists for each x-load level. In case x-load is not explicitly specified, MAP is intended at the equilibrium load provided by an excess of 0.9% wt saline solution. At each specific Swellable material x-load MAP is easily calculated with linear interpolation of the Capillary pressure data at that specific x-load.

Similarly a parameter call Medium Desorption Pressure (MDP) can be defined for the retention curve.

Overall, for each material the following parameters determined as explained above are required:

| | Fluid-swellable composite material | NON-swellable composite material |
|---|---|---|
| Porosity | $\varepsilon_{max}$ | $\varepsilon$ |
| | $\varepsilon_{scale}$ | |
| | $\varepsilon_{exp}$ | |
| Permeability | $k_{base}$ | $k$ |
| | $k_{coeff}$ | |
| | $k_{expcoeff}$ | |
| | $k_{sinecoeff}$ | |
| | $k_{sinephase}$ | |
| | $\delta (= 2.8)$ | $\delta (= 4)$ |
| Capillary pressure Uptake Curve | $s^l_s$ | $s^l_s$ |
| | $s^l_r$ | $s^l_r$ |
| | $\alpha_{max}$ | $\alpha$ |
| | $n$ | $n$ |
| | $m$ | $m$ |
| Capillary pressure Retention Curve | $s^l_s$ | $s^l_s$ |
| | $s^l_r$ | $s^l_r$ |
| | $\alpha_{max}$ | $\alpha$ |
| | $n$ | $n$ |
| | $m$ | $m$ |
| Capillary pressure Swelling effect | $m^s_{2,threshold}$ | / |
| | $\alpha_{scale}$ | |
| | $\alpha_{exp}$ | |
| Fluid swellable material Concentration | $C^s_{AGM0}$ | / |

(continued)

| | Fluid-swellable composite material | NON-swellable composite material |
|---|---|---|
| Speed rate constant and Maximum Fluid swellable material x-load | $m^s_{2max}$ | / |
| | $\tau_0$ | |
| | b | |
| | $\beta_{kinexp}(=2)$ | |
| | $s^l_{e,threshold}$ (=0.18) | |

Test Methods

Preparation of Artificial Menstrual Fluid (AMF)

Artificial Menstrual Fluid is based on modified sheep's blood that has been modified to ensure it closely resembles human menstrual fluid in viscosity, electrical conductivity, surface tension and appearance. It is prepared as explained in US Patent 6,417,424, assigned to The Procter & Gamble Company, from line 33 of column 17 to line 45 of column 18, to which reference is made.

Sample Preparation of Fluid Swellable Composite Material from Raw Materials

In the specific context of the present invention, the fluid-swellable composite material typically corresponds to the layer of absorbent polymer material 110 plus the layer of adhesive 120, and possibly fibres and/or inert materials if present in the layer of absorbent polymer material 110. In order to measure for the fluid-swellable composite material the parameters which are meant to represent the fluid-swellable composite material itself, and in turn be comprised in the input for the simulation model, a conventional layered material is prepared by comprising, actually sandwiching, the layer of absorbent polymer material 110, and possibly fibers and/or inert materials if present, and the layer of adhesive 120, between two layers of thin nonwoven, typically a 12 $g/m^2$ spunbonded hydrophilic (with Silastol PHP26 surfactant) polypropylene nonwoven available from Pegas Nonwovens s.r.o., Czech Republic, under the code 201201010100. It is believed that the presence of the nonwoven layers does not have any practical influence on the relevant parameters measured for the fluid-swellable composite material, and that in any case said influence can be ignored in running the simulation model. Actually, the conventional fluid-swellable composite material sample is prepared by providing between the two nonwoven layers with known means a layer of absorbent polymer material 110, plus any further material such as fibers and/or inert materials, as appropriate, and a layer of adhesive 120, in the same configuration, i.e., basis weight, distribution, etc., as in the actual product, also comprising any auxiliary adhesive, if present in the actual product. Samples of the desired shape and size as prescribed in the respective tests described herein are cut.

Sample Preparation of Fluid Swellable Composite Material from an Absorbent Article

When starting from an article comprising the absorbent core structure, in turn comprising the absorbent polymer material, typically in particles, said material can be isolated with known means, typically from the layer of thermoplastic material and the first layer, in order to be tested. Typically, in a disposable absorbent article the topsheet can be removed from the backsheet and the absorbent core can be separated from any additional layers, comprising the optional second layer, if present. The absorbent polymer material can be removed from the substrate layer and the layer of thermoplastic material, e.g. mechanically if possible, or by use of a suitable solvent, in case e.g. the thermoplastic material is a hot melt adhesive. Particles of absorbent polymer material can be hence isolated from other elements of the core e.g., by washing with a suitable solvent which does not interact with the absorbent polymer material, as can be readily determined by the man skilled in the art. The solvent is then let to evaporate and the fluid swellable absorbent polymer particles can be separated from the non swellable absorbent material, if present, with known means, and collected, for example from a plurality of articles of the same type, in the necessary amounts to prepare the samples as described above and to run the tests.

Sample Preparation of Non Swellable Materials from an Absorbent Article

With a similar procedure, component layers of an absorbent core structure, such as for example the first or substrate layer and the second or cover layer can be isolated from an absorbent article, in order to be tested, by carefully separating each layer from the other components of the absorbent core structure, for example mechanically freeing each layer from e.g. adhesive material, or alternatively by washing with a suitable solvent which does not interact with the materials of the respective layers. The solvent is then dried and the layers or the particles of absorbent polymer material can be collected, for example from a plurality of articles of the same type, in the necessary amounts to run the tests.

Viscosity

Viscosity can be determined with any viscosity method available in the art, e.g. ISO/TR 3666:1998 or DIN 53018-1:1976. Viscosity refers to the fluid used in the simulation model, typically Artificial Menstrual Fluid in the context of the present invention, measured with the suitable selected method at 23˚C± 2˚C.

Porosity Under Load

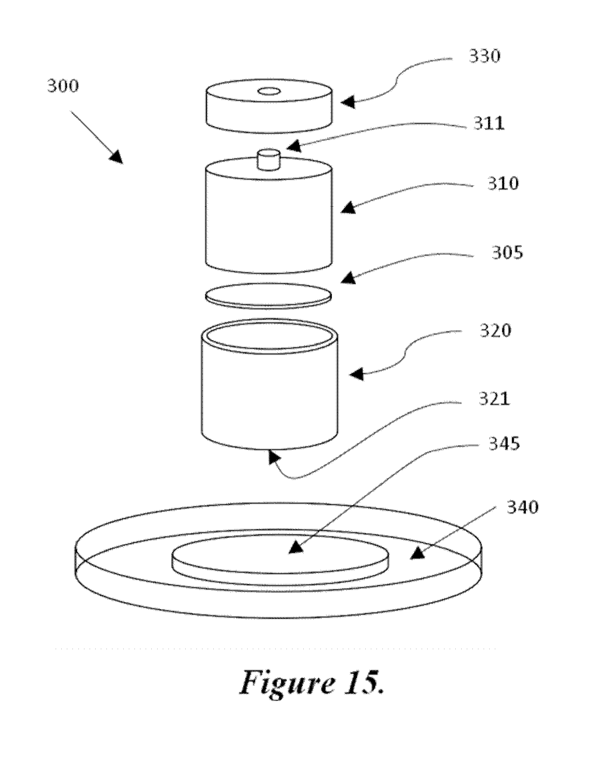

The porosity under load of a composite fluid-swellable material is measured as the ratio between the void volume and the total volume of a composite after uptake with an Artificial Menstrual Fluid (as described herein) under a confining pressure of 0.25 psi. Testing is performed at 23˚C± 2C˚ and a relative humidity 50% ± 5%. All samples are conditioned in this environment for twenty four (24) hours before testing. Referring to Figure 15, the cup assembly 300 consist of a cylinder 320 made of nylon material having an inner diameter of 58 mm, an outer diameter of 67 mm, and a height of 50 mm. A 400 mesh polyester screen 321 (e.g, Saatifil PES 18/13) which is also 67 mm in diameter, is glued to the bottom edges of the base of the cylinder 320, so that it spans the bottom end. A piston 310, made of the same nylon material has a diameter of 57 mm with a height of 57 mm. Additionally it has a milled protrusion 311 centered on top of the piston, which has a diameter of 16 mm and a height of 15 mm. The piston 310 should fit into cylinder 320 and be free to move vertically without binding. An annular stainless steel weight 330, having an outer diameter of 57 mm and an inner diameter of 16.4 mm, rest on top the piston 310 with the protrusion 311 fitting inside the hole of the weight 330. The mass of the weight 330 is such that the mass of the weight plus piston 310 is 430 g ± 0.5 g. Mass measurements are made with a top loading analytical balance with an accuracy of 0.01 g. Height measurements are made with a digital caliper which has a travel of 50 mm, an accuracy of 0.001 mm, and fitted with a rounded foot (e.g., Mitutoyo ID-C150B). Assemble the cylinder 320, piston 310 and weight 330 as shown in Figure 15 without a specimen 305. Measure and record the mass ($m_e$) of the cup assembly to the nearest 0.01 g. The caliper is affixed to a lab stand such that the cup assembly 300 can fit under the caliper foot. Place the cup assembly (without sample) under the caliper, lower the foot until it rest on the protrusion 311, then zero the caliper.

Die cut a 57 mm diameter disk of the dry composite. Measure and record the specimen's mass ($m_d$) to the nearest 0.01 g. Remove the weight 330 and piston 310 from the cylinder 320 and place the specimen 305 into the cylinder 320 resting flat on the bottom screen 321. Reinsert the piston 310 into the cylinder 320 and replace the weight 330. Place the cup assembly 300, with specimen 305 under the caliper. Lower the caliper foot until it rest on the protrusion 311 and record the specimen's thickness (B) to the nearest 0.001 mm

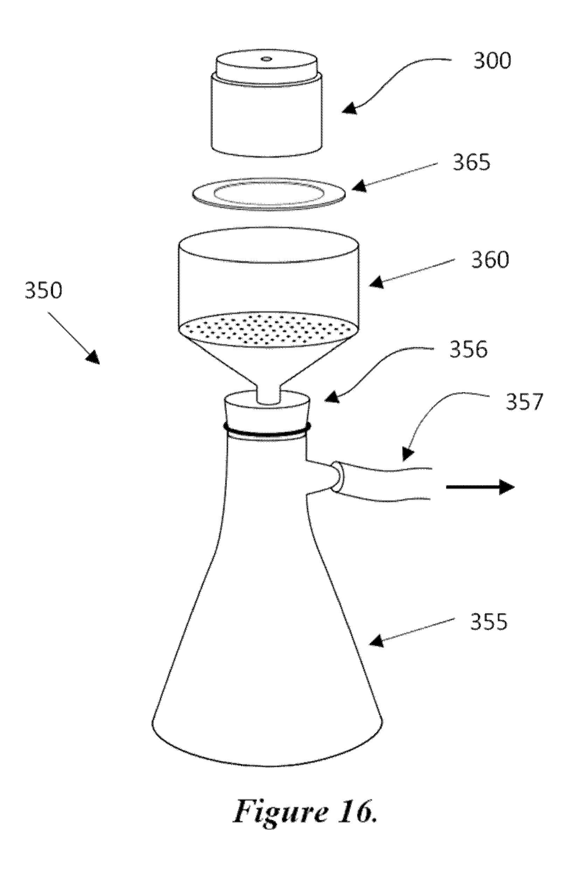

AMF is introduced into the specimen through a cycle of 1) uptake of AMF, 2) removal of excess AMF, and 3) swelling of the specimen. Referring to Figure 15, a Petri dish 340, 100 mm in diameter and 20 mm in height is used as a reservoir to introduce AMF into the specimen. A sintered glass frit 345, 90 mm in diameter, 5 mm in height, with a porosity grade of 1, is set in the center of the dish 340. The dish is then filled with AMF flush with the top surface of the frit 340. Referring to Figure 16, a vacuum assembly 350 is used to remove excess fluid from the specimen 305 after uptake of AMF. The vacuum assembly 350 consist of a 500 mL, side-arm, vacuum flask 355, fitted with a 365 mL capacity, 90 mm I.D. Buckner funnel 360. The funnel 360 is attached to the flask 355 using a 1-hole rubber stopper 356. An annular rubber gasket 365 with an outside diameter of 90 mm and an inside diameter of 60 mm is placed inside the Buckner funnel 360 to serve as a seal between the funnel bottom and the cylinder 320. The side-arm flask 355 is attached via vacuum tubing 357 to a vacuum pump (not shown) capable of providing a vacuum of 19 mm Hg.

Specifically, fill the Petri dish 340 with AMF until the level is flush with the top of the glass frit 345. Remove the piston 310 and the weight 330 from the cylinder 320. Place the cylinder 320 with specimen 305 onto the frit 345 to completely wet the specimen through the screen 321. Leave on frit until fluid can be visibly seen on top surface of specimen 305. Turn the vacuum source on and immediately place the cylinder 320 with specimen 305 into the vacuum assembly 350 for 5 seconds. Gently replace the piston 310 and weight 330 back into the cylinder 320 and continue to apply vacuum for an additional 10 ± 1 seconds. Release the vacuum and remove the cup assembly 300 from the vacuum assembly 350. Place the cup assembly on a clean bench top and allow the specimen 305 to swell for 10 min ± 10 sec. Place the assembly 300 with specimen under the caliper and measure the specimen's thickness (B) to the nearest 0.001 mm. Measure and record the total mass ($m_t$) of the cup assembly with specimen to the nearest 0.01 g. Subtract the mass ($m_e$) of the cup assembly 300 without the specimen from this total mass ($m_t$) to yield the final mass ($m_f$) of the specimen 305 after uptake and record to the nearest 0.01 g.

The following steps are repeated until a constant final mass ($m_f$) of the specimen 305 is obtained. Fill the Petri dish 340 with AMF until the level is flush with the top of the glass frit 345. Place the complete cup assembly 300 with specimen 305 (i.e., this time including piston 310 and weight 330) on the glass frit 345 for $3 \pm 1$ seconds to rewet the specimen 305 with AMF. Turn on the vacuum source and immediately place the assembly 300 on the vacuum assembly 350 and leave in place for $10 \pm 1$ seconds to remove the excess fluid. Release the vacuum and remove the cup assembly 300 from the vacuum assembly 350. Place the cup assembly 300 on a clean bench top and allow the specimen 305 to swell for 10 min 10 sec. Place the cup assembly 300 with specimen under the caliper, rest the caliper foot on the protrusion 311, then measure and record the specimen's thickness (B) to the nearest 0.001 mm. Measure and record the total mass ($m_t$) of the cup assembly with specimen to the nearest 0.01 g. Subtract the mass ($m_e$) of the cup assembly 300 without the specimen from this total mass ($m_t$) to yield the final mass ($m_f$) of the specimen 305 after uptake and record to the nearest 0.01 g. Compare the resulting final mass ($m_f$) to the previous final mass ($m_f$) and repeat if not within $\pm 0.02$ g. The porosity is calculated applying the following formula at each loading step:

$$\varepsilon = {^{V_V}}\!/\!{_{V_T}} \qquad\qquad \text{(E46-a)}$$

Where:

$$V_V = V_T - V_S \qquad\qquad \text{is the void volume (cm}^3) \qquad\qquad \text{(E46-b)}$$

$$V_T = A \cdot B \qquad\qquad \text{is the total volume (cm}^3) \qquad\qquad \text{(E46-c)}$$

$$V_s = \sum_i \frac{BW_i \cdot A}{10000 \cdot \rho_i} + \frac{\Delta m}{\rho_l} \qquad \text{for a wet sample is the solid volume (cm}^3) \quad \text{(E46-d)}$$

$$V_s = \sum_i \frac{BW_i \cdot A}{10000 \cdot \rho_i} \qquad \text{for a dry sample is the solid volume (cm}_3) \quad \text{(E46-e)}$$

| | |
|---|---|
| A | is the disk area (cm$^2$) |
| B | is the material thickness (cm) |
| i | is the index of all the material components |
| $\rho_i$ | is the material density of the component i (g/cm$^3$) |
| $\rho_l$ | is the liquid density (g/cm$^3$) |
| $BW_i$ | is the basis weight of the component i (g/m$^2$) |
| $\Delta m = m_f - m_e - m_d$ | is the weight increase (g)  (E46-f) |
| $m_f$ | is the final weight (g) |
| $m_e$ | is the equipment weight (g) |
| $m_d$ | dry material weight (g) |

The fluid swellable material x-load at each step is calculated as follows:

$$\text{x-load} = \Delta m / m_{AGM} \qquad (g/g) \qquad\qquad\qquad (E46\text{-}g)$$

where $m_{AGM}$ is the mass of the fluid swellable material.

Centrifuge Retention Rapacity CRC

The test is based on EDANA Test Method 441.2 02 (Centrifuge Retention Capacity). For the current invention the CRC is measured on the fluid swellable solid material which is used in the composite fluid swellable solid material. The present method determines the fluid retention capacity of fluid-swellable solid material (i.e. superabsorbent polyacrylate polymers according to the present invention) following centrifugation after immersion in 0.9% NaCl.

All materials are conditioned according to the requirements listed in EDANA Test Method 441.2 02 and references cited therein. 200 ml of a solution of 0.9% NaCl in distilled water is placed in a 600 ml beaker having an inner diameter of 95 mm. Four heat-sealed nonwoven sample bags each containing 0.200 ± 0.005 g of fluid-swellable solid material and four heat-sealed empty blank bags are prepared and weighed according to the directions in EDANA Test Method 441.2 02. As long as the bag materials and sealing conditions are unchanged, the use of historical data on the blanks may be considered. In this case, the tests on the four blank bags need not be carried out. The fluid-swellable solid material is distributed evenly throughout the sample bags with the bags held horizontally. Each bag is then placed on a flat plastic mesh support having 5mm square mesh pattern and the same dimensions as the nonwoven sample bag. A metal wire with an inverted U-shape is affixed to two opposite sides of the plastic mesh support to form a handle to facilitate immersing and withdrawing the bags from the 0.9% saline solution. Each bag is then immersed in the saline solution within the beaker by use of the wire handle, and a timer is started immediately upon immersion. The timer is accurate to within ± 1 second after 1 hour. Any entrapped air bubbles are removed by manipulating the bag in the solution. The bags are removed from the solution by use of the wire handle after the specific immersion time has elapsed. Each bag is then immediately removed from the plastic mesh support and is centrifuged at 250 G for 3 min ± 10 seconds as described in EDANA Test Method 441.2 02. The bags are then immediately removed from the centrifuge apparatus, weighed to within 0.001 grams, and the weight data are recorded.
The 0.9% NaCl is discarded after a maximum of four sample bags, and is replaced with fresh 0.9% NaCl solution. The procedure is repeated for the following immersion times: 1, 2.5, 5, 15, 30, 60 minutes.
Four sample replicates are used for each immersion time and the Centrifuge Retention Capacity value is taken as the average of the four values calculated as described in EDANA Test Method 441.202.

Menstrual Centrifuge Retention Capacity (CRC)

The test is based on EDANA Test Method 441.2 02 (Centrifuge Retention Capacity). For the current invention the CRC is measured on the fluid swellable solid material which is used in the composite fluid swellable solid material. The present method determines the fluid retention capacity of fluid-swellable solid material (i.e. superabsorbent polyacrylate polymers according to the present invention) following centrifugation after immersion in AMF.
All materials are conditioned according to the requirements listed in EDANA Test Method 441.2 02 and references cited therein. 200 ml of a solution of AMF is placed in a 600 ml beaker having an inner diameter of 95 mm. Four heat-sealed nonwoven sample bags each containing 0.200 ± 0.005 g of fluid-swellable solid material and four heat-sealed empty blank bags are prepared and weighed according to the directions in EDANA Test Method 441.2 02. As long as the bag materials and sealing conditions are unchanged, the use of historical data on the blanks may be considered. In this case, the tests on the four blank bags need not be carried out. The fluid-swellable solid material is distributed evenly throughout the sample bags with the bags held horizontally. Each bag is then placed on a flat plastic mesh support having 5 mm square mesh pattern and the same dimensions as the nonwoven sample bag. A metal wire with an inverted U-shape is affixed to two opposite sides of the plastic mesh support to form a handle to facilitate immersing and withdrawing the bags from the AMF. Each bag is then immersed in AMF within the beaker by use of the wire handle, and a timer is started immediately upon immersion. The timer is accurate to within ± 1 second after 1 hour. Any entrapped air bubbles are removed by manipulating the bag in the solution. The bags are removed from the solution by use of the wire handle after the specific immersion time has elapsed. Each bag is then immediately removed from the plastic mesh support and is centrifuged at 250 G for 3 min ± 10 seconds as described in EDANA Test Method 441.2 02. The bags are then immediately removed from the centrifuge apparatus, weighed to within ± 0.001 grams, and the weight data are recorded.
The AMF is discarded after a maximum of four sample bags, and is replaced with fresh AMF. The procedure is repeated for the following immersion times: 5, 15, 30, 60, 120, 240 minutes. Four sample replicates are used for each immersion time and the Centrifuge Retention Capacity value is taken as the average of the four values calculated as described in EDANA Test Method 441.202.

In Plane Radial Permeability (IPRP) for Non-Swelling Samples

This test is suitable for measurement of the In-Plane Radial Permeability (IPRP) of a porous material. The quantity of a saline solution (0.9% NaCl) flowing radially through an annular sample of the material under constant pressure is measured as a function of time.

Testing is performed at 23˚C $\pm$ 2C˚ and a relative humidity 50% $\pm$ 5%. All samples are conditioned in this environment for twenty four (24) hours before testing.

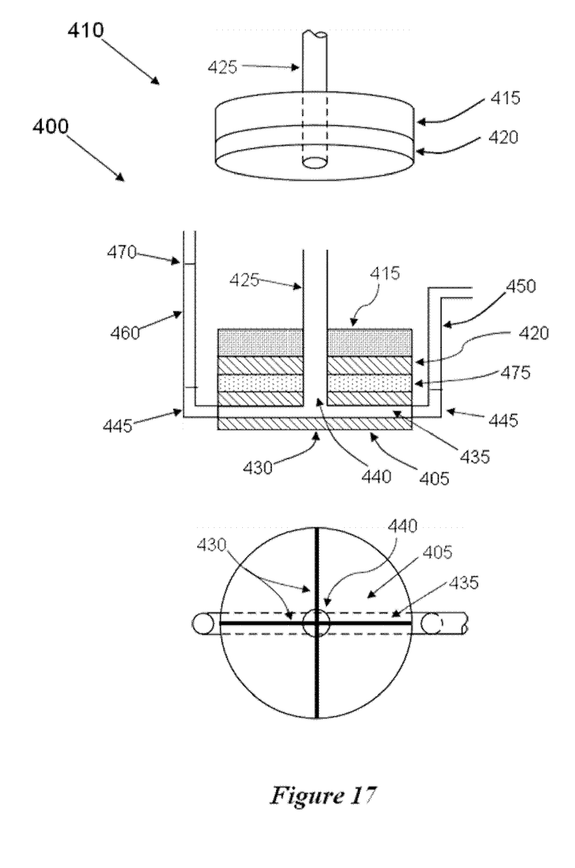

The IPRP sample holder 400 is shown in Figure 17 and comprises a cylindrical bottom plate 405, top plate 410, and cylindrical stainless steel weight 415.

Top plate 410 comprises an annular base plate 420 10 mm thick with an outer diameter of 70.0 mm and a tube 425 of 190 mm length fixed at the center thereof. The tube 425 has in outer diameter of 15.8 mm and an inner diameter of 12.0 mm. The tube is adhesively fixed into a circular 12 mm hole in the center of the base plate 420 such that the lower edge of the tube is flush with the lower surface of the base plate, as depicted in Figure 17. The bottom plate 405 and top plate 410 are fabricated from Lexan® or equivalent. The stainless steel weight 415 has an outer diameter of 70 mm and an inner diameter of 15.9 mm so that the weight is a close sliding fit on tube 425. The thickness of the stainless steel weight 415 is approximately 25 mm and is adjusted so that the total weight of the top plate 410 and the stainless steel weight 415 is 660 g $\pm$ 1g to provide 1.7 kPa of confining pressure during the measurement.

Bottom plate 405 is approximately 50 mm thick and has two registration grooves 430 cut into the lower surface of the plate such that each groove spans the diameter of the bottom plate and the grooves are perpendicular to each other. Each groove is 1.5 mm wide and 2 mm deep. Bottom plate 405 has a horizontal hole 435 which spans the diameter of the plate. The horizontal hole 435 has a diameter of 11 mm and its central axis is 12 mm below the upper surface of bottom plate 405. Bottom plate 405 also has a central vertical hole 440 which has a diameter of 10 mm and is 8 mm deep. The central hole 440 connects to the horizontal hole 435 to form a T-shaped cavity in the bottom plate 405. The outer portions of the horizontal hole 435 are threaded to accommodate pipe elbows 445 which are attached to the bottom plate 405 in a watertight fashion. One elbow is connected to a vertical transparent tube 460 with a height of 190 mm and an internal diameter of 10 mm. The tube 460 is scribed with a suitable mark 470 at a height of 100 mm above the upper surface of the bottom plate 420. This is the reference for the fluid level to be maintained during the measurement. The other elbow 445 is connected to the fluid delivery reservoir 700 (described below) via a flexible tube.

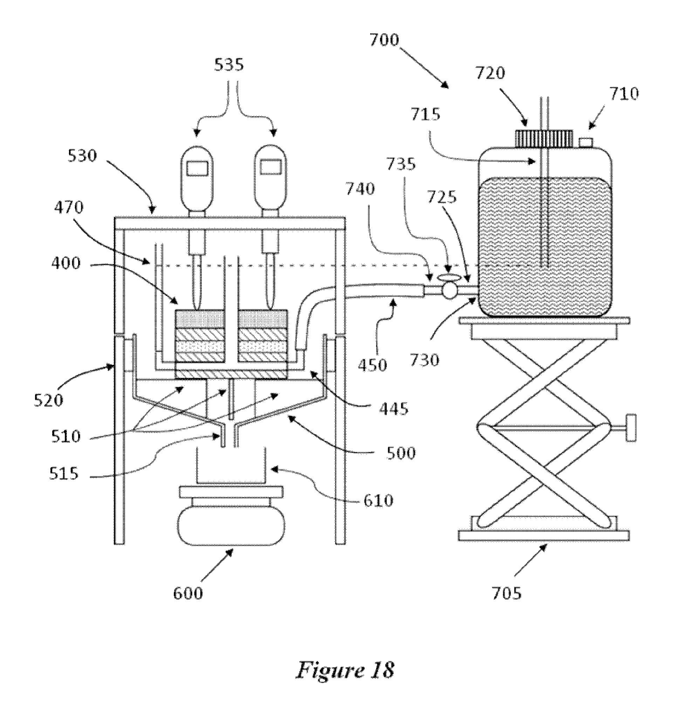

A suitable fluid delivery reservoir 700 is shown in Figure 18. Reservoir 700 is situated on a suitable laboratory jack 705 and has an air-tight stoppered opening 710 to facilitate filling of the reservoir with fluid. An open-ended glass tube 715 having an inner diameter of 10 mm extends through a port 720 in the top of the reservoir such that there is an airtight seal between the outside of the tube and the reservoir. Reservoir 700 is provided with an L-shaped delivery tube 725 having an inlet 730 that is below the surface of the fluid in the reservoir, a stopcock 735, and an outlet 740. The outlet 740 is connected to elbow 445 via flexible plastic tubing 450 (e.g. Tygon®). The internal diameter of the delivery tube 725, stopcock 735, and flexible plastic tubing 450 enable fluid delivery to the IPRP sample holder 400 at a high enough flow rate to maintain the level of fluid in tube 460 at the scribed mark 470 at all times during the measurement. The reservoir 700 has a capacity of approximately 6 litres, although larger reservoirs may be required depending on the sample thickness and permeability. Other fluid delivery systems may be employed provided that they are able to deliver the fluid to the sample holder 400 and maintain the level of fluid in tube 460 at the scribed mark 470 for the duration of the measurement.

The IPRP catchment funnel 500 is shown in Figure 18 and comprises an outer housing 505 with an internal diameter at the upper edge of the funnel of approximately 125 mm. Funnel 500 is constructed such that liquid falling into the funnel drains rapidly and freely from spout 515. A stand with horizontal flange 520 around the funnel 500 facilitates mounting the funnel in a horizontal position. Two integral vertical internal ribs 510 span the internal diameter of the funnel and are perpendicular to each other. Each rib 510 s 1.5 mm wide and the top surfaces of the ribs lie in a horizontal plane. The funnel housing 500 and ribs 510 are fabricated from a suitably rigid material such as Lexan® or equivalent in order to support sample holder 400. To facilitate loading of the sample it is advantageous for the height of the ribs to be sufficient to allow the upper surface of the bottom plate 405 to lie above the funnel flange 520 when the bottom plate 405 is located on ribs 510. A bridge 530 is attached to flange 520 in order to mount two digital calipers 535 to measure the relative height of the stainless steel weight 415. The digital calipers 535 have a resolution of $\pm$ 0.01 mm over a range of 25 mm. A suitable digital caliper is a Mitutoyo model 575-123 (available from McMaster Carr Co., catalog no. 19975-A73), or equivalent. Each caliper is interfaced with a computer to allow height readings to be recorded periodically and stored electronically on the computer. Bridge 530 has two circular holes 17 mm in diameter to accommodate tubes 425 and 460 without the tubes touching the bridge.

Funnel 500 is mounted over an electronic balance 600, as shown in Fig. 18. The balance has a resolution of $\pm$ 0.01 g

and a capacity of at least 1000g. The balance 600 is also interfaced with a computer to allow the balance reading to be recorded periodically and stored electronically on the computer. A suitable balance is Mettler-Toledo model PG5002-S or equivalent. A collection container 610 is situated on the balance pan so that liquid draining from the funnel spout 515 falls directly into the container 610.

The funnel 500 is mounted so that the upper surfaces of ribs 510 lie in a horizontal plane. Balance 600 and container 610 are positioned under the funnel 500 so that liquid draining from the funnel spout 515 falls directly into the container 610. The IPRP sample holder 400 is situated centrally in the funnel 500 with the ribs 510 located in grooves 430. The upper surface of the bottom plate 405 must be perfectly flat and level. The top plate 410 is aligned with and rests on the bottom plate 405. The stainless steel weight 415 surrounds the tube 425 and rests on the top plate 410. Tube 425 extends vertically through the central hole in the bridge 530. Both calipers 535 are mounted firmly to the bridge 530 with the foot resting on a point on the upper surface of the stainless steel weight 415. The calipers are set to zero in this state. The reservoir 700 is filled with 0.9% saline solution and re-sealed. The outlet 740 is connected to elbow 445 via flexible plastic tubing 450.

An annular sample 475 of the material to be tested is cut by suitable means. The sample has an outer diameter of 70 mm and an inner hole diameter of 12 mm. One suitable means of cutting the sample is to use a die cutter with sharp concentric blades.

The top plate 410 is lifted enough to insert the sample 475 between the top plate and the bottom plate 405 with the sample centered on the bottom plate and the plates aligned. The stopcock 735 is opened and the level of fluid in tube 460 is set to the scribed mark 470 by adjusting the height of the reservoir 700 using the jack 705 and by adjusting the position of the tube 715 in the reservoir. When the fluid level in the tube 460 is stable at the scribed mark 470 initiate recording data from the balance and calipers by the computer. Balance readings and time elapsed are recorded every 10 seconds for five minutes. The average sample thickness B is calculated from all caliper reading between 60 seconds and 300 seconds and expressed in cm. The flow rate in grams per second is the slope calculated by linear least squares regression fit of the balance reading (dependent variable) at different times (independent variable) considering only the readings between 60 seconds and 300 seconds.

Permeability k ($cm^2$) is then calculated by the following equation:

$$k = \frac{(Q/\rho_l) \cdot \mu \cdot \ln(R_0/R_i)}{2\pi \cdot B \cdot \Delta p} \qquad \text{(E47-a)}$$

Where:

k    is the permeability ($cm^2$).

Q    is the flow rate (g/s).

$\rho 1$    is the liquid density ($g/cm^3$).

$\mu$    is the liquid viscosity at 20 ˚C (Pa*s).

$R_0$    is the outer sample radius (cm).

$R_i$    is the inner sample radius (cm).

B    is the average sample thickness (cm)

$\Delta p$    is the pressure drop (Pa) calculated according to the following Equation E47-b:

$$\Delta p = \left( \Delta h - \frac{B}{2} \right) \cdot g \cdot \rho_l \cdot 10 \qquad \text{(E47-b)}$$

Where:

Δh    is the measured liquid hydrostatic pressure (cm)

g      is the acceleration constant (m/sec$^2$).

ρ$_1$    is the liquid density (g/cm$^3$).

The direct input into the model is conductivity (K) that is calculated by the permeability (k) as per equation E43-b.

In Plane Radial Permeability (IPRP) for Swelling Samples

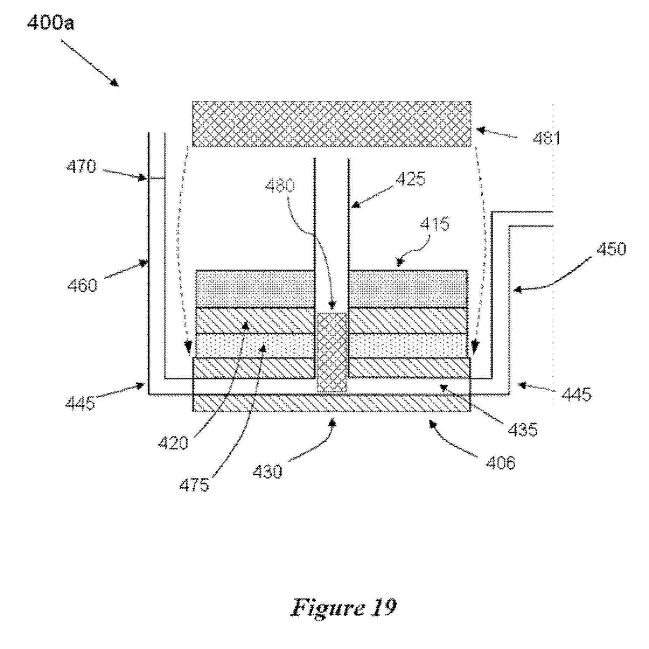

This test is suitable for measurement of the In-Plane Radial Permeability (IPRP) of a porous material. The quantity of a saline solution (0.9% NaCl) flowing radially through an annular sample of the material under constant pressure is measured as a function of time. This test is modified from the previous test to accommodate samples that significantly swell during testing. Testing is performed at 23˚C ± 2C˚ and a relative humidity 50% ± 5%. All samples are conditioned in this environment for twenty four (24) hours before testing.
The IPRP sample holder 400a is shown in Figure 19 and comprises a cylindrical bottom plate 405, top plate 410, and cylindrical stainless steel weight 415.
Top plate 410 comprises an annular base plate 420 10 mm thick with an outer diameter of 70.0 mm and a tube 425 of 190 mm length fixed at the center thereof. The tube 425 has in outer diameter of 15.8 mm and an inner diameter of 12.0 mm. The tube is adhesively fixed into a circular 12 mm hole in the center of the base plate 420 such that the lower edge of the tube is flush with the lower surface of the base plate, as depicted in Figure 17. The bottom plate 406 and top plate 410 are fabricated from Lexan® or equivalent. The stainless steel weight 415 has an outer diameter of 70 mm and an inner diameter of 15.9 mm so that the weight is a close sliding fit on tube 425. The thickness of the stainless steel weight 415 is approximately 25 mm and is adjusted so that the total weight of the top plate 410 and the stainless steel weight 415 is 660g ± 1g to provide 1.7 kPa of confining pressure during the measurement.
Referring to Figure 19, the bottom plate 406 has been modified for use with swellable samples. The plate 406 is 74.0 mm in diameter, leaving a 2 mm ledge where an outer restraining ring 481 can rest after the sample holder 400a is assembled. The ring 481 is 72.0 mm in diameter and 25.0 mm in height, made of a 400 mesh stainless steel screen. The restraining ring prevents the specimen 475 as it swells from extending past the top plate 420. Additionally an inner retraining cylinder 480 is designed to insert into the central hole 440 is 10.5 mm in diameter and 35.0 mm in height, made of a 400 mesh stainless steal screen. The restraining cylinder prevents the specimen 475 from blocking the center hole 440 during the test.
The bottom plate 406 is approximately 50 mm thick and has two registration grooves 430 cut into the lower surface of the plate such that each groove spans the diameter of the bottom plate and the grooves are perpendicular to each other. Each groove is 1.5 mm wide and 2 mm deep. Bottom plate 406 has a horizontal hole 435 which spans the diameter of the plate. The horizontal hole 435 has a diameter of 11 mm and its central axis is 12 mm below the upper surface of bottom plate 406. Bottom plate 406 also has a central vertical hole 440 which has a diameter of 10 mm and is 8 mm deep. The central hole 440 connects to the horizontal hole 435 to form a T-shaped cavity in the bottom plate 405. The outer portions of the horizontal hole 435 are threaded to accommodate pipe elbows 445 which are attached to the bottom plate 405 in a watertight fashion. One elbow is connected to a vertical transparent tube 460 with a height of 190 mm and an internal diameter of 10 mm. The tube 460 is scribed with a suitable mark 470 at a height of 100 mm above the upper surface of the bottom plate 420. This is the reference for the fluid level to be maintained during the measurement. The other elbow 445 is connected to the fluid delivery reservoir 700 (described below) via a flexible tube.
A suitable fluid delivery reservoir 700 is shown in Figure 18. Reservoir 700 is situated on a suitable laboratory jack 705 and has an air-tight stoppered opening 710 to facilitate filling of the reservoir with fluid. An open-ended glass tube 715 having an inner diameter of 10 mm extends through a port 720 in the top of the reservoir such that there is an airtight seal between the outside of the tube and the reservoir. Reservoir 700 is provided with an L-shaped delivery tube 725 having an inlet 730 that is below the surface of the fluid in the reservoir, a stopcock 735, and an outlet 740. The outlet 740 is connected to elbow 445 via flexible plastic tubing 450 (e.g. Tygon®). The internal diameter of the delivery tube 725, stopcock 735, and flexible plastic tubing 450 enable fluid delivery to the IPRP sample holder 400 at a high enough flow rate to maintain the level of fluid in tube 460 at the scribed mark 470 at all times during the measurement. The reservoir 700 has a capacity of approximately 6 litres, although larger reservoirs may be required depending on the sample thickness and permeability. Other fluid delivery systems may be employed provided that they are able to deliver the fluid to the sample holder 400 and maintain the level of fluid in tube 460 at the scribed mark 470 for the duration of the measurement.
The IPRP catchment funnel 500 is shown in Figure 18 and comprises an outer housing 505 with an internal diameter at the upper edge of the funnel of approximately 125 mm. Funnel 500 is constructed such that liquid falling into the funnel drains rapidly and freely from spout 515. A stand with horizontal flange 520 around the funnel 500 facilitates mounting the funnel in a horizontal position. Two integral vertical internal ribs 510 span the internal diameter of the funnel and are perpendicular to each other. Each rib 510 s 1.5 mm wide and the top surfaces of the ribs lie in a horizontal plane. The funnel housing 500 and ribs 510 are fabricated from a suitably rigid material such as Lexan® or equivalent in order to support sample holder 400. To facilitate loading of the sample it is advantageous for the height of the ribs to be sufficient to allow the upper surface of the bottom plate 405 to lie above the funnel flange 520 when the bottom plate 405 is located on ribs 510. A bridge 530 is attached to flange 520 in order to mount two digital calipers 535 to measure the relative height of the stainless steel weight 415. The digital calipers 535 have a resolution of $\pm$ 0.01 mm over a range of 25 mm. A suitable digital caliper is a Mitutoyo model 575-123 (available from McMaster Carr Co., catalog no. 19975-A73), or equivalent. Each caliper is interfaced with a computer to allow height readings to be recorded periodically and stored electronically on the computer. Bridge 530 has two circular holes 17 mm in diameter to accommodate tubes 425 and 460 without the tubes touching the bridge.

Funnel 500 is mounted over an electronic balance 600, as shown in Fig. 18. The balance has a resolution of $\pm$ 0.01 g and a capacity of at least 1000g. The balance 600 is also interfaced with a computer to allow the balance reading to be recorded periodically and stored electronically on the computer. A suitable balance is Mettler-Toledo model PG5002-S or equivalent. A collection container 610 is situated on the balance pan so that liquid draining from the funnel spout 515 falls directly into the container 610.

The funnel 500 is mounted so that the upper surfaces of ribs 510 lie in a horizontal plane. Balance 600 and container 610 are positioned under the funnel 500 so that liquid draining from the funnel spout 515 falls directly into the container 610. The IPRP sample holder 400a is situated centrally in the funnel 500 with the ribs 510 located in grooves 430. The upper surface of the bottom plate 405 must be perfectly flat and level. The top plate 410 is aligned with and rests on the bottom plate 405. The stainless steel weight 415 surrounds the tube 425 and rests on the top plate 410. Tube 425 extends vertically through the central hole in the bridge 530. Both calipers 535 are mounted firmly to the bridge 530 with the foot resting on a point on the upper surface of the stainless steel weight 415. The calipers are set to zero in this state. The reservoir 700 is filled with 0.9% saline solution and re-sealed. The outlet 740 is connected to elbow 445 via flexible plastic tubing 450.

An annular specimen 475 of the material to be tested is cut by suitable means. The specimen has an outer diameter of 70 mm and an inner hole diameter of 12 mm. One suitable means of cutting the sample is to use a die cutter with sharp concentric blades.

The top plate 410 is lifted enough to insert the sample 475 between the top plate and the bottom plate 405 with the sample centered on the bottom plate and the plates aligned. The inner hole of the sample 475 fits around the inner restraining cylinder 480 and the top plate 410 is placed on top of the specimen with the restraining cylinder fitting within the tube 425. After the sample assembly 400a is assembled the outer restraining ring 481 is placed around the specimen and top plate 410 resting on the ledge of the lower plate 406. The stopcock 735 is opened and the level of fluid in tube 460 is set to the scribed mark 470 by adjusting the height of the reservoir 700 using the jack 705 and by adjusting the position of the tube 715 in the reservoir. When the fluid level in the tube 460 is stable at the scribed mark 470, initiate the recording data from the balance and calipers by the computer. Balance readings and time elapsed are recorded every 10 seconds for five minutes. The computer output will consist of matching balance and average caliper values per time point.

Calculation:

The flow rate (g/s) calculation is calculated by the following equations:

$$Q(t_{1/2}) = \frac{\left(m^{l}{}_{(i-1)} - m^{l}{}_{(i)}\right)}{\left(t_{(i-1)} - t_{(i)}\right)} \qquad \text{(E48-a)}$$

$$t_{1/2} = \frac{\left(t_{(i-1)} + t_{(i)}\right)}{2} \qquad \text{(E48-b)}$$

Where:

$Q(t_{1/2})$    is the flow rate (g/s)

$t_{1/2}$     is the reference time for each interval (s)

$m^1_{(i)}$     is the fluid mass measured by the balance (g)

$Q(t_{1/2})$ value and the average thickness between two consecutive readings (B) are used in the below Equation (E48-c) to calculate the permeability k (cm2) at each time point.

$$k(t_{1/2}) = \frac{(Q(t_{1/2})/\rho_l) \cdot \mu \cdot \ln(R_0 / R_i)}{2\pi \cdot B(t_{1/2}) \cdot \Delta p} \qquad \text{(E48-c)}$$

Where:

$k(t_{1/2})$     is the permeability at time $t_{1/2}$ ($CM^2$).

$Q(t_{1/2})$     is the flow rate (g/s) between two consecutive readings.

$\rho_1$     is the liquid density ($g/cm^3$).

$\mu$     is the liquid viscosity at 20 ˚C (Pa*s).

Ro     is the outer sample radius (cm).

Ri     is the inner sample radius (cm).

$B(t_{1/2})$     is the average sample thickness (cm) between two consecutive readings

$\Delta p$     is the pressure drop (Pa) calculated according to the following (E48-d):

$$\Delta p = \left( \Delta h - \frac{B}{2} \right) \cdot g \cdot \rho_l \cdot 10 \qquad \text{(E48-d)}$$

Where:

$\Delta h$    is the measured liquid hydrostatic pressure (cm).

g    is the acceleration constant ($m/sec^2$).

$\rho 1$    is the liquid density ($g/cm^3$).

The final output of this methods is therefore an ordered table of time points ($t_{1/2}$) and the correspondent permeabilities ( $k(t_{1/2})$ ).
The direct input into the model is conductivity (K) that is calculated from the permeability (k) as per equation E43-a for each time point.

Capillary Pressure

Capillary pressure measurements are made on a TRI/Autoporosimeter (TRI/Princeton Inc. of Princeton, N.J.). The TRI/Autoporosimeter is an automated computer-controlled instrument for measuring capillary pressure in porous materials, which can be schematically represented in Figure 13. Complimentary Automated Instrument Software, Release Version 2007.2, and Data Treatment Software, Release Version 2007.2 is used to capture, analyze and output the data. More information on the TRI/Autoporosimeter, its operation and data treatments can be found in The Journal of Colloid and Interface Science 162 (1994), pgs 163-170, incorporated here by reference.

As used in this application, determining Capillary pressure hysteresis curve of a material as function of saturation, involves recording the increment of liquid that enters a porous material as the surrounding air pressure changes. A sample in the test chamber is exposed to precisely controlled changes in air pressure which at equilibrium (no more liquid uptake/release) correspond to the capillary pressure.

For swelling materials the behavior of capillary pressure versus load is determined by repeating the measurements using liquids with different salt concentration (0.9, 10, and 25 percent by weight in deionized water). It is in fact well known in the art that different saline solution concentration provide different swelling extent to the AGM.

The equipment operates by changing the test chamber air pressure in user-specified increments, either by decreasing pressure (increasing pore size) to absorb liquid, or increasing pressure (decreasing pore size) to drain liquid. The liquid volume absorbed (drained) is measured with a balance at each pressure increment. The saturation is automatically calculated from the cumulative volume.

All testing is performed at 23˚C $\pm$ 2C˚ and a relative humidity 50% $\pm$ 5%. Prepare three saline solutions of 0.9%, 10.0% and 25.0% weight to volume in deionized water. The surface tension (mN/m), contact angle (˚), and density (g/cc) for all solutions are determined by any method know in the art.

Input the surface tension (mN/m), contact angle (˚), and density (g/cm$^3$) into the instrument's software. Level the balance at 170.0g and set the equilibration rate to 5 mg/min. and equilibrium thickness of 5 $\mu$m/min. Assign the pore radius protocol (corresponding to capillary pressure steps) to scan capillary pressures according to equation R=2y cosθ/$\Delta$p, where:

   R is the pore radius,
   y is the surface tension
   θ is the contact angle
   $\Delta$p is the capillary pressure

Enter in the pore radius (R) steps into the program in $\mu$m:

- First absorption (pressure decreasing): 5, 10, 15, 20, 25, 30, 40, 50, 60, 70, 80, 90, 100, 110, 120, 130, 140, 150, 160, 170, 180, 190, 200, 225, 250, 275, 300, 400, 600, 800, 1000, 1200.
- Desorption (pressure increasing): 1200, 1000, 800, 600, 400, 300, 275, 250, 225, 200, 190, 180, 170, 160, 150, 140, 130, 120, 110, 100, 90, 80, 70, 60, 50, 40, 30, 25, 20, 15, 10,5.
- Second absorption (pressure decreasing): 5, 10, 15, 20, 25, 30, 40, 50, 60, 70, 80, 90, 100, 110, 120, 130, 140, 150, 160, 170, 180, 190, 200, 225, 250, 275, 300, 400, 600, 800, 1000, 1200.

Cut the sample into a 5 cm by 5 cm square specimen. If the material is non-swellable, the samples are conditioned at 23˚C $\pm$ 2C˚ and a relative humidity 50% $\pm$ 5% for twenty four (24) hours before testing. If the material is swellable (i.e., contains an absorbent gelling material), immerse the square in the test solution that will be used for testing for one hour. Place the material between two stacks of blotting paper (5 sheets each) for 3 minutes under a confining of weight equivalent to 0.25 psi. Repeat blotting the specimen three times, each time with new blotting paper stacks, in order to remove the excess fluid from the specimen.

Measure the weight to $\pm$ 0.0001 g and the caliper to $\pm$ 0.01 mm of the specimen. The caliper is measured at 0.25 psi using a 24 mm diameter foot, with the thickness read 5 sec. after placing the foot onto the sample. Place the cover plate and weight into the empty sample chamber, and close the chamber. After the instrument's internal caliper gauge is set to zero and has applied the appropriate air pressure to the cell, close the liquid valve, open the chamber and remove the cover plate. Place the specimen, cover plate and confming weight into the chamber and close it. After the instrument has applied the appropriate air pressure to the cell, open liquid valve to allow free movement of liquid to the balance and begin the test under the radius protocol. The instrument will proceed through one absorption/desorption/absorption cycle. A blank (without specimen) is run in like fashion.

Calculations and Reporting:

The mass uptake from a blank run is directly subtracted from the uptake of the sample. Saturation at each capillary pressure step is automatically calculated from liquid uptake as follows:

$$S = \frac{m^l}{m_{max}^l} \qquad (\text{E49})$$

Where:

S = saturation
$m^1$ = liquid uptake at the pressure step (mL)
$m^1_{max}$ = maximum liquid uptake (mL)

Pressure is reported in cm of water and saturation in %. Only the data from the first absorption curve and the desorption curve are used. The capillary pressure curve is rescaled from saline to AMF by multiplying each single capillary pressure value by the wicking scaling factor ($f_{SC}$) measured with the wicking test described herein.

Wicking test

The capillary pressure test method cannot be run directly with AMF. This method determines a wicking scaling factor used to recalculate the capillary pressure versus saturation curve determined in the capillary pressure test. Wicking height of the composite material (fluid swellable or non swellable) is measured in both 0.9% NaCl (w/v) in deionized water and AMF and compared. Testing is performed at 23°C $\pm$ 2C° and a relative humidity 50% $\pm$ 5%. All samples are conditioned in this environment for twenty four (24) hours before testing.
Cut a strip of sample 2.0 cm $\pm$ 0.05 cm wide and 20.0 cm $\pm$ 0.1 cm in length. Take a container, 90 mm in diameter and 3 cm in height and fill to approximately 2 mm depth with AMF. Using a convenient lab stand with horizontal arm, suspend the strip by one end, and immerse the remaining free end one cm into the fluid. Let the fluid rise through the strip. At each hour, measure the average wicking height across the width of the strip (i.e., the average of the highest point of the fluid front and the lowest point of the fluid front, within a strip) to the nearest 1.0 mm. Allow the sample to wick until two consecutive average heights are within 1.0 mm of each other. Record the final average wicking height of the liquid to the nearest 1.0 mm.
Perform the average wicking height with AMF for a total of ten replicates. Repeat this procedure with 0.9% NaCl (w/v) in deionized water for ten replicates. Average the ten measured wicking heights for each fluid and calculate the scaling factor:

$$f_{SC} = (h_{AMF} \cdot \rho_{AMF}) / (h_{saline} \cdot \rho_{saline}) \qquad (E50)$$

Where $h_{AMF}$ and $h_{saline}$ are the wicking height (cm) in AMF and 0.9% saline respectively, and $\rho_{AMF}$ and $\rho_{saline}$ are the densities of AMF and 0.9% saline respectively.

Rheological Creep Test

The Rheological Creep Test mentioned hereinabove for measuring the cohesive strength parameter y is as described in the copending patent application EP 1447067, assigned to the Procter & Gamble Company.
The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as"40 mm" is intended to mean"about 40 mm",

**Claims**

1.  An absorbent core structure for an absorbent article, said absorbent core structure comprising a first layer,
    said first layer comprising a first surface and a second surface,
    said absorbent core structure further comprising a layer of absorbent polymer material,
    said layer of absorbent polymer material comprising a first surface and a second surface,
    said absorbent core structure further comprising a layer of adhesive,
    said layer of adhesive comprising a first surface and a second surface,
    wherein said layer of absorbent polymer material is comprised between said layer of adhesive and said first layer;
    said second surface of said layer of absorbent polymer material is facing said first surface of said first layer;
    and said first surface of said layer of absorbent polymer material is facing said second surface of said layer of adhesive,
    said absorbent core structure further comprises a second layer having respective first and second surface, positioned such that said second surface of said second layer is facing said first surface of said layer of adhesive

**characterized in that**
said absorbent core structure has a Virtual Free Fluid at 20 min of below 2.2 g, and a Virtual Acquisition Time at $2^{nd}$ gush of 35 sec or less, said Virtual Free Fluid and Virtual Acquisition Time calculated with the simulation model as described herein.

2. An absorbent core structure according to claim 1, having a Virtual Free Fluid at 20 min of below 2 g, preferably below 1.5 g, and a Virtual Acquisition Time at $2^{nd}$ gush of 30 sec or less, preferably of 25 sec or less.

3. An absorbent core structure according to any preceding claim, having a Virtual Free Fluid at 60 min of less than 2.2 g, and a Virtual Acquisition Time at $3^{rd}$ gush of 40 sec or less.

4. An absorbent core structure according to any preceding claim, having a Virtual Free Fluid at 60 min of less than 2 g, preferably less than 1.5 g, and a Virtual Acquisition Time at $3^{rd}$ gush of 35 sec or less, preferably of 30 sec or less.

5. An absorbent core structure according to any of the preceding claims, wherein said second layer has a Permeability, MAP, Thickness, measured according to the respective test methods described herein, wherein said second layer has a Permeability of at least 250 Darcy, preferably of at least 300 Darcy, more preferably of at least 400 Darcy, a MAP of 0.020 to 0.050 m $H_2O$, and a thickness of 0.3 to 0.6 mm.

6. An absorbent core structure according to any preceding claim, wherein said first layer has a Permeability, MAP, Thickness, measured according to the respective test methods described herein, wherein said first layer has an Permeability of at least 500 Darcy, preferably of at least 600 Darcy, more preferably of at least 1000 Darcy, a MAP of 0.01 to 0.06 m $H_2O$, and a thickness of 0.4 to 1.0 mm

7. An absorbent core structure according to any preceding claim, wherein said second layer is selected among non-woven materials comprising synthetic fibres, such as polyethylene (PE), polyethylene terephthalate (PET), polypropylene (PP).

8. An absorbent core structure according to any preceding claim, wherein said first layer is selected among nonwoven materials, or airlaid or wetlaid fibrous materials, comprising synthetic fibres, or natural fibres, or mixtures thereof.

9. An absorbent core structure according to any preceding claim, wherein said layer of absorbent polymer material has an average basis weight of less than 250 $g/m^2$, preferably less than 200 $g/m^2$, more preferably from 60 $g/m^2$ to 180 $g/m^2$, even more preferably from 70 $g/m^2$ to 150 $g/m^2$.

10. An absorbent core structure according to any preceding claim, wherein said adhesive is a hot melt adhesive.

11. An absorbent core structure according to any preceding claim, wherein said adhesive is fiberized, comprising fibres having an average thickness from 1 $\mu$m to 100 $\mu$m, preferably from 25 $\mu$m to 75 $\mu$m, and an average length from 5 mm to 50 cm.

12. An absorbent core structure according to any preceding claim, wherein said adhesive is provided in a basis weight of from 11 $g/m^2$ to 3 $g/m^2$, preferably of from 9 $g/m^2$ to 5 $g/m^2$.

13. An absorbent core structure according to any preceding claim, wherein said second surface of said layer of absorbent polymer material is in contact with said first surface of said first layer;
said first surface of said layer of absorbent polymer material is in contact with said second surface of said layer of adhesive;
said second surface of said second layer is in contact with said first surface of said layer of adhesive.

14. An absorbent feminine hygiene product comprising the absorbent core structure according to any of the preceding claims.

15. The absorbent feminine hygiene product according to claim 14, wherein the absorbent feminine hygiene product is a sanitary napkin.

*Fig. 1*

*Fig. 2*

**Fig. 3**

**Fig. 4**

**Figure 5**

**Figure 6**

**Figure 7**

**Figure 8**

(a) $t = 1s$

(b) $t = 50$ s

(c) $t = 861s$

(d) $t = 902$ s

(e) $t = 1495$ s

**Figure 9**

**Figure 10**

boundary-segment shadow      boundary-segment normal vector

fixed external constraint      boundary-node normal vector

**Figure 11**

**Figure 12**

**Figure 13**

**Figure 14**

*Figure 15.*

*Figure 16.*

*Figure 17*

*Figure 18*

400a

*Figure 19*

Europäisches
Patentamt

European
Patent Office

Office européen
des brevets

# EUROPEAN SEARCH REPORT

Application Number

EP 10 16 9724

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | EP 1 447 067 A1 (PROCTER & GAMBLE [US]) 18 August 2004 (2004-08-18) * the whole document * ----- | 1-15 | INV. A61F13/15 |
| X | US 2009/118689 A1 (LAWSON KATHLEEN MARIE [US] ET AL) 7 May 2009 (2009-05-07) * the whole document * ----- | 1-15 | |
| X | US 2009/043273 A1 (CARLUCCI GIOVANNI [IT] ET AL) 12 February 2009 (2009-02-12) * the whole document * ----- | 1-15 | |

|  |  |
|---|---|
|  | TECHNICAL FIELDS SEARCHED (IPC) |
|  | A61F |

The present search report has been drawn up for all claims

| Place of search | Date of completion of the search | Examiner |
|---|---|---|
| The Hague | 20 April 2011 | Douskas, K |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 10 16 9724

This annex lists the patent family members relating to the patent documents cited in the above-mentioned European search report.
The members are as contained in the European Patent Office EDP file on
The European Patent Office is in no way liable for these particulars which are merely given for the purpose of information.

20-04-2011

| Patent document cited in search report | | Publication date | Patent family member(s) | | Publication date |
|---|---|---|---|---|---|
| EP 1447067 | A1 | 18-08-2004 | AR | 043165 A1 | 20-07-2005 |
| | | | AT | 380007 T | 15-12-2007 |
| | | | AT | 473717 T | 15-07-2010 |
| | | | AT | 455528 T | 15-02-2010 |
| | | | AU | 2004212006 A1 | 26-08-2004 |
| | | | BR | PI0407132 A | 07-02-2006 |
| | | | CA | 2515118 A1 | 26-08-2004 |
| | | | CL | 2462004 A1 | 08-04-2005 |
| | | | CN | 1741780 A | 01-03-2006 |
| | | | DE | 60317873 T2 | 13-11-2008 |
| | | | EG | 23770 A | 08-08-2007 |
| | | | EP | 1911425 A2 | 16-04-2008 |
| | | | EP | 1917940 A2 | 07-05-2008 |
| | | | EP | 1913912 A1 | 23-04-2008 |
| | | | EP | 1913913 A2 | 23-04-2008 |
| | | | EP | 1913914 A2 | 23-04-2008 |
| | | | EP | 1911426 A2 | 16-04-2008 |
| | | | EP | 2177188 A1 | 21-04-2010 |
| | | | EP | 2177189 A1 | 21-04-2010 |
| | | | ES | 2297062 T3 | 01-05-2008 |
| | | | ES | 2347827 T3 | 04-11-2010 |
| | | | ES | 2339713 T3 | 24-05-2010 |
| | | | JP | 4425865 B2 | 03-03-2010 |
| | | | JP | 2006513824 T | 27-04-2006 |
| | | | KR | 20050113179 A | 01-12-2005 |
| | | | KR | 20070103519 A | 23-10-2007 |
| | | | MX | PA05007634 A | 25-05-2006 |
| | | | US | 2004167486 A1 | 26-08-2004 |
| | | | US | 2010228210 A1 | 09-09-2010 |
| | | | US | 2008125735 A1 | 29-05-2008 |
| | | | WO | 2004071539 A2 | 26-08-2004 |
| | | | ZA | 200505366 A | 26-04-2006 |
| US 2009118689 | A1 | 07-05-2009 | AU | 2008326036 A1 | 14-05-2009 |
| | | | CA | 2705024 A1 | 14-05-2009 |
| | | | CN | 101854893 A | 06-10-2010 |
| | | | EP | 2207513 A1 | 21-07-2010 |
| | | | WO | 2009060384 A1 | 14-05-2009 |
| | | | JP | 2011500257 T | 06-01-2011 |
| US 2009043273 | A1 | 12-02-2009 | CA | 2695871 A1 | 19-02-2009 |
| | | | CN | 101772335 A | 07-07-2010 |
| | | | EP | 2022452 A1 | 11-02-2009 |
| | | | WO | 2009022277 A1 | 19-02-2009 |
| | | | JP | 2010535572 T | 25-11-2010 |
| | | | KR | 20100040744 A | 20-04-2010 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 1447067 A
- US 3929135 A
- US 4151240 A
- US 4319868 A
- US 4324426 A
- US 4343314 A
- US 4591523 A
- US 4609518 A
- US 4629643 A
- US 4695422 A
- WO 9600548 A
- WO 07047598 A
- WO 07046052 A
- EP 09153881 A
- US 6417424 B

### Non-patent literature cited in the description

- Dynamics of fluids in Porous Media. **JACOB BEAR.** Dover Science Books. 1988
- **DIERSCH ; PERROCHET.** On the primary variable switching techniques for simulating unsatured-saturated flows. *Adv. Water Resour.,* 1999, vol. 23, 271-301
- Fleflow finite element subsurface flow and transport simulation system. User's Manual/ reference Manual/ White papers; Release 5.4
- **FREDRIC L. BUCHHOLZ.** Model of Liquid Permeability in Swollen Composites of Superabsorbent Polymer and Fiber. *Journal of Applied Polymer Science,* 2006, vol. 102, 4075-4084
- *The Journal of Colloid and Interface Science,* 1994, vol. 162, 163-170